# EUROPEAN PATENT SPECIFICATION

(11) **EP 1 012 186 B1**
(45) Date of publication and mention of the grant of the patent: **17.07.2002**
(21) Application number: 97953157.1
(22) Date of filing: 08.12.1997
(51) Int. Cl.: C07K 14/50, C07K 14/605, A61K 38/18, A61K 38/26

(54) **USE OF A KGF PROTEIN PRODUCT(S) AND A GLP-2 PROTEIN PRODUCT(S) FOR THE PREPARATION OF A MEDICAMENT**
VERWENDUNG EINES KGF-PROTEINPRODUKTES UND EINES GLP-2 PROTEINPRODUKTES FÜR DIE HERSTELLUNG EINES MEDIKAMENTES
UTILISATION DE PRODUIT(S) PROTEIQUE(S) KGF ET DE PRODUIT(S) PROTEIQUE(S)GLP-2 POUR LA PREPARATION D'UN MEDICAMENT

(30) Priority: 06.12.1996 US 32533 P; 15.10.1997 US 62074 P
(43) Date of publication of application: 28.06.2000
(73) Proprietor: Amgen Inc.,, Thousand Oaks, California 91320-1799 (US)
(72) Inventor: FARRELL, Catherine, L., Canyon Country, CA 91351 (US); LI, Yue-Sheng, Thousand Oaks, CA 91360 (US)
(74) Representative: Grünecker, Kinkeldey, Stockmair & Schwanhäusser Anwaltssozietät
(86) International application number: US9722735
(87) International publication number: WO9824813

(56) References cited:
- D J DRUCKER ET AL.: "Induction of intestinal epithelial proliferation by glucagon-2 like peptide" PROCEEDINGS OF THE NATIONAL ACADEMY OF SCIENCES OF USA., vol. 93, no. 15, 23 July 1996, WASHINGTON US, pages 7911-7916, XP000602070
- BIOLOGICAL ABSTRACTS, vol. 102, no. 7, 1996 Philadelphia, PA, US; abstract no. 96747, M BRAUCHLE ET AL.: "Keratinocyte growth factor is highly overexpressed on inflammatory bowel disease" XP002063839 & AMERICAN JOURNAL OF PATHOLOGY, vol. 149, no. 2, August 1996, pages 521-529,

### SEQUENCE LISTING

(1) GENERAL INFORMATION:
   (i) APPLICANT: Amgen Inc.
   (ii) TITLE OF INVENTION: Keratinocyte Growth Factors and Uses Thereof
   (iii) NUMBER OF SEQUENCES: 69
   (iv) CORRESPONDENCE ADDRESS:
      (A) ADDRESSEE: Amgen Inc.
      (B) STREET: 1840 Dehavilland Drive
      (C) CITY: Thousand Oaks
      (D) STATE: California
      (E) COUNTRY: U.S.A.
      (F) ZIP: 91320-1789
   (v) COMPUTER READABLE FORM:
      (A) MEDIUM TYPE: Floppy disk
      (B) COMPUTER: IBM PC compatible
      (C) OPERATING SYSTEM: PC-DOS/MS-DOS
      (D) SOFTWARE: PatentIn Release #1.0, Version #1.30
   (vi) CURRENT APPLICATION DATA:
      (A) APPLICATION NUMBER: NOT YET KNOWN
      (B) FILING DATE: 08-DEC-1997
      (C) CLASSIFICATION:
   (vii) PRIOR APPLICATION DATA:
      (A) APPLICATION NUMBER: US 60/032,533
      (B) FILING DATE: 06-DEC-1996
   (vii) PRIOR APPLICATION DATA:
      (A) APPLICATION NUMBER: US 60/062,074
      (B) FILING DATE: 15-OCT-1997
   (viii) ATTORNEY/AGENT INFORMATION:
      (A) NAME: Zindrick, Thomas D.
      (B) REGISTRATION NUMBER: 32,185
      (C) REFERENCE/DOCKET NUMBER: A-481B
(2) INFORMATION FOR SEQ ID NO:1:
   (i) SEQUENCE CHARACTERISTICS:
      (A) LENGTH: 862 base pairs
      (B) TYPE: nucleic acid
      (C) STRANDEDNESS: unknown
      (D) TOPOLOGY: unknown
   (ii) MOLECULE TYPE: cDNA
   (ix) FEATURE:
      (A) NAME/KEY: CDS
      (B) LOCATION: 108..692
   (xi) SEQUENCE DESCRIPTION: SEQ ID NO:1:
   CAATCTACAA TTCACAGATA GGAAGAGGTC AATGACCTAG GAGTAACAAT CAACTCAAGA      60
   TTCATTTTCA TTATGTTATT CATGAACACC CGGAGCACTA CACTATA ATG CAC AAA      116
                                                        Met His Lys
                                                         1
   TGG ATA CTG ACA TGG ATC CTG CCA ACT TTG CTC TAC AGA TCA TGC TTT      164
   Trp Ile Leu Thr Trp Ile Leu Pro Thr Leu Leu Tyr Arg Ser Cys Phe
       5                  10                  15
   CAC ATT ATC TGT CTA GTG GGT ACT ATA TCT TTA GCT TGC AAT GAC ATG      212
   His Ile Ile Cys Leu Val Gly Thr Ile Ser Leu Ala Cys Asn Asp Met
    20                  25                  30                  35
   ACT CCA GAG CAA ATG GCT ACA AAT GTG AAC TGT TCC AGC CCT GAG CGA      260
   Thr Pro Glu Gln Met Ala Thr Asn Val Asn Cys Ser Ser Pro Glu Arg
                   40                  45                  50
   CAC ACA AGA AGT TAT GAT TAC ATG GAA GGA GGG GAT ATA AGA GTG AGA      308
   His Thr Arg Ser Tyr Asp Tyr Met Glu Gly Gly Asp Ile Arg Val Arg
               55                  60                  65
   AGA CTC TTC TGT CGA ACA CAG TGG TAC CTG AGG ATC GAT AAA AGA GGC      356
   Arg Leu Phe Cys Arg Thr Gln Trp Tyr Leu Arg Ile Asp Lys Arg Gly
           70                  75                  80
   AAA GTA AAA GGG ACC CAA GAG ATG AAG AAT AAT TAC AAT ATC ATG GAA      404
   Lys Val Lys Gly Thr Gln Glu Met Lys Asn Asn Tyr Asn Ile Met Glu
       85                  90                  95
   ATC AGG ACA GTG GCA GTT GGA ATT GTG GCA ATC AAA GGG GTG GAA AGT      452
   Ile Arg Thr Val Ala Val Gly Ile Val Ala Ile Lys Gly Val Glu Ser
   100                 105                 110                 115
   GAA TTC TAT CTT GCA ATG AAC AAG GAA GGA AAA CTC TAT GCA AAG AAA      500
   Glu Phe Tyr Leu Ala Met Asn Lys Glu Gly Lys Leu Tyr Ala Lys Lys
                   120                 125                 130
   GAA TGC AAT GAA GAT TGT AAC TTC AAA GAA CTA ATT CTG GAA AAC CAT      548
   Glu Cys Asn Glu Asp Cys Asn Phe Lys Glu Leu Ile Leu Glu Asn His
               135                 140                 145
   TAC AAC ACA TAT GCA TCA GCT AAA TGG ACA CAC AAC GGA GGG GAA ATG      596
   Tyr Asn Thr Tyr Ala Ser Ala Lys Trp Thr His Asn Gly Gly Glu Met
           150                 155                 160
   TTT GTT GCC TTA AAT CAA AAG GGG ATT CCT GTA AGA GGA AAA AAA ACG      644
   Phe Val Ala Leu Asn Gln Lys Gly Ile Pro Val Arg Gly Lys Lys Thr
       165                 170                 175
   AAG AAA GAA CAA AAA ACA GCC CAC TTT CTT CCT ATG GCA ATA ACT TAA      692
   Lys Lys Glu Gln Lys Thr Ala His Phe Leu Pro Met Ala Ile Thr  *
   180                 185                 190                 195
   TTGCATATGG TATATAAAGA ACCCAGTTCC AGCAGGGAGA TTTCTTTAAG TGGACTGTTT      752
   TCTTTCTTCT CAAAATTTTC TTTCCTTTTA TTTTTTAGTA ATCAAGAAAG GCTGGAAAAA      812
   CTACTGAAAA ACTGATCAAG CTGGACTTGT GCATTTATGT TTGTTTTAAG                 862
(2) INFORMATION FOR SEQ ID NO:2:
   (i) SEQUENCE CHARACTERISTICS:
      (A) LENGTH: 195 amino acids
      (B) TYPE: amino acid
      (D) TOPOLOGY: linear
   (ii) MOLECULE TYPE: protein
   (xi) SEQUENCE DESCRIPTION: SEQ ID NO:2:
   Met His Lys Trp Ile Leu Thr Trp Ile Leu Pro Thr Leu Leu Tyr Arg
    1               5                  10                  15
   Ser Cys Phe His Ile Ile Cys Leu Val Gly Thr Ile Ser Leu Ala Cys
               20                  25                  30
   Asn Asp Met Thr Pro Glu Gln Met Ala Thr Asn Val Asn Cys Ser Ser
           35                  40                  45
   Pro Glu Arg His Thr Arg Ser Tyr Asp Tyr Met Glu Gly Gly Asp Ile
       50                  55                  60
   Arg Val Arg Arg Leu Phe Cys Arg Thr Gln Trp Tyr Leu Arg Ile Asp
   65                  70                  75                  80
   Lys Arg Gly Lys Val Lys Gly Thr Gln Glu Met Lys Asn Asn Tyr Asn
                   85                  90                  95
   Ile Met Glu Ile Arg Thr Val Ala Val Gly Ile Val Ala Ile Lys Gly
               100                 105                 110
   Val Glu Ser Glu Phe Tyr Leu Ala Met Asn Lys Glu Gly Lys Leu Tyr
           115                 120                 125
   Ala Lys Lys Glu Cys Asn Glu Asp Cys Asn Phe Lys Glu Leu Ile Leu
       130                 135                 140
   Glu Asn His Tyr Asn Thr Tyr Ala Ser Ala Lys Trp Thr His Asn Gly
   145                 150                 155                 160
   Gly Glu Met Phe Val Ala Leu Asn Gln Lys Gly Ile Pro Val Arg Gly
                   165                 170                 175
   Lys Lys Thr Lys Lys Glu Gln Lys Thr Ala His Phe Leu Pro Met Ala
               180                 185                 190
   Ile Thr  *
           195
(2) INFORMATION FOR SEQ ID NO:3:
   (i) SEQUENCE CHARACTERISTICS:
      (A) LENGTH: 495 base pairs
      (B) TYPE: nucleic acid
      (C) STRANDEDNESS: unknown
      (D) TOPOLOGY: unknown
   (ii) MOLECULE TYPE: cDNA
   (ix) FEATURE:
      (A) NAME/KEY: CDS
      (B) LOCATION: 1..495
   (xi) SEQUENCE DESCRIPTION: SEQ ID NO:3:
   ATG TCT AAT GAT ATG ACT CCG GAA CAG ATG GCT ACC AAC GTT AAC TCC      48
   Met Ser Asn Asp Met Thr Pro Glu Gln Met Ala Thr Asn Val Asn Ser
    1               5                  10                  15
   TCC TCC CCG GAA CGT CAC ACG CGT TCC TAC GAC TAC ATG GAA GGT GGT      96
   Ser Ser Pro Glu Arg His Thr Arg Ser Tyr Asp Tyr Met Glu Gly Gly
               20                  25                  30
   GAC ATC CGC GTA CGT CGT CTG TTC TGC CGT ACC CAG TGG TAC CTG CGT     144
   Asp Ile Arg Val Arg Arg Leu Phe Cys Arg Thr Gln Trp Tyr Leu Arg
           35                  40                  45
   ATC GAC AAA CGC GGC AAA GTC AAG GGC ACC CAA GAG ATG AAA AAC AAC     192
   Ile Asp Lys Arg Gly Lys Val Lys Gly Thr Gln Glu Met Lys Asn Asn
       50                  55                  60
   TAC AAT ATT ATG GAA ATC CGT ACT GTT GCT GTT GGT ATC GTT GCA ATC     240
   Tyr Asn Ile Met Glu Ile Arg Thr Val Ala Val Gly Ile Val Ala Ile
   65                  70                  75                  80
   AAA GGT GTT GAA TCT GAA TTC TAC CTG GCA ATG AAC AAA GAA GGT AAA     288
   Lys Gly Val Glu Ser Glu Phe Tyr Leu Ala Met Asn Lys Glu Gly Lys
                   85                  90                  95
   CTG TAC GCA AAA AAA GAA TGC AAC GAA GAC TGC AAC TTC AAA GAA CTG     336
   Leu Tyr Ala Lys Lys Glu Cys Asn Glu Asp Cys Asn Phe Lys Glu Leu
               100                 105                 110
   ATC CTG GAA AAC CAC TAC AAC ACC TAC GCA TCT GCT AAA TGG ACC CAC     384
   Ile Leu Glu Asn His Tyr Asn Thr Tyr Ala Ser Ala Lys Trp Thr His
           115                 120                 125
   AAC GGT GGT GAA ATG TTC GTT GCT CTG AAC CAG AAA GGT ATC CCG GTT     432
   Asn Gly Gly Glu Met Phe Val Ala Leu Asn Gln Lys Gly Ile Pro Val
       130                 135                 140
   CGT GGT AAA AAA ACC AAA AAA GAA CAG AAA ACC GCT CAC TTC CTG CCG     480
   Arg Gly Lys Lys Thr Lys Lys Glu Gln Lys Thr Ala His Phe Leu Pro
   145                 150                 155                 160
   ATG GCA ATC ACT TAA                                                 495
   Met Ala Ile Thr  *
                   165
(2) INFORMATION FOR SEQ ID NO:4:
   (i) SEQUENCE CHARACTERISTICS:
      (A) LENGTH: 165 amino acids
      (B) TYPE: amino acid
      (D) TOPOLOGY: linear
   (ii) MOLECULE TYPE: protein
   (xi) SEQUENCE DESCRIPTION: SEQ ID NO:4:
   Met Ser Asn Asp Met Thr Pro Glu Gln Met Ala Thr Asn Val Asn Ser
    1               5                  10                  15
   Ser Ser Pro Glu Arg His Thr Arg Ser Tyr Asp Tyr Met Glu Gly Gly
               20                  25                  30
   Asp Ile Arg Val Arg Arg Leu Phe Cys Arg Thr Gln Trp Tyr Leu Arg
           35                  40                  45
   Ile Asp Lys Arg Gly Lys Val Lys Gly Thr Gln Glu Met Lys Asn Asn
       50                  55                  60
   Tyr Asn Ile Met Glu Ile Arg Thr Val Ala Val Gly Ile Val Ala Ile
   65                  70                  75                  80
   Lys Gly Val Glu Ser Glu Phe Tyr Leu Ala Met Asn Lys Glu Gly Lys
                   85                  90                  95
   Leu Tyr Ala Lys Lys Glu Cys Asn Glu Asp Cys Asn Phe Lys Glu Leu
               100                 105                 110
   Ile Leu Glu Asn His Tyr Asn Thr Tyr Ala Ser Ala Lys Trp Thr His
           115                 120                 125
   Asn Gly Gly Glu Met Phe Val Ala Leu Asn Gln Lys Gly Ile Pro Val
       130                 135                 140
   Arg Gly Lys Lys Thr Lys Lys Glu Gln Lys Thr Ala His Phe Leu Pro
   145                 150                 155                 160
   Met Ala Ile Thr  *
                   165
(2) INFORMATION FOR SEQ ID NO:5:
   (i) SEQUENCE CHARACTERISTICS:
      (A) LENGTH: 483 base pairs
      (B) TYPE: nucleic acid
      (C) STRANDEDNESS: unknown
      (D) TOPOLOGY: unknown
   (ii) MOLECULE TYPE: cDNA
   (ix) FEATURE:
      (A) NAME/KEY: CDS
      (B) LOCATION: 1..483
   (xi) SEQUENCE DESCRIPTION: SEQ ID NO:5:
   ATG ACT CCG GAA CAG ATG GCT ACC AAC GTT AAC TCC TCC TCC CCG GAA        48
   Met Thr Pro Glu Gln Met Ala Thr Asn Val Asn Ser Ser Ser Pro Glu
    1               5                  10                  15
   CGT CAC ACG CGT TCC TAC GAC TAC ATG GAA GGT GGT GAC ATC CGC GTA        96
   Arg His Thr Arg Ser Tyr Asp Tyr Met Glu Gly Gly Asp Ile Arg Val
               20                  25                  30
   CGT CGT CTG TTC TGC CGT ACC CAG TGG TAC CTG CGT ATC GAC AAA CGC       144
   Arg Arg Leu Phe Cys Arg Thr Gln Trp Tyr Leu Arg Ile Asp Lys Arg
           35                  40                  45
   GGC AAA GTC AAG GGC ACC CAA GAG ATG AAA AAC AAC TAC AAT ATT ATG       192
   Gly Lys Val Lys Gly Thr Gln Glu Met Lys Asn Asn Tyr Asn Ile Met
        50                  55                  60
   GAA ATC CGT ACT GTT GCT GTT GGT ATC GTT GCA ATC AAA GGT GTT GAA       240
   Glu Ile Arg Thr Val Ala Val Gly Ile Val Ala Ile Lys Gly Val Glu
    65                  70                  75                  80
   TCT GAA TTC TAC CTG GCA ATG AAC AAA GAA GGT AAA CTG TAC GCA AAA       288
   Ser Glu Phe Tyr Leu Ala Met Asn Lys Glu Gly Lys Leu Tyr Ala Lys
                   85                  90                  95
   AAA GAA TGC AAC GAA GAC TGC AAC TTC AAA GAA CTG ATC CTG GAA AAC       336
   Lys Glu Cys Asn Glu Asp Cys Asn Phe Lys Glu Leu Ile Leu Glu Asn
               100                 105                 110
   CAC TAC AAC ACC TAC GCA TCT GCT AAA TGG ACC CAC AAC GGT GGT GAA       384
   His Tyr Asn Thr Tyr Ala Ser Ala Lys Trp Thr His Asn Gly Gly Glu
           115                 120                 125
   ATG TTC GTT GCT CTG AAC CAG AAA GGT ATC CCG GTT CGT GGT AAA AAA       432
   Met Phe Val Ala Leu Asn Gln Lys Gly Ile Pro Val Arg Gly Lys Lys
       130                 135                 140
   ACC AAA AAA GAA CAG AAA ACC GCT CAC TTC CTG CCG ATG GCA ATC ACT       480
   Thr Lys Lys Glu Gln Lys Thr Ala His Phe Leu Pro Met Ala Ile Thr
   145                 150                 155                 160
   TAA                                                                   483
    *
(2) INFORMATION FOR SEQ ID NO:6:
   (i) SEQUENCE CHARACTERISTICS:
      (A) LENGTH: 161 amino acids
      (B) TYPE: amino acid
      (D) TOPOLOGY: linear
   (ii) MOLECULE TYPE: protein
   (xi) SEQUENCE DESCRIPTION: SEQ ID NO:6:
   Met Thr Pro Glu Gln Met Ala Thr Asn Val Asn Ser Ser Ser Pro Glu
     1               5                  10                  15
   Arg His Thr Arg Ser Tyr Asp Tyr Met Glu Gly Gly Asp Ile Arg Val
                20                  25                  30
   Arg Arg Leu Phe Cys Arg Thr Gln Trp Tyr Leu Arg Ile Asp Lys Arg
            35                  40                  45
   Gly Lys Val Lys Gly Thr Gln Glu Met Lys Asn Asn Tyr Asn Ile Met
        50                  55                  60
   Glu Ile Arg Thr Val Ala Val Gly Ile Val Ala Ile Lys Gly Val Glu
    65                  70                  75                  80
   Ser Glu Phe Tyr Leu Ala Met Asn Lys Glu Gly Lys Leu Tyr Ala Lys
                    85                  90                  95
   Lys Glu Cys Asn Glu Asp Cys Asn Phe Lys Glu Leu Ile Leu Glu Asn
               100                 105                 110
   His Tyr Asn Thr Tyr Ala Ser Ala Lys Trp Thr His Asn Gly Gly Glu
           115                 120                 125
   Met Phe Val Ala Leu Asn Gln Lys Gly Ile Pro Val Arg Gly Lys Lys
       130                 135                 140
   Thr Lys Lys Glu Gln Lys Thr Ala His Phe Leu Pro Met Ala Ile Thr
   145                 150                 155                 160
   *
(2) INFORMATION FOR SEQ ID NO:7:
   (i) SEQUENCE CHARACTERISTICS:
      (A) LENGTH: 480 base pairs
      (B) TYPE: nucleic acid
      (C) STRANDEDNESS: unknown
      (D) TOPOLOGY: unknown
   (ii) MOLECULE TYPE: cDNA
   (ix) FEATURE:
      (A) NAME/KEY: CDS
      (B) LOCATION: 1..480
   (xi) SEQUENCE DESCRIPTION: SEQ ID NO:7:
   ATG ACT CCG GAA CAG ATG GCT ACC AAC GTT AAC TCT TCT CCT GAA CGT      48
   Met Thr Pro Glu Gln Met Ala Thr Asn Val Asn Ser Ser Pro Glu Arg
    1               5                  10                  15
   CAT ACG CGT TCC TAC GAC TAC ATG GAA GGT GGT GAC ATC CGC GTA CGT      96
   His Thr Arg Ser Tyr Asp Tyr Met Glu Gly Gly Asp Ile Arg Val Arg
               20                  25                  30
   CGT CTG TTC TGC CGT ACC CAG TGG TAC CTG CGT ATC GAC AAA CGC GGC     144
   Arg Leu Phe Cys Arg Thr Gln Trp Tyr Leu Arg Ile Asp Lys Arg Gly
           35                  40                  45
   AAA GTC AAG GGC ACC CAA GAG ATG AAA AAC AAC TAC AAT ATT ATG GAA     192
   Lys Val Lys Gly Thr Gln Glu Met Lys Asn Asn Tyr Asn Ile Met Glu
       50                  55                  60
   ATC CGT ACT GTT GCT GTT GGT ATC GTT GCA ATC AAA GGT GTT GAA TCT     240
   Ile Arg Thr Val Ala Val Gly Ile Val Ala Ile Lys Gly Val Glu Ser
   65                  70                  75                  80
   GAA TTC TAC CTG GCA ATG AAC AAA GAA GGT AAA CTG TAC GCA AAA AAA     288
   Glu Phe Tyr Leu Ala Met Asn Lys Glu Gly Lys Leu Tyr Ala Lys Lys
                   85                  90                  95
   GAA TGC AAC GAA GAC TGC AAC TTC AAA GAA CTG ATC CTG GAA AAC CAC     336
   Glu Cys Asn Glu Asp Cys Asn Phe Lys Glu Leu Ile Leu Glu Asn His
               100                 105                 110
   TAC AAC ACC TAC GCA TCT GCT AAA TGG ACC CAC AAC GGT GGT GAA ATG     384
   Tyr Asn Thr Tyr Ala Ser Ala Lys Trp Thr His Asn Gly Gly Glu Met
           115                 120                 125
   TTC GTT GCT CTG AAC CAG AAA GGT ATC CCG GTT CGT GGT AAA AAA ACC     432
   Phe Val Ala Leu Asn Gln Lys Gly Ile Pro Val Arg Gly Lys Lys Thr
       130                 135                 140
   AAA AAA GAA CAG AAA ACC GCT CAC TTC CTG CCG ATG GCA ATC ACT TAA     480
   Lys Lys Glu Gln Lys Thr Ala His Phe Leu Pro Met Ala Ile Thr  *
   145                 150                 155                 160
(2) INFORMATION FOR SEQ ID NO:8:
   (i) SEQUENCE CHARACTERISTICS:
      (A) LENGTH: 160 amino acids
      (B) TYPE: amino acid
      (D) TOPOLOGY: linear
   (ii) MOLECULE TYPE: protein
   (xi) SEQUENCE DESCRIPTION: SEQ ID NO:8:
   Met Thr Pro Glu Gln Met Ala Thr Asn Val Asn Ser Ser Pro Glu Arg
    1               5                  10                  15
   His Thr Arg Ser Tyr Asp Tyr Met Glu Gly Gly Asp Ile Arg Val Arg
               20                  25                  30
   Arg Leu Phe Cys Arg Thr Gln Trp Tyr Leu Arg Ile Asp Lys Arg Gly
           35                  40                  45
   Lys Val Lys Gly Thr Gln Glu Met Lys Asn Asn Tyr Asn Ile Met Glu
       50                  55                  60
   Ile Arg Thr Val Ala Val Gly Ile Val Ala Ile Lys Gly Val Glu Ser
   65                  70                  75                  80
   Glu Phe Tyr Leu Ala Met Asn Lys Glu Gly Lys Leu Tyr Ala Lys Lys
                   85                  90                  95
   Glu Cys Asn Glu Asp Cys Asn Phe Lys Glu Leu Ile Leu Glu Asn His
               100                 105                 110
   Tyr Asn Thr Tyr Ala Ser Ala Lys Trp Thr His Asn Gly Gly Glu Met
           115                 120                 125
   Phe Val Ala Leu Asn Gln Lys Gly Ile Pro Val Arg Gly Lys Lys Thr
       130                 135                 140
   Lys Lys Glu Gln Lys Thr Ala His Phe Leu Pro Met Ala Ile Thr  *
   145                 150                 155                 160
(2) INFORMATION FOR SEQ ID NO:9:
   (i) SEQUENCE CHARACTERISTICS:
      (A) LENGTH: 468 base pairs
      (B) TYPE: nucleic acid
      (C) STRANDEDNESS: unknown
      (D) TOPOLOGY: unknown
   (ii) MOLECULE TYPE: cDNA
   (ix) FEATURE:
      (A) NAME/KEY: CDS
      (B) LOCATION: 1..468
   (xi) SEQUENCE DESCRIPTION: SEQ ID NO:9:
   ATG GCT ACT AAT GTT AAC TCC TCT TCT CCT GAA CGT CAT ACG CGT TCC          48
   Met Ala Thr Asn Val Asn Ser Ser Ser Pro Glu Arg His Thr Arg Ser
    1               5                  10                  15
   TAC GAC TAC ATG GAA GGT GGT GAC ATC CGC GTA CGT CGT CTG TTC TGC          96
   Tyr Asp Tyr Met Glu Gly Gly Asp Ile Arg Val Arg Arg Leu Phe Cys
               20                  25                  30
   CGT ACC CAG TGG TAC CTG CGT ATC GAC AAA CGC GGC AAA GTC AAG GGC         144
   Arg Thr Gln Trp Tyr Leu Arg Ile Asp Lys Arg Gly Lys Val Lys Gly
           35                  40                  45
   ACC CAA GAG ATG AAA AAC AAC TAC AAT ATT ATG GAA ATC CGT ACT GTT         192
   Thr Gln Glu Met Lys Asn Asn Tyr Asn Ile Met Glu Ile Arg Thr Val
       50                  55                  60
   GCT GTT GGT ATC GTT GCA ATC AAA GGT GTT GAA TCT GAA TTC TAC CTG         240
   Ala Val Gly Ile Val Ala Ile Lys Gly Val Glu Ser Glu Phe Tyr Leu
    65                  70                  75                  80
   GCA ATG AAC AAA GAA GGT AAA CTG TAC GCA AAA AAA GAA TGC AAC GAA         288
   Ala Met Asn Lys Glu Gly Lys Leu Tyr Ala Lys Lys Glu Cys Asn Glu
                   85                  90                  95
   GAC TGC AAC TTC AAA GAA CTG ATC CTG GAA AAC CAC TAC AAC ACC TAC         336
   Asp Cys Asn Phe Lys Glu Leu Ile Leu Glu Asn His Tyr Asn Thr Tyr
               100                 105                 110
   GCA TCT GCT AAA TGG ACC CAC AAC GGT GGT GAA ATG TTC GTT GCT CTG         384
   Ala Ser Ala Lys Trp Thr His Asn Gly Gly Glu Met Phe Val Ala Leu
           115                 120                 125
   AAC CAG AAA GGT ATC CCG GTT CGT GGT AAA AAA ACC AAA AAA GAA CAG         432
   Asn Gln Lys Gly Ile Pro Val Arg Gly Lys Lys Thr Lys Lys Glu Gln
       130                 135                 140
   AAA ACC GCT CAC TTC CTG CCG ATG GCA ATC ACT TAA                         468
   Lys Thr Ala His Phe Leu Pro Met Ala Ile Thr  *
   145                 150                 155
(2) INFORMATION FOR SEQ ID NO:10:
   (i) SEQUENCE CHARACTERISTICS:
      (A) LENGTH: 156 amino acids
      (B) TYPE: amino acid
      (D) TOPOLOGY: linear
   (ii) MOLECULE TYPE: protein
   (xi) SEQUENCE DESCRIPTION: SEQ ID NO:10:
   Met Ala Thr Asn Val Asn Ser Ser Ser Pro Glu Arg His Thr Arg Ser
    1               5                  10                  15
   Tyr Asp Tyr Met Glu Gly Gly Asp Ile Arg Val Arg Arg Leu Phe Cys
               20                  25                  30
   Arg Thr Gln Trp Tyr Leu Arg Ile Asp Lys Arg Gly Lys Val Lys Gly
           35                  40                  45
   Thr Gln Glu Met Lys Asn Asn Tyr Asn Ile Met Glu Ile Arg Thr Val
       50                  55                  60
   Ala Val Gly Ile Val Ala Ile Lys Gly Val Glu Ser Glu Phe Tyr Leu
   65                  70                  75                  80
   Ala Met Asn Lys Glu Gly Lys Leu Tyr Ala Lys Lys Glu Cys Asn Glu
                   85                  90                  95
   Asp Cys Asn Phe Lys Glu Leu Ile Leu Glu Asn His Tyr Asn Thr Tyr
               100                 105                 110
   Ala Ser Ala Lys Trp Thr His Asn Gly Gly Glu Met Phe Val Ala Leu
           115                 120                 125
   Asn Gln Lys Gly Ile Pro Val Arg Gly Lys Lys Thr Lys Lys Glu Gln
       130                 135                 140
   Lys Thr Ala His Phe Leu Pro Met Ala Ile Thr  *
   145                 150                 155
(2) INFORMATION FOR SEQ ID NO:11:
   (i) SEQUENCE CHARACTERISTICS:
      (A) LENGTH: 465 base pairs
      (B) TYPE: nucleic acid
      (C) STRANDEDNESS: unknown
      (D) TOPOLOGY: unknown
   (ii) MOLECULE TYPE: cDNA
   (ix) FEATURE:
      (A) NAME/KEY: CDS
      (B) LOCATION: 1..465
   (xi) SEQUENCE DESCRIPTION: SEQ ID NO:11:
   ATG GCT ACT AAT GTT AAC TCT TCT CCT GAA CGT CAT ACG CGT TCC TAC       48
   Met Ala Thr Asn Val Asn Ser Ser Pro Glu Arg His Thr Arg Ser Tyr
    1               5                  10                  15
   GAC TAC ATG GAA GGT GGT GAC ATC CGC GTA CGT CGT CTG TTC TGC CGT       96
   Asp Tyr Met Glu Gly Gly Asp Ile Arg Val Arg Arg Leu Phe Cys Arg
               20                  25                  30
   ACC CAG TGG TAC CTG CGT ATC GAC AAA CGC GGC AAA GTC AAG GGC ACC      144
   Thr Gln Trp Tyr Leu Arg Ile Asp Lys Arg Gly Lys Val Lys Gly Thr
           35                  40                  45
   CAA GAG ATG AAA AAC AAC TAC AAT ATT ATG GAA ATC CGT ACT GTT GCT      192
   Gln Glu Met Lys Asn Asn Tyr Asn Ile Met Glu Ile Arg Thr Val Ala
       50                  55                  60
   GTT GGT ATC GTT GCA ATC AAA GGT GTT GAA TCT GAA TTC TAC CTG GCA      240
   Val Gly Ile Val Ala Ile Lys Gly Val Glu Ser Glu Phe Tyr Leu Ala
   65                  70                  75                  80
   ATG AAC AAA GAA GGT AAA CTG TAC GCA AAA AAA GAA TGC AAC GAA GAC      288
   Met Asn Lys Glu Gly Lys Leu Tyr Ala Lys Lys Glu Cys Asn Glu Asp
                   85                  90                  95
   TGC AAC TTC AAA GAA CTG ATC CTG GAA AAC CAC TAC AAC ACC TAC GCA      336
   Cys Asn Phe Lys Glu Leu Ile Leu Glu Asn His Tyr Asn Thr Tyr Ala
               100                 105                 110
   TCT GCT AAA TGG ACC CAC AAC GGT GGT GAA ATG TTC GTT GCT CTG AAC      384
   Ser Ala Lys Trp Thr His Asn Gly Gly Glu Met Phe Val Ala Leu Asn
           115                 120                 125
   CAG AAA GGT ATC CCG GTT CGT GGT AAA AAA ACC AAA AAA GAA CAG AAA      432
   Gln Lys Gly Ile Pro Val Arg Gly Lys Lys Thr Lys Lys Glu Gln Lys
       130                 135                 140
   ACC GCT CAC TTC CTG CCG ATG GCA ATC ACT TAA                          465
   Thr Ala His Phe Leu Pro Met Ala Ile Thr  *
   145                 150                  155
(2) INFORMATION FOR SEQ ID NO:12:
   (i) SEQUENCE CHARACTERISTICS:
      (A) LENGTH: 155 amino acids
      (B) TYPE: amino acid
      (D) TOPOLOGY: linear
   (ii) MOLECULE TYPE: protein
   (xi) SEQUENCE DESCRIPTION: SEQ ID NO:12:
   Met Ala Thr Asn Val Asn Ser Ser Pro Glu Arg His Thr Arg Ser Tyr
    1               5                  10                  15
   Asp Tyr Met Glu Gly Gly Asp Ile Arg Val Arg Arg Leu Phe Cys Arg
               20                  25                  30
   Thr Gln Trp Tyr Leu Arg Ile Asp Lys Arg Gly Lys Val Lys Gly Thr
           35                  40                  45
   Gln Glu Met Lys Asn Asn Tyr Asn Ile Met Glu Ile Arg Thr Val Ala
       50                  55                  60
   Val Gly Ile Val Ala Ile Lys Gly Val Glu Ser Glu Phe Tyr Leu Ala
   65                  70                  75                  80
   Met Asn Lys Glu Gly Lys Leu Tyr Ala Lys Lys Glu Cys Asn Glu Asp
                   85                  90                  95
   Cys Asn Phe Lys Glu Leu Ile Leu Glu Asn His Tyr Asn Thr Tyr Ala
               100                 105                 110
   Ser Ala Lys Trp Thr His Asn Gly Gly Glu Met Phe Val Ala Leu Asn
           115                 120                 125
   Gln Lys Gly Ile Pro Val Arg Gly Lys Lys Thr Lys Lys Glu Gln Lys
       130                 135                 140
   Thr Ala His Phe Leu Pro Met Ala Ile Thr  *
   145                 150                 155
(2) INFORMATION FOR SEQ ID NO:13:
   (i) SEQUENCE CHARACTERISTICS:
      (A) LENGTH: 450 base pairs
      (B) TYPE: nucleic acid
      (C) STRANDEDNESS: unknown
      (D) TOPOLOGY: unknown
   (ii) MOLECULE TYPE: cDNA
   (ix) FEATURE:
      (A) NAME/KEY: CDS
      (B) LOCATION: 1..450
   (xi) SEQUENCE DESCRIPTION: SEQ ID NO:13:
   ATG TCT TCT CCT GAA CGT CAT ACG CGT TCC TAC GAC TAC ATG GAA GGT      48
   Met Ser Ser Pro Glu Arg His Thr Arg Ser Tyr Asp Tyr Met Glu Gly
     1               5                  10                  15
   GGT GAC ATC CGC GTA CGT CGT CTG TTC TGC CGT ACC CAG TGG TAC CTG      96
   Gly Asp Ile Arg Val Arg Arg Leu Phe Cys Arg Thr Gln Trp Tyr Leu
                20                  25                  30
   CGT ATC GAC AAA CGC GGC AAA GTC AAG GGC ACC CAA GAG ATG AAA AAC     144
   Arg Ile Asp Lys Arg Gly Lys Val Lys Gly Thr Gln Glu Met Lys Asn
            35                  40                  45
   AAC TAC AAT ATT ATG GAA ATC CGT ACT GTT GCT GTT GGT ATC GTT GCA     192
   Asn Tyr Asn Ile Met Glu Ile Arg Thr Val Ala Val Gly Ile Val Ala
        50                  55                  60
   ATC AAA GGT GTT GAA TCT GAA TTC TAC CTG GCA ATG AAC AAA GAA GGT     240
   Ile Lys Gly Val Glu Ser Glu Phe Tyr Leu Ala Met Asn Lys Glu Gly
    65                  70                  75                  80
   AAA CTG TAC GCA AAA AAA GAA TGC AAC GAA GAC TGC AAC TTC AAA GAA     288
   Lys Leu Tyr Ala Lys Lys Glu Cys Asn Glu Asp Cys Asn Phe Lys Glu
                    85                  90                  95
   CTG ATC CTG GAA AAC CAC TAC AAC ACC TAC GCA TCT GCT AAA TGG ACC     336
   Leu Ile Leu Glu Asn His Tyr Asn Thr Tyr Ala Ser Ala Lys Trp Thr
               100                 105                 110
   CAC AAC GGT GGT GAA ATG TTC GTT GCT CTG AAC CAG AAA GGT ATC CCG     384
   His Asn Gly Gly Glu Met Phe Val Ala Leu Asn Gln Lys Gly Ile Pro
           115                 120                 125
   GTT CGT GGT AAA AAA ACC AAA AAA GAA CAG AAA ACC GCT CAC TTC CTG     432
   Val Arg Gly Lys Lys Thr Lys Lys Glu Gln Lys Thr Ala His Phe Leu
       130                 135                 140
   CCG ATG GCA ATC ACT TAA                                             450
   Pro Met Ala Ile Thr  *
   145                 150
(2) INFORMATION FOR SEQ ID NO:14:
   (i) SEQUENCE CHARACTERISTICS:
      (A) LENGTH: 150 amino acids
      (B) TYPE: amino acid
      (D) TOPOLOGY: linear
   (ii) MOLECULE TYPE: protein
   (xi) SEQUENCE DESCRIPTION: SEQ ID NO:14:
   Met Ser Ser Pro Glu Arg His Thr Arg Ser Tyr Asp Tyr Met Glu Gly
    1               5                  10                  15
   Gly Asp Ile Arg Val Arg Arg Leu Phe Cys Arg Thr Gln Trp Tyr Leu
               20                  25                  30
   Arg Ile Asp Lys Arg Gly Lys Val Lys Gly Thr Gln Glu Met Lys Asn
           35                  40                  45
   Asn Tyr Asn Ile Met Glu Ile Arg Thr Val Ala Val Gly Ile Val Ala
       50                  55                  60
   Ile Lys Gly Val Glu Ser Glu Phe Tyr Leu Ala Met Asn Lys Glu Gly
   65                  70                  75                  80
   Lys Leu Tyr Ala Lys Lys Glu Cys Asn Glu Asp Cys Asn Phe Lys Glu
                   85                  90                  95
   Leu Ile Leu Glu Asn His Tyr Asn Thr Tyr Ala Ser Ala Lys Trp Thr
               100                 105                 110
   His Asn Gly Gly Glu Met Phe Val Ala Leu Asn Gln Lys Gly Ile Pro
           115                 120                 125
   Val Arg Gly Lys Lys Thr Lys Lys Glu Gln Lys Thr Ala His Phe Leu
       130                 135                 140
   Pro Met Ala Ile Thr  *
   145                 150
(2) INFORMATION FOR SEQ ID NO:15:
   (i) SEQUENCE CHARACTERISTICS:
      (A) LENGTH: 447 base pairs
      (B) TYPE: nucleic acid
      (C) STRANDEDNESS: unknown
      (D) TOPOLOGY: unknown
   (ii) MOLECULE TYPE: cDNA
   (ix) FEATURE:
      (A) NAME/KEY: CDS
      (B) LOCATION: 1..447
   (xi) SEQUENCE DESCRIPTION: SEQ ID NO:15:
   ATG TCT CCT GAA CGT CAT ACG CGT TCC TAC GAC TAC ATG GAA GGT GGT         48
   Met Ser Pro Glu Arg His Thr Arg Ser Tyr Asp Tyr Met Glu Gly Gly
    1               5                  10                  15
   GAC ATC CGC GTA CGT CGT CTG TTC TGC CGT ACC CAG TGG TAC CTG CGT          96
   Asp Ile Arg Val Arg Arg Leu Phe Cys Arg Thr Gln Trp Tyr Leu Arg
               20                  25                  30
   ATC GAC AAA CGC GGC AAA GTC AAG GGC ACC CAA GAG ATG AAA AAC AAC         144
   Ile Asp Lys Arg Gly Lys Val Lys Gly Thr Gln Glu Met Lys Asn Asn
           35                  40                  45
   TAC AAT ATT ATG GAA ATC CGT ACT GTT GCT GTT GGT ATC GTT GCA ATC         192
   Tyr Asn Ile Met Glu Ile Arg Thr Val Ala Val Gly Ile Val Ala Ile
       50                  55                  60
   AAA GGT GTT GAA TCT GAA TTC TAC CTG GCA ATG AAC AAA GAA GGT AAA         240
   Lys Gly Val Glu Ser Glu Phe Tyr Leu Ala Met Asn Lys Glu Gly Lys
    65                  70                  75                  80
   CTG TAC GCA AAA AAA GAA TGC AAC GAA GAC TGC AAC TTC AAA GAA CTG         288
   Leu Tyr Ala Lys Lys Glu Cys Asn Glu Asp Cys Asn Phe Lys Glu Leu
                   85                  90                  95
   ATC CTG GAA AAC CAC TAC AAC ACC TAC GCA TCT GCT AAA TGG ACC CAC         336
   Ile Leu Glu Asn His Tyr Asn Thr Tyr Ala Ser Ala Lys Trp Thr His
               100                 105                 110
   AAC GGT GGT GAA ATG TTC GTT GCT CTG AAC CAG AAA GGT ATC CCG GTT         384
   Asn Gly Gly Glu Met Phe Val Ala Leu Asn Gln Lys Gly Ile Pro Val
           115                 120                 125
   CGT GGT AAA AAA ACC AAA AAA GAA CAG AAA ACC GCT CAC TTC CTG CCG         432
   Arg Gly Lys Lys Thr Lys Lys Glu Gln Lys Thr Ala His Phe Leu Pro
       130                 135                 140
   ATG GCA ATC ACT TAA                                                     447
   Met Ala Ile Thr  *
   145
(2) INFORMATION FOR SEQ ID NO:16:
   (i) SEQUENCE CHARACTERISTICS:
      (A) LENGTH: 149 amino acids
      (B) TYPE: amino acid
      (D) TOPOLOGY: linear
   (ii) MOLECULE TYPE: protein
   (xi) SEQUENCE DESCRIPTION: SEQ ID NO:16:
   Met Ser Pro Glu Arg His Thr Arg Ser Tyr Asp Tyr Met Glu Gly Gly
    1               5                  10                  15
   Asp Ile Arg Val Arg Arg Leu Phe Cys Arg Thr Gln Trp Tyr Leu Arg
               20                  25                  30
   Ile Asp Lys Arg Gly Lys Val Lys Gly Thr Gln Glu Met Lys Asn Asn
           35                  40                  45
   Tyr Asn Ile Met Glu Ile Arg Thr Val Ala Val Gly Ile Val Ala Ile
       50                  55                  60
   Lys Gly Val Glu Ser Glu Phe Tyr Leu Ala Met Asn Lys Glu Gly Lys
    65                  70                  75                  80
   Leu Tyr Ala Lys Lys Glu Cys Asn Glu Asp Cys Asn Phe Lys Glu Leu
                   85                  90                  95
   Ile Leu Glu Asn His Tyr Asn Thr Tyr Ala Ser Ala Lys Trp Thr His
               100                 105                 110
   Asn Gly Gly Glu Met Phe Val Ala Leu Asn Gln Lys Gly Ile Pro Val
           115                 120                 125
   Arg Gly Lys Lys Thr Lys Lys Glu Gln Lys Thr Ala His Phe Leu Pro
       130                 135                 140
   Met Ala Ile Thr  *
   145
(2) INFORMATION FOR SEQ ID NO:17:
   (i) SEQUENCE CHARACTERISTICS:
      (A) LENGTH: 444 base pairs
      (B) TYPE: nucleic acid
      (C) STRANDEDNESS: unknown
      (D) TOPOLOGY: unknown
   (ii) MOLECULE TYPE: cDNA
   (ix) FEATURE:
      (A) NAME/KEY: CDS
      (B) LOCATION: 1..444
   (xi) SEQUENCE DESCRIPTION: SEQ ID NO:17:
   ATG CCT GAA CGT CAT ACG CGT TCC TAC GAC TAC ATG GAA GGT GGT GAC       48
   Met Pro Glu Arg His Thr Arg Ser Tyr Asp Tyr Met Glu Gly Gly Asp
     1               5                  10                  15
   ATC CGC GTA CGT CGT CTG TTC TGC CGT ACC CAG TGG TAC CTG CGT ATC       96
   Ile Arg Val Arg Arg Leu Phe Cys Arg Thr Gln Trp Tyr Leu Arg Ile
               20                  25                  30
   GAC AAA CGC GGC AAA GTC AAG GGC ACC CAA GAG ATG AAA AAC AAC TAC      144
   Asp Lys Arg Gly Lys Val Lys Gly Thr Gln Glu Met Lys Asn Asn Tyr
           35                  40                  45
   AAT ATT ATG GAA ATC CGT ACT GTT GCT GTT GGT ATC GTT GCA ATC AAA      192
   Asn Ile Met Glu Ile Arg Thr Val Ala Val Gly Ile Val Ala Ile Lys
       50                  55                  60
   GGT GTT GAA TCT GAA TTC TAC CTG GCA ATG AAC AAA GAA GGT AAA CTG      240
   Gly Val Glu Ser Glu Phe Tyr Leu Ala Met Asn Lys Glu Gly Lys Leu
    65                  70                  75                  80
   TAC GCA AAA AAA GAA TGC AAC GAA GAC TGC AAC TTC AAA GAA CTG ATC      288
   Tyr Ala Lys Lys Glu Cys Asn Glu Asp Cys Asn Phe Lys Glu Leu Ile
                    85                  90                  95
   CTG GAA AAC CAC TAC AAC ACC TAC GCA TCT GCT AAA TGG ACC CAC AAC      336
   Leu Glu Asn His Tyr Asn Thr Tyr Ala Ser Ala Lys Trp Thr His Asn
               100                 105                 110
   GGT GGT GAA ATG TTC GTT GCT CTG AAC CAG AAA GGT ATC CCG GTT CGT        384
   Gly Gly Glu Met Phe Val Ala Leu Asn Gln Lys Gly Ile Pro Val Arg
           115                 120                 125
   GGT AAA AAA ACC AAA AAA GAA CAG AAA ACC GCT CAC TTC CTG CCG ATG        432
   Gly Lys Lys Thr Lys Lys Glu Gln Lys Thr Ala His Phe Leu Pro Met
       130                 135                 140
   GCA ATC ACT TAA                                                        444
   Ala Ile Thr  *
   145
(2) INFORMATION FOR SEQ ID NO:18:
   (i) SEQUENCE CHARACTERISTICS:
      (A) LENGTH: 148 amino acids
      (B) TYPE: amino acid
      (D) TOPOLOGY: linear
   (ii) MOLECULE TYPE: protein
   (xi) SEQUENCE DESCRIPTION: SEQ ID NO:18:
   Met Pro Glu Arg His Thr Arg Ser Tyr Asp Tyr Met Glu Gly Gly Asp
    1               5                  10                  15
   Ile Arg Val Arg Arg Leu Phe Cys Arg Thr Gln Trp Tyr Leu Arg Ile
               20                  25                  30
   Asp Lys Arg Gly Lys Val Lys Gly Thr Gln Glu Met Lys Asn Asn Tyr
           35                  40                  45
   Asn Ile Met Glu Ile Arg Thr Val Ala Val Gly Ile Val Ala Ile Lys
       50                  55                  60
   Gly Val Glu Ser Glu Phe Tyr Leu Ala Met Asn Lys Glu Gly Lys Leu
   65                  70                  75                  80
   Tyr Ala Lys Lys Glu Cys Asn Glu Asp Cys Asn Phe Lys Glu Leu Ile
                   85                  90                  95
   Leu Glu Asn His Tyr Asn Thr Tyr Ala Ser Ala Lys Trp Thr His Asn
               100                 105                 110
   Gly Gly Glu Met Phe Val Ala Leu Asn Gln Lys Gly Ile Pro Val Arg
           115                 120                 125
   Gly Lys Lys Thr Lys Lys Glu Gln Lys Thr Ala His Phe Leu Pro Met
       130                 135                 140
   Ala Ile Thr  *
   145
(2) INFORMATION FOR SEQ ID NO:19:
   (i) SEQUENCE CHARACTERISTICS:
      (A) LENGTH: 441 base pairs
      (B) TYPE: nucleic acid
      (C) STRANDEDNESS: unknown
      (D) TOPOLOGY: unknown
   (ii) MOLECULE TYPE: cDNA
   (ix) FEATURE:
      (A) NAME/KEY: CDS
      (B) LOCATION: 1..441
   (xi) SEQUENCE DESCRIPTION: SEQ ID NO:19:
   ATG GAA CGT CAT ACG CGT TCC TAC GAC TAC ATG GAA GGT GGT GAC ATC       48
   Met Glu Arg His Thr Arg Ser Tyr Asp Tyr Met Glu Gly Gly Asp Ile
     1               5                  10                  15
   CGC GTA CGT CGT CTG TTC TGC CGT ACC CAG TGG TAC CTG CGT ATC GAC       96
   Arg Val Arg Arg Leu Phe Cys Arg Thr Gln Trp Tyr Leu Arg Ile Asp
               20                  25                  30
   AAA CGC GGC AAA GTC AAG GGC ACC CAA GAG ATG AAA AAC AAC TAC AAT      144
   Lys Arg Gly Lys Val Lys Gly Thr Gln Glu Met Lys Asn Asn Tyr Asn
           35                  40                  45
   ATT ATG GAA ATC CGT ACT GTT GCT GTT GGT ATC GTT GCA ATC AAA GGT      192
   Ile Met Glu Ile Arg Thr Val Ala Val Gly Ile Val Ala Ile Lys Gly
       50                  55                  60
   GTT GAA TCT GAA TTC TAC CTG GCA ATG AAC AAA GAA GGT AAA CTG TAC      240
   Val Glu Ser Glu Phe Tyr Leu Ala Met Asn Lys Glu Gly Lys Leu Tyr
    65                  70                  75                  80
   GCA AAA AAA GAA TGC AAC GAA GAC TGC AAC TTC AAA GAA CTG ATC CTG      288
   Ala Lys Lys Glu Cys Asn Glu Asp Cys Asn Phe Lys Glu Leu Ile Leu
                    85                  90                  95
   GAA AAC CAC TAC AAC ACC TAC GCA TCT GCT AAA TGG ACC CAC AAC GGT      336
   Glu Asn His Tyr Asn Thr Tyr Ala Ser Ala Lys Trp Thr His Asn Gly
               100                 105                 110
   GGT GAA ATG TTC GTT GCT CTG AAC CAG AAA GGT ATC CCG GTT CGT GGT      384
   Gly Glu Met Phe Val Ala Leu Asn Gln Lys Gly Ile Pro Val Arg Gly
           115                 120                 125
   AAA AAA ACC AAA AAA GAA CAG AAA ACC GCT CAC TTC CTG CCG ATG GCA      432
   Lys Lys Thr Lys Lys Glu Gln Lys Thr Ala His Phe Leu Pro Met Ala
       130                 135                 140
   ATC ACT TAA                                                          441
   Ile Thr  *
   145
(2) INFORMATION FOR SEQ ID NO:20:
   (i) SEQUENCE CHARACTERISTICS:
      (A) LENGTH: 147 amino acids
      (B) TYPE: amino acid
      (D) TOPOLOGY: linear
   (ii) MOLECULE TYPE: protein
   (xi) SEQUENCE DESCRIPTION: SEQ ID NO:20:
   Met Glu Arg His Thr Arg Ser Tyr Asp Tyr Met Glu Gly Gly Asp Ile
    1               5                  10                  15
   Arg Val Arg Arg Leu Phe Cys Arg Thr Gln Trp Tyr Leu Arg Ile Asp
               20                  25                  30
   Lys Arg Gly Lys Val Lys Gly Thr Gln Glu Met Lys Asn Asn Tyr Asn
           35                  40                  45
   Ile Met Glu Ile Arg Thr Val Ala Val Gly Ile Val Ala Ile Lys Gly
       50                  55                  60
   Val Glu Ser Glu Phe Tyr Leu Ala Met Asn Lys Glu Gly Lys Leu Tyr
   65                  70                  75                  80
   Ala Lys Lys Glu Cys Asn Glu Asp Cys Asn Phe Lys Glu Leu Ile Leu
                   85                  90                  95
   Glu Asn His Tyr Asn Thr Tyr Ala Ser Ala Lys Trp Thr His Asn Gly
               100                 105                 110
   Gly Glu Met Phe Val Ala Leu Asn Gln Lys Gly Ile Pro Val Arg Gly
           115                 120                 125
   Lys Lys Thr Lys Lys Glu Gln Lys Thr Ala His Phe Leu Pro Met Ala
       130                 135                 140
   Ile Thr  *
   145
(2) INFORMATION FOR SEQ ID NO:21:
   (i) SEQUENCE CHARACTERISTICS:
      (A) LENGTH: 438 base pairs
      (B) TYPE: nucleic acid
      (C) STRANDEDNESS: unknown
      (D) TOPOLOGY: unknown
   (ii) MOLECULE TYPE: cDNA
   (ix) FEATURE:
      (A) NAME/KEY: CDS
      (B) LOCATION: 1..438
   (xi) SEQUENCE DESCRIPTION: SEQ ID NO:21:
   ATG CGT CAT ACG CGT TCC TAC GAC TAC ATG GAA GGT GGT GAC ATC CGC       48
   Met Arg His Thr Arg Ser Tyr Asp Tyr Met Glu Gly Gly Asp Ile Arg
    1               5                  10                  15
   GTA CGT CGT CTG TTC TGC CGT ACC CAG TGG TAC CTG CGT ATC GAC AAA       96
   Val Arg Arg Leu Phe Cys Arg Thr Gln Trp Tyr Leu Arg Ile Asp Lys
               20                  25                  30
   CGC GGC AAA GTC AAG GGC ACC CAA GAG ATG AAA AAC AAC TAC AAT ATT      144
   Arg Gly Lys Val Lys Gly Thr Gln Glu Met Lys Asn Asn Tyr Asn Ile
           35                  40                  45
   ATG GAA ATC CGT ACT GTT GCT GTT GGT ATC GTT GCA ATC AAA GGT GTT        192
   Met Glu Ile Arg Thr Val Ala Val Gly Ile Val Ala Ile Lys Gly Val
       50                  55                  60
   GAA TCT GAA TTC TAC CTG GCA ATG AAC AAA GAA GGT AAA CTG TAC GCA        240
   Glu Ser Glu Phe Tyr Leu Ala Met Asn Lys Glu Gly Lys Leu Tyr Ala
    65                  70                  75                  80
   AAA AAA GAA TGC AAC GAA GAC TGC AAC TTC AAA GAA CTG ATC CTG GAA        288
   Lys Lys Glu Cys Asn Glu Asp Cys Asn Phe Lys Glu Leu Ile Leu Glu
                    85                  90                  95
   AAC CAC TAC AAC ACC TAC GCA TCT GCT AAA TGG ACC CAC AAC GGT GGT        336
   Asn His Tyr Asn Thr Tyr Ala Ser Ala Lys Trp Thr His Asn Gly Gly
               100                 105                 110
   GAA ATG TTC GTT GCT CTG AAC CAG AAA GGT ATC CCG GTT CGT GGT AAA        384
   Glu Met Phe Val Ala Leu Asn Gln Lys Gly Ile Pro Val Arg Gly Lys
           115                 120                 125
   AAA ACC AAA AAA GAA CAG AAA ACC GCT CAC TTC CTG CCG ATG GCA ATC        432
   Lys Thr Lys Lys Glu Gln Lys Thr Ala His Phe Leu Pro Met Ala Ile
       130                 135                 140
   ACT TAA                                                                438
   Thr  *
   145
(2) INFORMATION FOR SEQ ID NO:22:
   (i) SEQUENCE CHARACTERISTICS:
      (A) LENGTH: 146 amino acids
      (B) TYPE: amino acid
      (D) TOPOLOGY: linear
   (ii) MOLECULE TYPE: protein
   (xi) SEQUENCE DESCRIPTION: SEQ ID NO:22:
   Met Arg His Thr Arg Ser Tyr Asp Tyr Met Glu Gly Gly Asp Ile Arg
    1               5                  10                  15
   Val Arg Arg Leu Phe Cys Arg Thr Gln Trp Tyr Leu Arg Ile Asp Lys
               20                  25                  30
   Arg Gly Lys Val Lys Gly Thr Gln Glu Met Lys Asn Asn Tyr Asn Ile
           35                  40                  45
   Met Glu Ile Arg Thr Val Ala Val Gly Ile Val Ala Ile Lys Gly Val
       50                  55                  60
   Glu Ser Glu Phe Tyr Leu Ala Met Asn Lys Glu Gly Lys Leu Tyr Ala
    65                  70                  75                  80
   Lys Lys Glu Cys Asn Glu Asp Cys Asn Phe Lys Glu Leu Ile Leu Glu
                    85                  90                  95
   Asn His Tyr Asn Thr Tyr Ala Ser Ala Lys Trp Thr His Asn Gly Gly
               100                 105                 110
   Glu Met Phe Val Ala Leu Asn Gln Lys Gly Ile Pro Val Arg Gly Lys
           115                 120                 125
   Lys Thr Lys Lys Glu Gln Lys Thr Ala His Phe Leu Pro Met Ala Ile
       130                 135                 140
   Thr  *
   145
(2) INFORMATION FOR SEQ ID NO:23:
   (i) SEQUENCE CHARACTERISTICS:
      (A) LENGTH: 435 base pairs
      (B) TYPE: nucleic acid
      (C) STRANDEDNESS: unknown
      (D) TOPOLOGY: unknown
   (ii) MOLECULE TYPE: cDNA
   (ix) FEATURE:
      (A) NAME/KEY: CDS
      (B) LOCATION: 1..435
   (xi) SEQUENCE DESCRIPTION: SEQ ID NO:23:
   ATG CAT ACT CGT TCC TAC GAC TAC ATG GAA GGT GGT GAC ATC CGC GTA        48
   Met His Thr Arg Ser Tyr Asp Tyr Met Glu Gly Gly Asp Ile Arg Val
     1             5                  10                  15
   CGT CGT CTG TTC TGC CGT ACC CAG TGG TAC CTG CGT ATC GAC AAA CGC        96
   Arg Arg Leu Phe Cys Arg Thr Gln Trp Tyr Leu Arg Ile Asp Lys Arg
                20                  25                  30
   GGC AAA GTC AAG GGC ACC CAA GAG ATG AAA AAC AAC TAC AAT ATT ATG       144
   Gly Lys Val Lys Gly Thr Gln Glu Met Lys Asn Asn Tyr Asn Ile Met
            35                  40                  45
   GAA ATC CGT ACT GTT GCT GTT GGT ATC GTT GCA ATC AAA GGT GTT GAA       192
   Glu Ile Arg Thr Val Ala Val Gly Ile Val Ala Ile Lys Gly Val Glu
        50                  55                  60
   TCT GAA TTC TAC CTG GCA ATG AAC AAA GAA GGT AAA CTG TAC GCA AAA       240
   Ser Glu Phe Tyr Leu Ala Met Asn Lys Glu Gly Lys Leu Tyr Ala Lys
    65                  70                  75                  80
   AAA GAA TGC AAC GAA GAC TGC AAC TTC AAA GAA CTG ATC CTG GAA AAC       288
   Lys Glu Cys Asn Glu Asp Cys Asn Phe Lys Glu Leu Ile Leu Glu Asn
                    85                  90                  95
   CAC TAC AAC ACC TAC GCA TCT GCT AAA TGG ACC CAC AAC GGT GGT GAA       336
   His Tyr Asn Thr Tyr Ala Ser Ala Lys Trp Thr His Asn Gly Gly Glu
               100                 105                 110
   ATG TTC GTT GCT CTG AAC CAG AAA GGT ATC CCG GTT CGT GGT AAA AAA       384
   Met Phe Val Ala Leu Asn Gln Lys Gly Ile Pro Val Arg Gly Lys Lys
           115                 120                 125
   ACC AAA AAA GAA CAG AAA ACC GCT CAC TTC CTG CCG ATG GCA ATC ACT       432
   Thr Lys Lys Glu Gln Lys Thr Ala His Phe Leu Pro Met Ala Ile Thr
       130                 135                 140
   TAA                                                                   435
    *
   145
(2) INFORMATION FOR SEQ ID NO:24:
   (i) SEQUENCE CHARACTERISTICS:
      (A) LENGTH: 145 amino acids
      (B) TYPE: amino acid
      (D) TOPOLOGY: linear
   (ii) MOLECULE TYPE: protein
   (xi) SEQUENCE DESCRIPTION: SEQ ID NO:24:
   Met His Thr Arg Ser Tyr Asp Tyr Met Glu Gly Gly Asp Ile Arg Val
    1               5                  10                  15
   Arg Arg Leu Phe Cys Arg Thr Gln Trp Tyr Leu Arg Ile Asp Lys Arg
               20                  25                  30
   Gly Lys Val Lys Gly Thr Gln Glu Met Lys Asn Asn Tyr Asn Ile Met
           35                  40                  45
   Glu Ile Arg Thr Val Ala Val Gly Ile Val Ala Ile Lys Gly Val Glu
       50                  55                  60
   Ser Glu Phe Tyr Leu Ala Met Asn Lys Glu Gly Lys Leu Tyr Ala Lys
   65                  70                  75                  80
   Lys Glu Cys Asn Glu Asp Cys Asn Phe Lys Glu Leu Ile Leu Glu Asn
                   85                  90                  95
   His Tyr Asn Thr Tyr Ala Ser Ala Lys Trp Thr His Asn Gly Gly Glu
               100                 105                 110
   Met Phe Val Ala Leu Asn Gln Lys Gly Ile Pro Val Arg Gly Lys Lys
           115                 120                 125
   Thr Lys Lys Glu Gln Lys Thr Ala His Phe Leu Pro Met Ala Ile Thr
       130                 135                 140
    *
   145
(2) INFORMATION FOR SEQ ID NO:25:
   (i) SEQUENCE CHARACTERISTICS:
      (A) LENGTH: 432 base pairs
      (B) TYPE: nucleic acid
      (C) STRANDEDNESS: unknown
      (D) TOPOLOGY: unknown
   (ii) MOLECULE TYPE: cDNA
   (ix) FEATURE:
      (A) NAME/KEY: CDS
      (B) LOCATION: 1..432
   (xi) SEQUENCE DESCRIPTION: SEQ ID NO:25:
   ATG ACT CGT TCC TAC GAC TAC ATG GAA GGT GGT GAC ATC CGC GTA CGT        48
   Met Thr Arg Ser Tyr Asp Tyr Met Glu Gly Gly Asp Ile Arg Val Arg
     1               5                  10                  15
   CGT CTG TTC TGC CGT ACC CAG TGG TAC CTG CGT ATC GAC AAA CGC GGC        96
   Arg Leu Phe Cys Arg Thr Gln Trp Tyr Leu Arg Ile Asp Lys Arg Gly
                20                  25                  30
   AAA GTC AAG GGC ACC CAA GAG ATG AAA AAC AAC TAC AAT ATT ATG GAA       144
   Lys Val Lys Gly Thr Gln Glu Met Lys Asn Asn Tyr Asn Ile Met Glu
            35                  40                  45
   ATC CGT ACT GTT GCT GTT GGT ATC GTT GCA ATC AAA GGT GTT GAA TCT       192
   Ile Arg Thr Val Ala Val Gly Ile Val Ala Ile Lys Gly Val Glu Ser
        50                  55                  60
   GAA TTC TAC CTG GCA ATG AAC AAA GAA GGT AAA CTG TAC GCA AAA AAA       240
   Glu Phe Tyr Leu Ala Met Asn Lys Glu Gly Lys Leu Tyr Ala Lys Lys
    65                  70                  75                  80
   GAA TGC AAC GAA GAC TGC AAC TTC AAA GAA CTG ATC CTG GAA AAC CAC       288
   Glu Cys Asn Glu Asp Cys Asn Phe Lys Glu Leu Ile Leu Glu Asn His
                    85                  90                  95
   TAC AAC ACC TAC GCA TCT GCT AAA TGG ACC CAC AAC GGT GGT GAA ATG       336
   Tyr Asn Thr Tyr Ala Ser Ala Lys Trp Thr His Asn Gly Gly Glu Met
               100                 105                 110
   TTC GTT GCT CTG AAC CAG AAA GGT ATC CCG GTT CGT GGT AAA AAA ACC       384
   Phe Val Ala Leu Asn Gln Lys Gly Ile Pro Val Arg Gly Lys Lys Thr
           115                 120                 125
   AAA AAA GAA CAG AAA ACC GCT CAC TTC CTG CCG ATG GCA ATC ACT TAA       432
   Lys Lys Glu Gln Lys Thr Ala His Phe Leu Pro Met Ala Ile Thr  *
       130                 135                 140
(2) INFORMATION FOR SEQ ID NO:26:
   (i) SEQUENCE CHARACTERISTICS:
      (A) LENGTH: 144 amino acids
      (B) TYPE: amino acid
      (D) TOPOLOGY: linear
   (ii) MOLECULE TYPE: protein
   (xi) SEQUENCE DESCRIPTION: SEQ ID NO:26:
   Met Thr Arg Ser Tyr Asp Tyr Met Glu Gly Gly Asp Ile Arg Val Arg
     1               5                  10                  15
   Arg Leu Phe Cys Arg Thr Gln Trp Tyr Leu Arg Ile Asp Lys Arg Gly
                20                  25                  30
   Lys Val Lys Gly Thr Gln Glu Met Lys Asn Asn Tyr Asn Ile Met Glu
            35                  40                  45
   Ile Arg Thr Val Ala Val Gly Ile Val Ala Ile Lys Gly Val Glu Ser
        50                  55                  60
   Glu Phe Tyr Leu Ala Met Asn Lys Glu Gly Lys Leu Tyr Ala Lys Lys
    65                 70                  75                  80
   Glu Cys Asn Glu Asp Cys Asn Phe Lys Glu Leu Ile Leu Glu Asn His
                    85                  90                  95
   Tyr Asn Thr Tyr Ala Ser Ala Lys Trp Thr His Asn Gly Gly Glu Met
               100                 105                 110
   Phe Val Ala Leu Asn Gln Lys Gly Ile Pro Val Arg Gly Lys Lys Thr
           115                 120                 125
   Lys Lys Glu Gln Lys Thr Ala His Phe Leu Pro Met Ala Ile Thr  *
       130                 135                 140
(2) INFORMATION FOR SEQ ID NO:27:
   (i) SEQUENCE CHARACTERISTICS:
      (A) LENGTH: 429 base pairs
      (B) TYPE: nucleic acid
      (C) STRANDEDNESS: unknown
      (D) TOPOLOGY: unknown
   (ii) MOLECULE TYPE: cDNA
   (ix) FEATURE:
      (A) NAME/KEY: CDS
      (B) LOCATION: 1..429
   (xi) SEQUENCE DESCRIPTION: SEQ ID NO:27:
   ATG CGT TCC TAC GAC TAC ATG GAA GGT GGT GAC ATC CGC GTA CGT CGT          48
   Met Arg Ser Tyr Asp Tyr Met Glu Gly Gly Asp Ile Arg Val Arg Arg
     1               5                  10                  15
   CTG TTC TGC CGT ACC CAG TGG TAC CTG CGT ATC GAC AAA CGC GGC AAA          96
   Leu Phe Cys Arg Thr Gln Trp Tyr Leu Arg Ile Asp Lys Arg Gly Lys
                20                  25                  30
   GTC AAG GGC ACC CAA GAG ATG AAA AAC AAC TAC AAT ATT ATG GAA ATC         144
   Val Lys Gly Thr Gln Glu Met Lys Asn Asn Tyr Asn Ile Met Glu Ile
            35                  40                  45
   CGT ACT GTT GCT GTT GGT ATC GTT GCA ATC AAA GGT GTT GAA TCT GAA         192
   Arg Thr Val Ala Val Gly Ile Val Ala Ile Lys Gly Val Glu Ser Glu
        50                  55                  60
   TTC TAC CTG GCA ATG AAC AAA GAA GGT AAA CTG TAC GCA AAA AAA GAA         240
   Phe Tyr Leu Ala Met Asn Lys Glu Gly Lys Leu Tyr Ala Lys Lys Glu
    65                  70                  75                  80
   TGC AAC GAA GAC TGC AAC TTC AAA GAA CTG ATC CTG GAA AAC CAC TAC         288
   Cys Asn Glu Asp Cys Asn Phe Lys Glu Leu Ile Leu Glu Asn His Tyr
                    85                  90                  95
   AAC ACC TAC GCA TCT GCT AAA TGG ACC CAC AAC GGT GGT GAA ATG TTC         336
   Asn Thr Tyr Ala Ser Ala Lys Trp Thr His Asn Gly Gly Glu Met Phe
               100                 105                 110
   GTT GCT CTG AAC CAG AAA GGT ATC CCG GTT CGT GGT AAA AAA ACC AAA      384
   Val Ala Leu Asn Gln Lys Gly Ile Pro Val Arg Gly Lys Lys Thr Lys
           115                 120                 125
   AAA GAA CAG AAA ACC GCT CAC TTC CTG CCG ATG GCA ATC ACT TAA          429
   Lys Glu Gln Lys Thr Ala His Phe Leu Pro Met Ala Ile Thr  *
       130                 135                 140
(2) INFORMATION FOR SEQ ID NO:28:
   (i) SEQUENCE CHARACTERISTICS:
      (A) LENGTH: 143 amino acids
      (B) TYPE: amino acid
      (D) TOPOLOGY: linear
   (ii) MOLECULE TYPE: protein
   (xi) SEQUENCE DESCRIPTION: SEQ ID NO:28:
   Met Arg Ser Tyr Asp Tyr Met Glu Gly Gly Asp Ile Arg Val Arg Arg
    1               5                  10                  15
   Leu Phe Cys Arg Thr Gln Trp Tyr Leu Arg Ile Asp Lys Arg Gly Lys
               20                  25                  30
   Val Lys Gly Thr Gln Glu Met Lys Asn Asn Tyr Asn Ile Met Glu Ile
           35                  40                  45
   Arg Thr Val Ala Val Gly Ile Val Ala Ile Lys Gly Val Glu Ser Glu
       50                  55                  60
   Phe Tyr Leu Ala Met Asn Lys Glu Gly Lys Leu Tyr Ala Lys Lys Glu
   65                  70                  75                  80
   Cys Asn Glu Asp Cys Asn Phe Lys Glu Leu Ile Leu Glu Asn His Tyr
                   85                  90                  95
   Asn Thr Tyr Ala Ser Ala Lys Trp Thr His Asn Gly Gly Glu Met Phe
               100                 105                 110
   Val Ala Leu Asn Gln Lys Gly Ile Pro Val Arg Gly Lys Lys Thr Lys
           115                 120                 125
   Lys Glu Gln Lys Thr Ala His Phe Leu Pro Met Ala Ile Thr  *
       130                 135                 140
(2) INFORMATION FOR SEQ ID NO:29:
   (i) SEQUENCE CHARACTERISTICS:
      (A) LENGTH: 426 base pairs
      (B) TYPE: nucleic acid
      (C) STRANDEDNESS: unknown
      (D) TOPOLOGY: unknown
   (ii) MOLECULE TYPE: cDNA
   (ix) FEATURE:
      (A) NAME/KEY: CDS
      (B) LOCATION: 1..426
   (xi) SEQUENCE DESCRIPTION: SEQ ID NO:29:
   ATG TCC TAC GAC TAC ATG GAA GGT GGT GAC ATC CGC GTA CGT CGT CTG      48
   Met Ser Tyr Asp Tyr Met Glu Gly Gly Asp Ile Arg Val Arg Arg Leu
     1               5                  10                  15
   TTC TGC CGT ACC CAG TGG TAC CTG CGT ATC GAC AAA CGC GGC AAA GTC      96
   Phe Cys Arg Thr Gln Trp Tyr Leu Arg Ile Asp Lys Arg Gly Lys Val
                20                  25                  30
   AAG GGC ACC CAA GAG ATG AAA AAC AAC TAC AAT ATT ATG GAA ATC CGT     144
   Lys Gly Thr Gln Glu Met Lys Asn Asn Tyr Asn Ile Met Glu Ile Arg
            35                  40                  45
   ACT GTT GCT GTT GGT ATC GTT GCA ATC AAA GGT GTT GAA TCT GAA TTC     192
   Thr Val Ala Val Gly Ile Val Ala Ile Lys Gly Val Glu Ser Glu Phe
        50                  55                  60
   TAC CTG GCA ATG AAC AAA GAA GGT AAA CTG TAC GCA AAA AAA GAA TGC     240
   Tyr Leu Ala Met Asn Lys Glu Gly Lys Leu Tyr Ala Lys Lys Glu Cys
    65                  70                  75                  80
   AAC GAA GAC TGC AAC TTC AAA GAA CTG ATC CTG GAA AAC CAC TAC AAC     288
   Asn Glu Asp Cys Asn Phe Lys Glu Leu Ile Leu Glu Asn His Tyr Asn
                    85                  90                  95
   ACC TAC GCA TCT GCT AAA TGG ACC CAC AAC GGT GGT GAA ATG TTC GTT     336
   Thr Tyr Ala Ser Ala Lys Trp Thr His Asn Gly Gly Glu Met Phe Val
               100                 105                 110
   GCT CTG AAC CAG AAA GGT ATC CCG GTT CGT GGT AAA AAA ACC AAA AAA     384
   Ala Leu Asn Gln Lys Gly Ile Pro Val Arg Gly Lys Lys Thr Lys Lys
           115                 120                 125
   GAA CAG AAA ACC GCT CAC TTC CTG CCG ATG GCA ATC ACT TAA             426
   Glu Gln Lys Thr Ala His Phe Leu Pro Met Ala Ile Thr  *
       130                 135                 140
(2) INFORMATION FOR SEQ ID NO:30:
   (i) SEQUENCE CHARACTERISTICS:
      (A) LENGTH: 142 amino acids
      (B) TYPE: amino acid
      (D) TOPOLOGY: linear
   (ii) MOLECULE TYPE: protein
   (xi) SEQUENCE DESCRIPTION: SEQ ID NO:30:
   Met Ser Tyr Asp Tyr Met Glu Gly Gly Asp Ile Arg Val Arg Arg Leu
     1               5                  10                  15
   Phe Cys Arg Thr Gln Trp Tyr Leu Arg Ile Asp Lys Arg Gly Lys Val
                20                  25                  30
   Lys Gly Thr Gln Glu Met Lys Asn Asn Tyr Asn Ile Met Glu Ile Arg
           35                  40                  45
   Thr Val Ala Val Gly Ile Val Ala Ile Lys Gly Val Glu Ser Glu Phe
       50                  55                  60
   Tyr Leu Ala Met Asn Lys Glu Gly Lys Leu Tyr Ala Lys Lys Glu Cys
   65                  70                  75                  80
   Asn Glu Asp Cys Asn Phe Lys Glu Leu Ile Leu Glu Asn His Tyr Asn
                   85                  90                  95
   Thr Tyr Ala Ser Ala Lys Trp Thr His Asn Gly Gly Glu Met Phe Val
               100                 105                 110
   Ala Leu Asn Gln Lys Gly Ile Pro Val Arg Gly Lys Lys Thr Lys Lys
           115                 120                 125
   Glu Gln Lys Thr Ala His Phe Leu Pro Met Ala Ile Thr  *
       130                 135                 140
(2) INFORMATION FOR SEQ ID NO:31:
   (i) SEQUENCE CHARACTERISTICS:
      (A) LENGTH: 423 base pairs
      (B) TYPE: nucleic acid
      (C) STRANDEDNESS: unknown
      (D) TOPOLOGY: unknown
   (ii) MOLECULE TYPE: cDNA
   (ix) FEATURE:
      (A) NAME/KEY: CDS
      (B) LOCATION: 1..423
   (xi) SEQUENCE DESCRIPTION: SEQ ID NO:31:
   ATG TAC GAC TAC ATG GAA GGT GGT GAC ATC CGC GTA CGT CGT CTG TTC        48
   Met Tyr Asp Tyr Met Glu Gly Gly Asp Ile Arg Val Arg Arg Leu Phe
     1               5                  10                  15
   TGC CGT ACC CAG TGG TAC CTG CGT ATC GAC AAA CGC GGC AAA GTC AAG        96
   Cys Arg Thr Gln Trp Tyr Leu Arg Ile Asp Lys Arg Gly Lys Val Lys
               20                  25                  30
   GGC ACC CAA GAG ATG AAA AAC AAC TAC AAT ATT ATG GAA ATC CGT ACT       144
   Gly Thr Gln Glu Met Lys Asn Asn Tyr Asn Ile Met Glu Ile Arg Thr
           35                  40                  45
   GTT GCT GTT GGT ATC GTT GCA ATC AAA GGT GTT GAA TCT GAA TTC TAC       192
   Val Ala Val Gly Ile Val Ala Ile Lys Gly Val Glu Ser Glu Phe Tyr
        50                  55                  60
   CTG GCA ATG AAC AAA GAA GGT AAA CTG TAC GCA AAA AAA GAA TGC AAC       240
   Leu Ala Met Asn Lys Glu Gly Lys Leu Tyr Ala Lys Lys Glu Cys Asn
    65                  70                  75                  80
   GAA GAC TGC AAC TTC AAA GAA CTG ATC CTG GAA AAC CAC TAC AAC ACC       288
   Glu Asp Cys Asn Phe Lys Glu Leu Ile Leu Glu Asn His Tyr Asn Thr
                    85                  90                  95
   TAC GCA TCT GCT AAA TGG ACC CAC AAC GGT GGT GAA ATG TTC GTT GCT       336
   Tyr Ala Ser Ala Lys Trp Thr His Asn Gly Gly Glu Met Phe Val Ala
               100                 105                 110
   CTG AAC CAG AAA GGT ATC CCG GTT CGT GGT AAA AAA ACC AAA AAA GAA       384
   Leu Asn Gln Lys Gly Ile Pro Val Arg Gly Lys Lys Thr Lys Lys Glu
           115                 120                 125
   CAG AAA ACC GCT CAC TTC CTG CCG ATG GCA ATC ACT TAA                   423
   Gln Lys Thr Ala His Phe Leu Pro Met Ala Ile Thr  *
       130                 135                 140
(2) INFORMATION FOR SEQ ID NO:32:
   (i) SEQUENCE CHARACTERISTICS:
      (A) LENGTH: 141 amino acids
      (B) TYPE: amino acid
      (D) TOPOLOGY: linear
   (ii) MOLECULE TYPE: protein
   (xi) SEQUENCE DESCRIPTION: SEQ ID NO:32:
   Met Tyr Asp Tyr Met Glu Gly Gly Asp Ile Arg Val Arg Arg Leu Phe
    1               5                  10                  15
   Cys Arg Thr Gln Trp Tyr Leu Arg Ile Asp Lys Arg Gly Lys Val Lys
               20                  25                  30
   Gly Thr Gln Glu Met Lys Asn Asn Tyr Asn Ile Met Glu Ile Arg Thr
           35                  40                  45
   Val Ala Val Gly Ile Val Ala Ile Lys Gly Val Glu Ser Glu Phe Tyr
       50                  55                  60
   Leu Ala Met Asn Lys Glu Gly Lys Leu Tyr Ala Lys Lys Glu Cys Asn
   65                  70                  75                  80
   Glu Asp Cys Asn Phe Lys Glu Leu Ile Leu Glu Asn His Tyr Asn Thr
                   85                  90                  95
   Tyr Ala Ser Ala Lys Trp Thr His Asn Gly Gly Glu Met Phe Val Ala
               100                 105                 110
   Leu Asn Gln Lys Gly Ile Pro Val Arg Gly Lys Lys Thr Lys Lys Glu
           115                 120                 125
   Gln Lys Thr Ala His Phe Leu Pro Met Ala Ile Thr  *
       130                 135                 140
(2) INFORMATION FOR SEQ ID NO:33:
   (i) SEQUENCE CHARACTERISTICS:
      (A) LENGTH: 495 base pairs
      (B) TYPE: nucleic acid
      (C) STRANDEDNESS: unknown
      (D) TOPOLOGY: unknown
   (ii) MOLECULE TYPE: cDNA
   (ix) FEATURE:
      (A) NAME/KEY: CDS
      (B) LOCATION: 1..495
   (xi) SEQUENCE DESCRIPTION: SEQ ID NO:33:
   ATG TCT AAT GAT ATG ACT CCG GAA CAG ATG GCT ACC AAC GTT AAC TCC      48
   Met Ser Asn Asp Met Thr Pro Glu Gln Met Ala Thr Asn Val Asn Ser
    1               5                  10                  15
   TCC TCC CCG GAA CGT CAC ACG CGT TCC TAC GAC TAC ATG GAA GGT GGT      96
   Ser Ser Pro Glu Arg His Thr Arg Ser Tyr Asp Tyr Met Glu Gly Gly
               20                  25                  30
   GAC ATC CGC GTA CGT CGT CTG TTC TGC CGT ACC CAG TGG TAC CTG CGT     144
   Asp Ile Arg Val Arg Arg Leu Phe Cys Arg Thr Gln Trp Tyr Leu Arg
           35                  40                  45
   ATC GAC AAA CGC GGC AAA GTC AAG GGC ACC CAA GAG ATG AAA AAC AAC     192
   Ile Asp Lys Arg Gly Lys Val Lys Gly Thr Gln Glu Met Lys Asn Asn
       50                  55                  60
   TAC AAT ATT ATG GAA ATC CGT ACT GTT GCT GTT GGT ATC GTT GCA ATC     240
   Tyr Asn Ile Met Glu Ile Arg Thr Val Ala Val Gly Ile Val Ala Ile
   65                  70                  75                  80
   AAA GGT GTT GAA TCT GAA TTC TAT CTT GCA ATG AAC AAG GAA GGA AAA     288
   Lys Gly Val Glu Ser Glu Phe Tyr Leu Ala Met Asn Lys Glu Gly Lys
                   85                  90                  95
   CTC TAT GCA AAG AAA GAA TGC AAT GAA GAT TGT AAC TTC AAA GAA CTA     336
   Leu Tyr Ala Lys Lys Glu Cys Asn Glu Asp Cys Asn Phe Lys Glu Leu
               100                 105                 110
   ATT CTG GAA AAC CAT TAC AAC ACA TAT GCA TCT GCT AAA TGG ACC CAC     384
   Ile Leu Glu Asn His Tyr Asn Thr Tyr Ala Ser Ala Lys Trp Thr His
           115                 120                 125
   AAC GGT GGT GAA ATG TTC GTT GCT CTG AAC CAG AAA GGT ATC CCT GTT     432
   Asn Gly Gly Glu Met Phe Val Ala Leu Asn Gln Lys Gly Ile Pro Val
       130                 135                 140
   GAA GGT AAG AAA ACC AAG AAA GAA CAG AAA ACC GCT CAC TTC CTG CCG     480
   Glu Gly Lys Lys Thr Lys Lys Glu Gln Lys Thr Ala His Phe Leu Pro
   145                 150                 155                 160
   ATG GCA ATC ACT TAA                                                 495
   Met Ala Ile Thr  *
                   165
(2) INFORMATION FOR SEQ ID NO:34:
   (i) SEQUENCE CHARACTERISTICS:
      (A) LENGTH: 165 amino acids
      (B) TYPE: amino acid
      (D) TOPOLOGY: linear
   (ii) MOLECULE TYPE: protein
   (xi) SEQUENCE DESCRIPTION: SEQ ID NO:34:
   Met Ser Asn Asp Met Thr Pro Glu Gln Met Ala Thr Asn Val Asn Ser
     1             5                  10                  15
   Ser Ser Pro Glu Arg His Thr Arg Ser Tyr Asp Tyr Met Glu Gly Gly
               20                  25                  30
   Asp Ile Arg Val Arg Arg Leu Phe Cys Arg Thr Gln Trp Tyr Leu Arg
           35                  40                  45
   Ile Asp Lys Arg Gly Lys Val Lys Gly Thr Gln Glu Met Lys Asn Asn
       50                  55                  60
   Tyr Asn Ile Met Glu Ile Arg Thr Val Ala Val Gly Ile Val Ala Ile
    65                 70                  75                  80
   Lys Gly Val Glu Ser Glu Phe Tyr Leu Ala Met Asn Lys Glu Gly Lys
                    85                  90                  95
   Leu Tyr Ala Lys Lys Glu Cys Asn Glu Asp Cys Asn Phe Lys Glu Leu
               100                 105                 110
   Ile Leu Glu Asn His Tyr Asn Thr Tyr Ala Ser Ala Lys Trp Thr His
           115                 120                 125
   Asn Gly Gly Glu Met Phe Val Ala Leu Asn Gln Lys Gly Ile Pro Val
       130                 135                 140
   Glu Gly Lys Lys Thr Lys Lys Glu Gln Lys Thr Ala His Phe Leu Pro
   145                 150                 155                 160
   Met Ala Ile Thr  *
                   165
(2) INFORMATION FOR SEQ ID NO:35:
   (i) SEQUENCE CHARACTERISTICS:
      (A) LENGTH: 495 base pairs
      (B) TYPE: nucleic acid
      (C) STRANDEDNESS: unknown
      (D) TOPOLOGY: unknown
   (ii) MOLECULE TYPE: cDNA
   (ix) FEATURE:
      (A) NAME/KEY: CDS
      (B) LOCATION: 1..495
   (xi) SEQUENCE DESCRIPTION: SEQ ID NO:35:
   ATG TCT AAT GAT ATG ACT CCG GAA CAG ATG GCT ACC AAC GTT AAC TCC      48
   Met Ser Asn Asp Met Thr Pro Glu Gln Met Ala Thr Asn Val Asn Ser
     1             5                  10                  15
   TCC TCC CCG GAA CGT CAC ACG CGT TCC TAC GAC TAC ATG GAA GGT GGT      96
   Ser Ser Pro Glu Arg His Thr Arg Ser Tyr Asp Tyr Met Glu Gly Gly
               20                  25                  30
   GAC ATC CGC GTA CGT CGT CTG TTC TGC CGT ACC CAG TGG TAC CTG CGT     144
   Asp Ile Arg Val Arg Arg Leu Phe Cys Arg Thr Gln Trp Tyr Leu Arg
           35                  40                  45
   ATC GAC AAA CGC GGC AAA GTC AAG GGC ACC CAA GAG ATG AAA AAC AAC      192
   Ile Asp Lys Arg Gly Lys Val Lys Gly Thr Gln Glu Met Lys Asn Asn
       50                  55                  60
   TAC AAT ATT ATG GAA ATC CGT ACT GTT GCT GTT GGT ATC GTT GCA ATC      240
   Tyr Asn Ile Met Glu Ile Arg Thr Val Ala Val Gly Ile Val Ala Ile
   65                  70                  75                  80
   AAA GGT GTT GAA TCT GAA TTC TAT CTT GCA ATG AAC AAG GAA GGA AAA      288
   Lys Gly Val Glu Ser Glu Phe Tyr Leu Ala Met Asn Lys Glu Gly Lys
                   85                  90                  95
   CTC TAT GCA AAG AAA GAA TGC AAT GAA GAT TGT AAC TTC AAA GAA CTA      336
   Leu Tyr Ala Lys Lys Glu Cys Asn Glu Asp Cys Asn Phe Lys Glu Leu
               100                 105                 110
   ATT CTG GAA AAC CAT TAC AAC ACA TAT GCA TCT GCT AAA TGG ACC CAC      384
   Ile Leu Glu Asn His Tyr Asn Thr Tyr Ala Ser Ala Lys Trp Thr His
           115                 120                 125
   AAC GGT GGT GAA ATG TTC GTT GCT CTG AAC CAG AAA GGT ATC CCT GTT      432
   Asn Gly Gly Glu Met Phe Val Ala Leu Asn Gln Lys Gly Ile Pro Val
       130                 135                 140
   CAA GGT AAG AAA ACC AAG AAA GAA CAG AAA ACC GCT CAC TTC CTG CCG      480
   Gln Gly Lys Lys Thr Lys Lys Glu Gln Lys Thr Ala His Phe Leu Pro
   145                 150                 155                 160
   ATG GCA ATC ACT TAA                                                  495
   Met Ala Ile Thr  *
                   165
(2) INFORMATION FOR SEQ ID NO:36:
   (i) SEQUENCE CHARACTERISTICS:
      (A) LENGTH: 165 amino acids
      (B) TYPE: amino acid
      (D) TOPOLOGY: linear
   (ii) MOLECULE TYPE: protein
   (xi) SEQUENCE DESCRIPTION: SEQ ID NO:36:
   Met Ser Asn Asp Met Thr Pro Glu Gln Met Ala Thr Asn Val Asn Ser
    1               5                  10                  15
   Ser Ser Pro Glu Arg His Thr Arg Ser Tyr Asp Tyr Met Glu Gly Gly
               20                  25                  30
   Asp Ile Arg Val Arg Arg Leu Phe Cys Arg Thr Gln Trp Tyr Leu Arg
           35                  40                  45
   Ile Asp Lys Arg Gly Lys Val Lys Gly Thr Gln Glu Met Lys Asn Asn
       50                  55                  60
   Tyr Asn Ile Met Glu Ile Arg Thr Val Ala Val Gly Ile Val Ala Ile
   65                  70                  75                  80
   Lys Gly Val Glu Ser Glu Phe Tyr Leu Ala Met Asn Lys Glu Gly Lys
                   85                  90                  95
   Leu Tyr Ala Lys Lys Glu Cys Asn Glu Asp Cys Asn Phe Lys Glu Leu
               100                 105                 110
   Ile Leu Glu Asn His Tyr Asn Thr Tyr Ala Ser Ala Lys Trp Thr His
           115                 120                 125
   Asn Gly Gly Glu Met Phe Val Ala Leu Asn Gln Lys Gly Ile Pro Val
       130                 135                 140
   Gln Gly Lys Lys Thr Lys Lys Glu Gln Lys Thr Ala His Phe Leu Pro
   145                 150                 155                 160
   Met Ala Ile Thr  *
                   165
(2) INFORMATION FOR SEQ ID NO:37:
   (i) SEQUENCE CHARACTERISTICS:
      (A) LENGTH: 426 base pairs
      (B) TYPE: nucleic acid
      (C) STRANDEDNESS: unknown
      (D) TOPOLOGY: unknown
   (ii) MOLECULE TYPE: cDNA
   (ix) FEATURE:
      (A) NAME/KEY: CDS
      (B) LOCATION: 1..426
   (xi) SEQUENCE DESCRIPTION: SEQ ID NO:37:
   ATG TCC TAC GAC TAC ATG GAA GGT GGT GAC ATC CGC GTA CGT CGT CTG       48
   Met Ser Tyr Asp Tyr Met Glu Gly Gly Asp Ile Arg Val Arg Arg Leu
     1               5                  10                  15
   TTC TGC CGT ACC CAG TGG TAC CTG CGT ATC GAC AAA CGC GGC AAA GTC       96
   Phe Cys Arg Thr Gln Trp Tyr Leu Arg Ile Asp Lys Arg Gly Lys Val
                20                  25                  30
   AAG GGC ACC CAA GAG ATG AAA AAC AAC TAC AAT ATT ATG GAA ATC CGT      144
   Lys Gly Thr Gln Glu Met Lys Asn Asn Tyr Asn Ile Met Glu Ile Arg
            35                  40                  45
   ACT GTT GCT GTT GGT ATC GTT GCA ATC AAA GGT GTT GAA TCT GAA TTC      192
   Thr Val Ala Val Gly Ile Val Ala Ile Lys Gly Val Glu Ser Glu Phe
        50                  55                  60
   TAT CTT GCA ATG AAC AAG GAA GGA AAA CTC TAT GCA AAG AAA GAA TGC      240
   Tyr Leu Ala Met Asn Lys Glu Gly Lys Leu Tyr Ala Lys Lys Glu Cys
    65                  70                  75                  80
   AAT GAA GAT TGT AAC TTC AAA GAA CTA ATT CTG GAA AAC CAT TAC AAC      288
   Asn Glu Asp Cys Asn Phe Lys Glu Leu Ile Leu Glu Asn His Tyr Asn
                    85                  90                  95
   ACA TAT GCA TCT GCT AAA TGG ACC CAC AAC GGT GGT GAA ATG TTC GTT      336
   Thr Tyr Ala Ser Ala Lys Trp Thr His Asn Gly Gly Glu Met Phe Val
               100                 105                 110
   GCT CTG AAC CAG AAA GGT ATC CCT GTT CAA GGT AAG AAA ACC AAG AAA        384
   Ala Leu Asn Gln Lys Gly Ile Pro Val Gln Gly Lys Lys Thr Lys Lys
           115                 120                 125
   GAA CAG AAA ACC GCT CAC TTC CTG CCG ATG GCA ATC ACT TAA                426
   Glu Gln Lys Thr Ala His Phe Leu Pro Met Ala Ile Thr  *
       130                 135                 140
(2) INFORMATION FOR SEQ ID NO:38:
   (i) SEQUENCE CHARACTERISTICS:
      (A) LENGTH: 142 amino acids
      (B) TYPE: amino acid
      (D) TOPOLOGY: linear
   (ii) MOLECULE TYPE: protein
   (xi) SEQUENCE DESCRIPTION: SEQ ID NO:38:
   Met Ser Tyr Asp Tyr Met Glu Gly Gly Asp Ile Arg Val Arg Arg Leu
    1               5                  10                  15
   Phe Cys Arg Thr Gln Trp Tyr Leu Arg Ile Asp Lys Arg Gly Lys Val
               20                  25                  30
   Lys Gly Thr Gln Glu Met Lys Asn Asn Tyr Asn Ile Met Glu Ile Arg
           35                  40                  45
   Thr Val Ala Val Gly Ile Val Ala Ile Lys Gly Val Glu Ser Glu Phe
       50                  55                  60
   Tyr Leu Ala Met Asn Lys Glu Gly Lys Leu Tyr Ala Lys Lys Glu Cys
   65                  70                  75                  80
   Asn Glu Asp Cys Asn Phe Lys Glu Leu Ile Leu Glu Asn His Tyr Asn
                   85                  90                  95
   Thr Tyr Ala Ser Ala Lys Trp Thr His Asn Gly Gly Glu Met Phe Val
               100                 105                 110
   Ala Leu Asn Gln Lys Gly Ile Pro Val Gln Gly Lys Lys Thr Lys Lys
           115                 120                 125
   Glu Gln Lys Thr Ala His Phe Leu Pro Met Ala Ile Thr  *
       130                 135                 140
(2) INFORMATION FOR SEQ ID NO:39:
   (i) SEQUENCE CHARACTERISTICS:
      (A) LENGTH: 495 base pairs
      (B) TYPE: nucleic acid
      (C) STRANDEDNESS: unknown
      (D) TOPOLOGY: unknown
   (ii) MOLECULE TYPE: cDNA
   (ix) FEATURE:
      (A) NAME/KEY: CDS
      (B) LOCATION: 1..495
   (xi) SEQUENCE DESCRIPTION: SEQ ID NO:39:
   ATG TCT AAT GAT ATG ACT CCG GAA CAG ATG GCT ACC AAC GTT AAC TCC       48
   Met Ser Asn Asp Met Thr Pro Glu Gln Met Ala Thr Asn Val Asn Ser
    1               5                  10                  15
   TCC TCC CCG GAA CGT CAC ACG CGT TCC TAC GAC TAC ATG GAA GGT GGT       96
   Ser Ser Pro Glu Arg His Thr Arg Ser Tyr Asp Tyr Met Glu Gly Gly
               20                  25                  30
   GAC ATC CGC GTA CGC CGT TTG TTC TCT CGT ACC CAG TGG TAC CTG CGT      144
   Asp Ile Arg Val Arg Arg Leu Phe Ser Arg Thr Gln Trp Tyr Leu Arg
           35                  40                  45
   ATC GAC AAA CGC GGC AAA GTC AAG GGC ACC CAA GAG ATG AAA AAC AAC      192
   Ile Asp Lys Arg Gly Lys Val Lys Gly Thr Gln Glu Met Lys Asn Asn
       50                  55                  60
   TAC AAT ATT ATG GAA ATC CGT ACT GTT GCT GTT GGT ATC GTT GCA ATC      240
   Tyr Asn Ile Met Glu Ile Arg Thr Val Ala Val Gly Ile Val Ala Ile
   65                  70                  75                  80
   AAA GGT GTT GAA TCT GAA TTC TAT CTT GCA ATG AAC AAG GAA GGA AAA      288
   Lys Gly Val Glu Ser Glu Phe Tyr Leu Ala Met Asn Lys Glu Gly Lys
                   85                  90                  95
   CTC TAT GCA AAG AAA GAA TGC AAT GAA GAT TGT AAC TTC AAA GAA CTA      336
   Leu Tyr Ala Lys Lys Glu Cys Asn Glu Asp Cys Asn Phe Lys Glu Leu
               100                 105                 110
   ATT CTG GAA AAC CAT TAC AAC ACA TAT GCA TCA GCT AAA TGG ACA CAC      384
   Ile Leu Glu Asn His Tyr Asn Thr Tyr Ala Ser Ala Lys Trp Thr His
           115                 120                 125
   AAC GGA GGG GAA ATG TTT GTT GCC TTA AAT CAA AAG GGG ATT CCT GTA      432
   Asn Gly Gly Glu Met Phe Val Ala Leu Asn Gln Lys Gly Ile Pro Val
       130                 135                 140
   AGA GGA AAA AAA ACG AAG AAA GAA CAA AAA ACA GCC CAC TTT CTT CCT      480
   Arg Gly Lys Lys Thr Lys Lys Glu Gln Lys Thr Ala His Phe Leu Pro
   145                 150                 155                 160
   ATG GCA ATA ACT TAA                                                  495
   Met Ala Ile Thr  *
                   165
(2) INFORMATION FOR SEQ ID NO:40:
   (i) SEQUENCE CHARACTERISTICS:
      (A) LENGTH: 165 amino acids
      (B) TYPE: amino acid
      (D) TOPOLOGY: linear
   (ii) MOLECULE TYPE: protein
   (xi) SEQUENCE DESCRIPTION: SEQ ID NO:40:
   Met Ser Asn Asp Met Thr Pro Glu Gln Met Ala Thr Asn Val Asn Ser
    1               5                  10                  15
   Ser Ser Pro Glu Arg His Thr Arg Ser Tyr Asp Tyr Met Glu Gly Gly
               20                  25                  30
   Asp Ile Arg Val Arg Arg Leu Phe Ser Arg Thr Gln Trp Tyr Leu Arg
           35                  40                  45
   Ile Asp Lys Arg Gly Lys Val Lys Gly Thr Gln Glu Met Lys Asn Asn
       50                  55                  60
   Tyr Asn Ile Met Glu Ile Arg Thr Val Ala Val Gly Ile Val Ala Ile
   65                  70                  75                  80
   Lys Gly Val Glu Ser Glu Phe Tyr Leu Ala Met Asn Lys Glu Gly Lys
                   85                  90                  95
   Leu Tyr Ala Lys Lys Glu Cys Asn Glu Asp Cys Asn Phe Lys Glu Leu
               100                 105                 110
   Ile Leu Glu Asn His Tyr Asn Thr Tyr Ala Ser Ala Lys Trp Thr His
           115                 120                 125
   Asn Gly Gly Glu Met Phe Val Ala Leu Asn Gln Lys Gly Ile Pro Val
       130                 135                 140
   Arg Gly Lys Lys Thr Lys Lys Glu Gln Lys Thr Ala His Phe Leu Pro
   145                 150                 155                 160
   Met Ala Ile Thr  *
                   165
(2) INFORMATION FOR SEQ ID NO:41:
   (i) SEQUENCE CHARACTERISTICS:
      (A) LENGTH: 495 base pairs
      (B) TYPE: nucleic acid
      (C) STRANDEDNESS: unknown
      (D) TOPOLOGY: unknown
   (ii) MOLECULE TYPE: cDNA
   (ix) FEATURE:
      (A) NAME/KEY: CDS
      (B) LOCATION: 1..495
   (xi) SEQUENCE DESCRIPTION: SEQ ID NO:41:
   ATG TCT AAT GAT ATG ACT CCG GAA CAG ATG GCT ACC AAC GTT AAC TCC      48
   Met Ser Asn Asp Met Thr Pro Glu Gln Met Ala Thr Asn Val Asn Ser
    1               5                  10                  15
   TCC TCC CCG GAA CGT CAC ACG CGT TCC TAC GAC TAC ATG GAA GGT GGT      96
   Ser Ser Pro Glu Arg His Thr Arg Ser Tyr Asp Tyr Met Glu Gly Gly
               20                  25                  30
   GAC ATC CGC GTA CGT CGT CTG TTC TGC CGT ACC CAG TGG TAC CTG CGT     144
   Asp Ile Arg Val Arg Arg Leu Phe Cys Arg Thr Gln Trp Tyr Leu Arg
           35                  40                  45
   ATC GAC AAA CGC GGC AAA GTC AAG GGC ACC CAA GAG ATG AAA AAC AAC     192
   Ile Asp Lys Arg Gly Lys Val Lys Gly Thr Gln Glu Met Lys Asn Asn
       50                  55                  60
   TAC AAT ATT ATG GAA ATC CGT ACT GTT GCT GTT GGT ATC GTT GCA ATC     240
   Tyr Asn Ile Met Glu Ile Arg Thr Val Ala Val Gly Ile Val Ala Ile
   65                  70                  75                  80
   AAA GGT GTT GAA TCT GAA TTC TAC CTG GCT ATG AAC AAA GAA GGT AAA     288
   Lys Gly Val Glu Ser Glu Phe Tyr Leu Ala Met Asn Lys Glu Gly Lys
                   85                  90                  95
   CTG TAC GCT AAA AAA GAA TCC AAT GAA GAT TGT AAC TTC AAA GAA CTA     336
   Leu Tyr Ala Lys Lys Glu Ser Asn Glu Asp Cys Asn Phe Lys Glu Leu
               100                 105                 110
   ATT CTG GAA AAC CAT TAC AAC ACA TAT GCA TCA GCT AAA TGG ACA CAC     384
   Ile Leu Glu Asn His Tyr Asn Thr Tyr Ala Ser Ala Lys Trp Thr His
           115                 120                 125
   AAC GGA GGG GAA ATG TTT GTT GCC TTA AAT CAA AAG GGG ATT CCT GTA     432
   Asn Gly Gly Glu Met Phe Val Ala Leu Asn Gln Lys Gly Ile Pro Val
       130                 135                 140
   AGA GGA AAA AAA ACG AAG AAA GAA CAA AAA ACA GCC CAC TTT CTT CCT     480
   Arg Gly Lys Lys Thr Lys Lys Glu Gln Lys Thr Ala His Phe Leu Pro
   145                 150                 155                 160
   ATG GCA ATA ACT TAA                                                 495
   Met Ala Ile Thr  *
                   165
(2) INFORMATION FOR SEQ ID NO:42:
   (i) SEQUENCE CHARACTERISTICS:
      (A) LENGTH: 165 amino acids
      (B) TYPE: amino acid
      (D) TOPOLOGY: linear
   (ii) MOLECULE TYPE: protein
   (xi) SEQUENCE DESCRIPTION: SEQ ID NO:42:
   Met Ser Asn Asp Met Thr Pro Glu Gln Met Ala Thr Asn Val Asn Ser
    1               5                  10                  15
   Ser Ser Pro Glu Arg His Thr Arg Ser Tyr Asp Tyr Met Glu Gly Gly
               20                  25                  30
   Asp Ile Arg Val Arg Arg Leu Phe Cys Arg Thr Gln Trp Tyr Leu Arg
           35                  40                  45
   Ile Asp Lys Arg Gly Lys Val Lys Gly Thr Gln Glu Met Lys Asn Asn
       50                  55                  60
   Tyr Asn Ile Met Glu Ile Arg Thr Val Ala Val Gly Ile Val Ala Ile
   65                  70                  75                  80
   Lys Gly Val Glu Ser Glu Phe Tyr Leu Ala Met Asn Lys Glu Gly Lys
                   85                  90                  95
   Leu Tyr Ala Lys Lys Glu Ser Asn Glu Asp Cys Asn Phe Lys Glu Leu
               100                 105                 110
   Ile Leu Glu Asn His Tyr Asn Thr Tyr Ala Ser Ala Lys Trp Thr His
           115                 120                 125
   Asn Gly Gly Glu Met Phe Val Ala Leu Asn Gln Lys Gly Ile Pro Val
       130                 135                 140
   Arg Gly Lys Lys Thr Lys Lys Glu Gln Lys Thr Ala His Phe Leu Pro
   145                 150                 155                 160
   Met Ala Ile Thr  *
                   165
(2) INFORMATION FOR SEQ ID NO:43:
   (i) SEQUENCE CHARACTERISTICS:
      (A) LENGTH: 495 base pairs
      (B) TYPE: nucleic acid
      (C) STRANDEDNESS: unknown
      (D) TOPOLOGY: unknown
   (ii) MOLECULE TYPE: cDNA
   (ix) FEATURE:
      (A) NAME/KEY: CDS
      (B) LOCATION: 1..495
   (xi) SEQUENCE DESCRIPTION: SEQ ID NO:43:
   ATG TCT AAT GAT ATG ACT CCG GAA CAG ATG GCT ACC AAC GTT AAC TCC    48
   Met Ser Asn Asp Met Thr Pro Glu Gln Met Ala Thr Asn Val Asn Ser
     1               5                  10                  15
   TCC TCC CCG GAA CGT CAC ACG CGT TCC TAC GAC TAC ATG GAA GGT GGT    96
   Ser Ser Pro Glu Arg His Thr Arg Ser Tyr Asp Tyr Met Glu Gly Gly
                20                  25                  30
   GAC ATC CGC GTA CGT CGT CTG TTC TGC CGT ACC CAG TGG TAC CTG CGT   144
   Asp Ile Arg Val Arg Arg Leu Phe Cys Arg Thr Gln Trp Tyr Leu Arg
            35                  40                  45
   ATC GAC AAA CGC GGC AAA GTC AAG GGC ACC CAA GAG ATG AAA AAC AAC   192
   Ile Asp Lys Arg Gly Lys Val Lys Gly Thr Gln Glu Met Lys Asn Asn
        50                  55                  60
   TAC AAT ATT ATG GAA ATC CGT ACT GTT GCT GTT GGT ATC GTT GCA ATC   240
   Tyr Asn Ile Met Glu Ile Arg Thr Val Ala Val Gly Ile Val Ala Ile
    65                  70                  75                  80
   AAA GGT GTT GAA TCT GAA TTC TAC CTG GCT ATG AAC AAA GAA GGT AAA   288
   Lys Gly Val Glu Ser Glu Phe Tyr Leu Ala Met Asn Lys Glu Gly Lys
                    85                  90                  95
   CTG TAC GCT AAA AAA GAA TCC AAT GAA GAT TCT AAC TTC AAA GAA CTA   336
   Leu Tyr Ala Lys Lys Glu Ser Asn Glu Asp Ser Asn Phe Lys Glu Leu
               100                 105                 110
   ATT CTG GAA AAC CAT TAC AAC ACA TAT GCA TCA GCT AAA TGG ACA CAC   384
   Ile Leu Glu Asn His Tyr Asn Thr Tyr Ala Ser Ala Lys Trp Thr His
           115                 120                 125
   AAC GGA GGG GAA ATG TTT GTT GCC TTA AAT CAA AAG GGG ATT CCT GTA   432
   Asn Gly Gly Glu Met Phe Val Ala Leu Asn Gln Lys Gly Ile Pro Val
       130                 135                 140
   AGA GGA AAA AAA ACG AAG AAA GAA CAA AAA ACA GCC CAC TTT CTT CCT   480
   Arg Gly Lys Lys Thr Lys Lys Glu Gln Lys Thr Ala His Phe Leu Pro
   145                 150                 155                 160
   ATG GCA ATA ACT TAA                                               495
   Met Ala Ile Thr  *
                   165
(2) INFORMATION FOR SEQ ID NO:44:
   (i) SEQUENCE CHARACTERISTICS:
      (A) LENGTH: 165 amino acids
      (B) TYPE: amino acid
      (D) TOPOLOGY: linear
   (ii) MOLECULE TYPE: protein
   (xi) SEQUENCE DESCRIPTION: SEQ ID NO:44:
   Met Ser Asn Asp Met Thr Pro Glu Gln Met Ala Thr Asn Val Asn Ser
    1               5                  10                  15
   Ser Ser Pro Glu Arg His Thr Arg Ser Tyr Asp Tyr Met Glu Gly Gly
               20                  25                  30
   Asp Ile Arg Val Arg Arg Leu Phe Cys Arg Thr Gln Trp Tyr Leu Arg
           35                  40                  45
   Ile Asp Lys Arg Gly Lys Val Lys Gly Thr Gln Glu Met Lys Asn Asn
       50                  55                  60
   Tyr Asn Ile Met Glu Ile Arg Thr Val Ala Val Gly Ile Val Ala Ile
   65                  70                  75                  80
   Lys Gly Val Glu Ser Glu Phe Tyr Leu Ala Met Asn Lys Glu Gly Lys
                   85                  90                  95
   Leu Tyr Ala Lys Lys Glu Ser Asn Glu Asp Ser Asn Phe Lys Glu Leu
               100                 105                 110
   Ile Leu Glu Asn His Tyr Asn Thr Tyr Ala Ser Ala Lys Trp Thr His
           115                 120                 125
   Asn Gly Gly Glu Met Phe Val Ala Leu Asn Gln Lys Gly Ile Pro Val
       130                 135                 140
   Arg Gly Lys Lys Thr Lys Lys Glu Gln Lys Thr Ala His Phe Leu Pro
   145                 150                 155                 160
   Met Ala Ile Thr  *
                   165
(2) INFORMATION FOR SEQ ID NO:45:
   (i) SEQUENCE CHARACTERISTICS:
      (A) LENGTH: 426 base pairs
      (B) TYPE: nucleic acid
      (C) STRANDEDNESS: unknown
      (D) TOPOLOGY: unknown
   (ii) MOLECULE TYPE: cDNA
   (ix) FEATURE:
      (A) NAME/KEY: CDS
      (B) LOCATION: 1..426
   (xi) SEQUENCE DESCRIPTION: SEQ ID NO:45:
   ATG TCC TAC GAC TAC ATG GAA GGT GGT GAC ATC CGC GTA CGT CGT CTG      48
   Met Ser Tyr Asp Tyr Met Glu Gly Gly Asp Ile Arg Val Arg Arg Leu
    1               5                  10                  15
   TTC TGC CGT ACC CAG TGG TAC CTG CGT ATC GAC AAA CGC GGC AAA GTC      96
   Phe Cys Arg Thr Gln Trp Tyr Leu Arg Ile Asp Lys Arg Gly Lys Val
               20                  25                  30
   AAG GGC ACC CAA GAG ATG AAA AAC AAC TAC AAT ATT ATG GAA ATC CGT     144
   Lys Gly Thr Gln Glu Met Lys Asn Asn Tyr Asn Ile Met Glu Ile Arg
           35                  40                  45
   ACT GTT GCT GTT GGT ATC GTT GCA ATC AAA GGT GTT GAA TCT GAA TTC     192
   Thr Val Ala Val Gly Ile Val Ala Ile Lys Gly Val Glu Ser Glu Phe
       50                  55                  60
   TAC CTG GCA ATG AAC AAA GAA GGT AAA CTG TAC GCA AAA AAA GAA TGC     240
   Tyr Leu Ala Met Asn Lys Glu Gly Lys Leu Tyr Ala Lys Lys Glu Cys
    65                  70                  75                  80
   AAC GAA GAC TGC AAC TTC AAA GAA CTG ATC CTG GAA AAC CAC TAC AAC     288
   Asn Glu Asp Cys Asn Phe Lys Glu Leu Ile Leu Glu Asn His Tyr Asn
                   85                  90                  95
   ACC TAC GCA TCT GCT AAA TGG ACC CAC AAC GGT GGT GAA ATG TTC GTT     336
   Thr Tyr Ala Ser Ala Lys Trp Thr His Asn Gly Gly Glu Met Phe Val
               100                 105                 110
   GCT CTG GAA CAG AAA GGT ATC CCT GTT CGT GGT AAG AAA ACC AAG AAA     384
   Ala Leu Glu Gln Lys Gly Ile Pro Val Arg Gly Lys Lys Thr Lys Lys
           115                 120                 125
   GAA CAG AAA ACC GCT CAC TTC CTG CCG ATG GCA ATC ACT TAA             426
   Glu Gln Lys Thr Ala His Phe Leu Pro Met Ala Ile Thr  *
       130                 135                 140
(2) INFORMATION FOR SEQ ID NO:46:
   (i) SEQUENCE CHARACTERISTICS:
      (A) LENGTH: 142 amino acids
      (B) TYPE: amino acid
      (D) TOPOLOGY: linear
   (ii) MOLECULE TYPE: protein
   (xi) SEQUENCE DESCRIPTION: SEQ ID NO:46:
   Met Ser Tyr Asp Tyr Met Glu Gly Gly Asp Ile Arg Val Arg Arg Leu
    1               5                  10                  15
   Phe Cys Arg Thr Gln Trp Tyr Leu Arg Ile Asp Lys Arg Gly Lys Val
               20                  25                  30
   Lys Gly Thr Gln Glu Met Lys Asn Asn Tyr Asn Ile Met Glu Ile Arg
           35                  40                  45
   Thr Val Ala Val Gly Ile Val Ala Ile Lys Gly Val Glu Ser Glu Phe
       50                  55                  60
   Tyr Leu Ala Met Asn Lys Glu Gly Lys Leu Tyr Ala Lys Lys Glu Cys
    65                  70                  75                  80
   Asn Glu Asp Cys Asn Phe Lys Glu Leu Ile Leu Glu Asn His Tyr Asn
                    85                  90                  95
   Thr Tyr Ala Ser Ala Lys Trp Thr His Asn Gly Gly Glu Met Phe Val
               100                 105                 110
   Ala Leu Glu Gln Lys Gly Ile Pro Val Arg Gly Lys Lys Thr Lys Lys
           115                 120                 125
   Glu Gln Lys Thr Ala His Phe Leu Pro Met Ala Ile Thr  *
       130                 135                 140
(2) INFORMATION FOR SEQ ID NO:47:
   (i) SEQUENCE CHARACTERISTICS:
      (A) LENGTH: 426 base pairs
      (B) TYPE: nucleic acid
      (C) STRANDEDNESS: unknown
      (D) TOPOLOGY: unknown
   (ii) MOLECULE TYPE: cDNA
   (ix) FEATURE:
      (A) NAME/KEY: CDS
      (B) LOCATION: 1..426
   (xi) SEQUENCE DESCRIPTION: SEQ ID NO:47:
   ATG TCC TAC GAC TAC ATG GAA GGT GGT GAC ATC CGC GTA CGT CGT CTG       48
   Met Ser Tyr Asp Tyr Met Glu Gly Gly Asp Ile Arg Val Arg Arg Leu
    1               5                  10                  15
   TTC TGC CGT ACC CAG TGG TAC CTG CGT ATC GAC AAA CGC GGC AAA GTC       96
   Phe Cys Arg Thr Gln Trp Tyr Leu Arg Ile Asp Lys Arg Gly Lys Val
               20                  25                  30
   AAG GGC ACC CAA GAG ATG AAA AAC AAC TAC AAT ATT ATG GAA ATC CGT      144
   Lys Gly Thr Gln Glu Met Lys Asn Asn Tyr Asn Ile Met Glu Ile Arg
           35                  40                  45
   ACT GTT GCT GTT GGT ATC GTT GCA ATC AAA GGT GTT GAA TCT GAA TTC      192
   Thr Val Ala Val Gly Ile Val Ala Ile Lys Gly Val Glu Ser Glu Phe
       50                  55                  60
   TAC CTG GCA ATG AAC AAA GAA GGT AAA CTG TAC GCA AAA AAA GAA TGC          240
   Tyr Leu Ala Met Asn Lys Glu Gly Lys Leu Tyr Ala Lys Lys Glu Cys
    65                 70                  75                  80
   AAC GAA GAC TGC AAC TTC AAA GAA CTG ATC CTG GAA AAC CAC TAC AAC          288
   Asn Glu Asp Cys Asn Phe Lys Glu Leu Ile Leu Glu Asn His Tyr Asn
                    85                  90                  95
   ACC TAC GCA TCT GCT AAA TGG ACC CAC AAC GGT GGT GAA ATG TTC GTT          336
   Thr Tyr Ala Ser Ala Lys Trp Thr His Asn Gly Gly Glu Met Phe Val
               100                 105                 110
   GCT CTG AAC CAG GAA GGT ATC CCT GTT CGT GGT AAG AAA ACC AAG AAA          384
   Ala Leu Asn Gln Glu Gly Ile Pro Val Arg Gly Lys Lys Thr Lys Lys
           115                 120                 125
   GAA CAG AAA ACC GCT CAC TTC CTG CCG ATG GCA ATC ACT TAA                  426
   Glu Gln Lys Thr Ala His Phe Leu Pro Met Ala Ile Thr  *
       130                 135                 140
(2) INFORMATION FOR SEQ ID NO:48:
   (i) SEQUENCE CHARACTERISTICS:
      (A) LENGTH: 142 amino acids
      (B) TYPE: amino acid
      (D) TOPOLOGY: linear
   (ii) MOLECULE TYPE: protein
   (xi) SEQUENCE DESCRIPTION: SEQ ID NO:48:
   Met Ser Tyr Asp Tyr Met Glu Gly Gly Asp Ile Arg Val Arg Arg Leu
     1               5                  10                  15
   Phe Cys Arg Thr Gln Trp Tyr Leu Arg Ile Asp Lys Arg Gly Lys Val
               20                  25                  30
   Lys Gly Thr Gln Glu Met Lys Asn Asn Tyr Asn Ile Met Glu Ile Arg
           35                  40                  45
   Thr Val Ala Val Gly Ile Val Ala Ile Lys Gly Val Glu Ser Glu Phe
       50                  55                  60
   Tyr Leu Ala Met Asn Lys Glu Gly Lys Leu Tyr Ala Lys Lys Glu Cys
    65                  70                  75                  80
   Asn Glu Asp Cys Asn Phe Lys Glu Leu Ile Leu Glu Asn His Tyr Asn
                    85                  90                  95
   Thr Tyr Ala Ser Ala Lys Trp Thr His Asn Gly Gly Glu Met Phe Val
               100                 105                 110
   Ala Leu Asn Gln Glu Gly Ile Pro Val Arg Gly Lys Lys Thr Lys Lys
           115                 120                 125
   Glu Gln Lys Thr Ala His Phe Leu Pro Met Ala Ile Thr  *
       130                 135                 140
(2) INFORMATION FOR SEQ ID NO:49:
   (i) SEQUENCE CHARACTERISTICS:
      (A) LENGTH: 426 base pairs
      (B) TYPE: nucleic acid
      (C) STRANDEDNESS: unknown
      (D) TOPOLOGY: unknown
   (ii) MOLECULE TYPE: cDNA
   (ix) FEATURE:
      (A) NAME/KEY: CDS
      (B) LOCATION: 1..426
   (xi) SEQUENCE DESCRIPTION: SEQ ID NO:49:
   ATG TCC TAC GAC TAC ATG GAA GGT GGT GAC ATC CGC GTA CGT CGT CTG        48
   Met Ser Tyr Asp Tyr Met Glu Gly Gly Asp Ile Arg Val Arg Arg Leu
     1               5                  10                  15
   TTC TGC CGT ACC CAG TGG TAC CTG CGT ATC GAC AAA CGC GGC AAA GTC        96
   Phe Cys Arg Thr Gln Trp Tyr Leu Arg Ile Asp Lys Arg Gly Lys Val
                20                  25                  30
   AAG GGC ACC CAA GAG ATG AAA AAC AAC TAC AAT ATT ATG GAA ATC CGT       144
   Lys Gly Thr Gln Glu Met Lys Asn Asn Tyr Asn Ile Met Glu Ile Arg
            35                  40                  45
   ACT GTT GCT GTT GGT ATC GTT GCA ATC AAA GGT GTT GAA TCT GAA TTC       192
   Thr Val Ala Val Gly Ile Val Ala Ile Lys Gly Val Glu Ser Glu Phe
        50                  55                  60
   TAC CTG GCA ATG AAC AAA GAA GGT AAA CTG TAC GCA AAA AAA GAA TGC       240
   Tyr Leu Ala Met Asn Lys Glu Gly Lys Leu Tyr Ala Lys Lys Glu Cys
    65                  70                  75                  80
   AAC GAA GAC TGC AAC TTC AAA GAA CTG ATC CTG GAA AAC CAC TAC AAC       288
   Asn Glu Asp Cys Asn Phe Lys Glu Leu Ile Leu Glu Asn His Tyr Asn
                    85                  90                  95
   ACC TAC GCA TCT GCT AAA TGG ACC CAC AAC GGT GGT GAA ATG TTC GTT       336
   Thr Tyr Ala Ser Ala Lys Trp Thr His Asn Gly Gly Glu Met Phe Val
               100                 105                 110
   GCT CTG AAC CAG CAA GGT ATC CCT GTT CGT GGT AAG AAA ACC AAG AAA       384
   Ala Leu Asn Gln Gln Gly Ile Pro Val Arg Gly Lys Lys Thr Lys Lys
           115                 120                 125
   GAA CAG AAA ACC GCT CAC TTC CTG CCG ATG GCA ATC ACT TAA               426
   Glu Gln Lys Thr Ala His Phe Leu Pro Met Ala Ile Thr  *
       130                 135                 140
(2) INFORMATION FOR SEQ ID NO:50:
   (i) SEQUENCE CHARACTERISTICS:
      (A) LENGTH: 142 amino acids
      (B) TYPE: amino acid
      (D) TOPOLOGY: linear
   (ii) MOLECULE TYPE: protein
   (xi) SEQUENCE DESCRIPTION: SEQ ID NO:50:
   Met Ser Tyr Asp Tyr Met Glu Gly Gly Asp Ile Arg Val Arg Arg Leu
    1               5                  10                  15
   Phe Cys Arg Thr Gln Trp Tyr Leu Arg Ile Asp Lys Arg Gly Lys Val
               20                  25                  30
   Lys Gly Thr Gln Glu Met Lys Asn Asn Tyr Asn Ile Met Glu Ile Arg
           35                  40                  45
   Thr Val Ala Val Gly Ile Val Ala Ile Lys Gly Val Glu Ser Glu Phe
       50                  55                  60
   Tyr Leu Ala Met Asn Lys Glu Gly Lys Leu Tyr Ala Lys Lys Glu Cys
   65                  70                  75                  80
   Asn Glu Asp Cys Asn Phe Lys Glu Leu Ile Leu Glu Asn His Tyr Asn
                   85                  90                  95
   Thr Tyr Ala Ser Ala Lys Trp Thr His Asn Gly Gly Glu Met Phe Val
               100                 105                 110
   Ala Leu Asn Gln Gln Gly Ile Pro Val Arg Gly Lys Lys Thr Lys Lys
           115                 120                 125
   Glu Gln Lys Thr Ala His Phe Leu Pro Met Ala Ile Thr  *
       130                 135                 140
(2) INFORMATION FOR SEQ ID NO:51:
   (i) SEQUENCE CHARACTERISTICS:
      (A) LENGTH: 426 base pairs
      (B) TYPE: nucleic acid
      (C) STRANDEDNESS: unknown
      (D) TOPOLOGY: unknown
   (ii) MOLECULE TYPE: cDNA
   (ix) FEATURE:
      (A) NAME/KEY: CDS
      (B) LOCATION: 1..426
   (xi) SEQUENCE DESCRIPTION: SEQ ID NO:51:
   ATG TCC TAC GAC TAC ATG GAA GGT GGT GAC ATC CGC GTA CGT CGT CTG       48
   Met Ser Tyr Asp Tyr Met Glu Gly Gly Asp Ile Arg Val Arg Arg Leu
    1               5                  10                  15
   TTC TGC CGT ACC CAG TGG TAC CTG CGT ATC GAC AAA CGC GGC AAA GTC       96
   Phe Cys Arg Thr Gln Trp Tyr Leu Arg Ile Asp Lys Arg Gly Lys Val
               20                  25                  30
   AAG GGC ACC CAA GAG ATG AAA AAC AAC TAC AAT ATT ATG GAA ATC CGT      144
   Lys Gly Thr Gln Glu Met Lys Asn Asn Tyr Asn Ile Met Glu Ile Arg
           35                  40                  45
   ACT GTT GCT GTT GGT ATC GTT GCA ATC AAA GGT GTT GAA TCT GAA TTC        192
   Thr Val Ala Val Gly Ile Val Ala Ile Lys Gly Val Glu Ser Glu Phe
        50                  55                  60
   TAC CTG GCA ATG AAC AAA GAA GGT AAA CTG TAC GCA AAA AAA GAA TGC        240
   Tyr Leu Ala Met Asn Lys Glu Gly Lys Leu Tyr Ala Lys Lys Glu Cys
    65                  70                  75                  80
   AAC GAA GAC TGC AAC TTC AAA GAA CTG ATC CTG GAA AAC CAC TAC AAC        288
   Asn Glu Asp Cys Asn Phe Lys Glu Leu Ile Leu Glu Asn His Tyr Asn
                    85                  90                  95
   ACC TAC GCA TCT GCT AAA TGG ACC CAC AAC GGT GGT GAA ATG TTC GTT        336
   Thr Tyr Ala Ser Ala Lys Trp Thr His Asn Gly Gly Glu Met Phe Val
               100                 105                 110
   GCT CTG AAC CAG AAA GGT ATC CCT GTT GCT GGT AAG AAA ACC AAG AAA        384
   Ala Leu Asn Gln Lys Gly Ile Pro Val Ala Gly Lys Lys Thr Lys Lys
           115                 120                 125
   GAA CAG AAA ACC GCT CAC TTC CTG CCG ATG GCA ATC ACT TAA                426
   Glu Gln Lys Thr Ala His Phe Leu Pro Met Ala Ile Thr  *
       130                 135                 140
(2) INFORMATION FOR SEQ ID NO:52:
   (i) SEQUENCE CHARACTERISTICS:
      (A) LENGTH: 142 amino acids
      (B) TYPE: amino acid
      (D) TOPOLOGY: linear
   (ii) MOLECULE TYPE: protein
   (xi) SEQUENCE DESCRIPTION: SEQ ID NO:52:
   Met Ser Tyr Asp Tyr Met Glu Gly Gly Asp Ile Arg Val Arg Arg Leu
     1               5                  10                  15
   Phe Cys Arg Thr Gln Trp Tyr Leu Arg Ile Asp Lys Arg Gly Lys Val
               20                  25                  30
   Lys Gly Thr Gln Glu Met Lys Asn Asn Tyr Asn Ile Met Glu Ile Arg
           35                  40                  45
   Thr Val Ala Val Gly Ile Val Ala Ile Lys Gly Val Glu Ser Glu Phe
       50                  55                  60
   Tyr Leu Ala Met Asn Lys Glu Gly Lys Leu Tyr Ala Lys Lys Glu Cys
    65                  70                  75                  80
   Asn Glu Asp Cys Asn Phe Lys Glu Leu Ile Leu Glu Asn His Tyr Asn
                    85                  90                  95
   Thr Tyr Ala Ser Ala Lys Trp Thr His Asn Gly Gly Glu Met Phe Val
               100                 105                 110
   Ala Leu Asn Gln Lys Gly Ile Pro Val Ala Gly Lys Lys Thr Lys Lys
           115                 120                 125
   Glu Gln Lys Thr Ala His Phe Leu Pro Met Ala Ile Thr  *
       130                 135                 140
(2) INFORMATION FOR SEQ ID NO:53:
   (i) SEQUENCE CHARACTERISTICS:
      (A) LENGTH: 426 base pairs
      (B) TYPE: nucleic acid
      (C) STRANDEDNESS: unknown
      (D) TOPOLOGY: unknown
   (ii) MOLECULE TYPE: cDNA
   (ix) FEATURE:
      (A) NAME/KEY: CDS
      (B) LOCATION: 1..426
   (xi) SEQUENCE DESCRIPTION: SEQ ID NO:53:
   ATG TCC TAC GAC TAC ATG GAA GGT GGT GAC ATC CGC GTA CGT CGT CTG      48
   Met Ser Tyr Asp Tyr Met Glu Gly Gly Asp Ile Arg Val Arg Arg Leu
    1               5                  10                  15
   TTC TGC CGT ACC CAG TGG TAC CTG CGT ATC GAC AAA CGC GGC AAA GTC      96
   Phe Cys Arg Thr Gln Trp Tyr Leu Arg Ile Asp Lys Arg Gly Lys Val
               20                  25                  30
   AAG GGC ACC CAA GAG ATG AAA AAC AAC TAC AAT ATT ATG GAA ATC CGT     144
   Lys Gly Thr Gln Glu Met Lys Asn Asn Tyr Asn Ile Met Glu Ile Arg
           35                  40                  45
   ACT GTT GCT GTT GGT ATC GTT GCA ATC AAA GGT GTT GAA TCT GAA TTC     192
   Thr Val Ala Val Gly Ile Val Ala Ile Lys Gly Val Glu Ser Glu Phe
       50                  55                  60
   TAT CTT GCA ATG AAC AAG GAA GGA AAA CTC TAT GCA AAG AAA GAA TGC     240
   Tyr Leu Ala Met Asn Lys Glu Gly Lys Leu Tyr Ala Lys Lys Glu Cys
   65                  70                  75                  80
   AAT GAA GAT TGT AAC TTC AAA GAA CTA ATT CTG GAA AAC CAT TAC AAC     288
   Asn Glu Asp Cys Asn Phe Lys Glu Leu Ile Leu Glu Asn His Tyr Asn
                   85                  90                  95
   ACA TAT GCA TCT GCT AAA TGG ACC CAC AAC GGT GGT GAA ATG TTC GTT     336
   Thr Tyr Ala Ser Ala Lys Trp Thr His Asn Gly Gly Glu Met Phe Val
               100                 105                 110
   GCT CTG AAC CAG AAA GGT ATC CCT GTT GAA GGT AAG AAA ACC AAG AAA     384
   Ala Leu Asn Gln Lys Gly Ile Pro Val Glu Gly Lys Lys Thr Lys Lys
           115                 120                 125
   GAA CAG AAA ACC GCT CAC TTC CTG CCG ATG GCA ATC ACT TAA             426
   Glu Gln Lys Thr Ala His Phe Leu Pro Met Ala Ile Thr  *
       130                 135                 140
(2) INFORMATION FOR SEQ ID NO:54:
   (i) SEQUENCE CHARACTERISTICS:
      (A) LENGTH: 142 amino acids
      (B) TYPE: amino acid
      (D) TOPOLOGY: linear
   (ii) MOLECULE TYPE: protein
   (xi) SEQUENCE DESCRIPTION: SEQ ID NO:54:
   Met Ser Tyr Asp Tyr Met Glu Gly Gly Asp Ile Arg Val Arg Arg Leu
    1               5                  10                  15
   Phe Cys Arg Thr Gln Trp Tyr Leu Arg Ile Asp Lys Arg Gly Lys Val
               20                  25                  30
   Lys Gly Thr Gln Glu Met Lys Asn Asn Tyr Asn Ile Met Glu Ile Arg
           35                  40                  45
   Thr Val Ala Val Gly Ile Val Ala Ile Lys Gly Val Glu Ser Glu Phe
       50                  55                  60
   Tyr Leu Ala Met Asn Lys Glu Gly Lys Leu Tyr Ala Lys Lys Glu Cys
   65                  70                  75                  80
   Asn Glu Asp Cys Asn Phe Lys Glu Leu Ile Leu Glu Asn His Tyr Asn
                   85                  90                  95
   Thr Tyr Ala Ser Ala Lys Trp Thr His Asn Gly Gly Glu Met Phe Val
               100                 105                 110
   Ala Leu Asn Gln Lys Gly Ile Pro Val Glu Gly Lys Lys Thr Lys Lys
           115                 120                 125
   Glu Gln Lys Thr Ala His Phe Leu Pro Met Ala Ile Thr  *
       130                 135                 140
(2) INFORMATION FOR SEQ ID NO:55:
   (i) SEQUENCE CHARACTERISTICS:
      (A) LENGTH: 426 base pairs
      (B) TYPE: nucleic acid
      (C) STRANDEDNESS: unknown
      (D) TOPOLOGY: unknown
   (ii) MOLECULE TYPE: cDNA
   (ix) FEATURE:
      (A) NAME/KEY: CDS
      (B) LOCATION: 1..426
   (xi) SEQUENCE DESCRIPTION: SEQ ID NO:55:
   ATG TCC TAC GAC TAC ATG GAA GGT GGT GAC ATC CGC GTA CGT CGT CTG         48
   Met Ser Tyr Asp Tyr Met Glu Gly Gly Asp Ile Arg Val Arg Arg Leu
    1               5                  10                  15
   TTC TGC CGT ACC CAG TGG TAC CTG CGT ATC GAC AAA CGC GGC AAA GTC         96
   Phe Cys Arg Thr Gln Trp Tyr Leu Arg Ile Asp Lys Arg Gly Lys Val
               20                  25                  30
   AAG GGC ACC CAA GAG ATG AAA AAC AAC TAC AAT ATT ATG GAA ATC CGT        144
   Lys Gly Thr Gln Glu Met Lys Asn Asn Tyr Asn Ile Met Glu Ile Arg
           35                  40                  45
   ACT GTT GCT GTT GGT ATC GTT GCA ATC AAA GGT GTT GAA TCT GAA TTC    192
   Thr Val Ala Val Gly Ile Val Ala Ile Lys Gly Val Glu Ser Glu Phe
        50                  55                  60
   TAC CTG GCA ATG AAC AAA GAA GGT AAA CTG TAC GCA AAA AAA GAA TGC    240
   Tyr Leu Ala Met Asn Lys Glu Gly Lys Leu Tyr Ala Lys Lys Glu Cys
    65                  70                  75                  80
   AAC GAA GAC TGC AAC TTC AAA GAA CTG ATC CTG GAA AAC CAC TAC AAC    288
   Asn Glu Asp Cys Asn Phe Lys Glu Leu Ile Leu Glu Asn His Tyr Asn
                    85                  90                  95
   ACC TAC GCA TCT GCT AAA TGG ACC CAC AAC GGT GGT GAA ATG TTC GTT    336
   Thr Tyr Ala Ser Ala Lys Trp Thr His Asn Gly Gly Glu Met Phe Val
               100                 105                 110
   GCT CTG AAC CAG AAA GGT ATC CCT GTT CTG GGT AAG AAA ACC AAG AAA    384
   Ala Leu Asn Gln Lys Gly Ile Pro Val Leu Gly Lys Lys Thr Lys Lys
           115                 120                 125
   GAA CAG AAA ACC GCT CAC TTC CTG CCG ATG GCA ATC ACT TAA            426
   Glu Gln Lys Thr Ala His Phe Leu Pro Met Ala Ile Thr  *
       130                 135                 140
(2) INFORMATION FOR SEQ ID NO:56:
   (i) SEQUENCE CHARACTERISTICS:
      (A) LENGTH: 142 amino acids
      (B) TYPE: amino acid
      (D) TOPOLOGY: linear
   (ii) MOLECULE TYPE: protein
   (xi) SEQUENCE DESCRIPTION: SEQ ID NO:56:
   Met Ser Tyr Asp Tyr Met Glu Gly Gly Asp Ile Arg Val Arg Arg Leu
    1               5                  10                  15
   Phe Cys Arg Thr Gln Trp Tyr Leu Arg Ile Asp Lys Arg Gly Lys Val
               20                  25                  30
   Lys Gly Thr Gln Glu Met Lys Asn Asn Tyr Asn Ile Met Glu Ile Arg
           35                  40                  45
   Thr Val Ala Val Gly Ile Val Ala Ile Lys Gly Val Glu Ser Glu Phe
       50                  55                  60
   Tyr Leu Ala Met Asn Lys Glu Gly Lys Leu Tyr Ala Lys Lys Glu Cys
   65                  70                  75                  80
   Asn Glu Asp Cys Asn Phe Lys Glu Leu Ile Leu Glu Asn His Tyr Asn
                   85                  90                  95
   Thr Tyr Ala Ser Ala Lys Trp Thr His Asn Gly Gly Glu Met Phe Val
               100                 105                 110
   Ala Leu Asn Gln Lys Gly Ile Pro Val Leu Gly Lys Lys Thr Lys Lys
           115                 120                 125
   Glu Gln Lys Thr Ala His Phe Leu Pro Met Ala Ile Thr  *
       130                 135                 140
(2) INFORMATION FOR SEQ ID NO:57:
   (i) SEQUENCE CHARACTERISTICS:
      (A) LENGTH: 426 base pairs
      (B) TYPE: nucleic acid
      (C) STRANDEDNESS: unknown
      (D) TOPOLOGY: unknown
   (ii) MOLECULE TYPE: CDNA
   (ix) FEATURE:
      (A) NAME/KEY: CDS
      (B) LOCATION: 1..426
   (xi) SEQUENCE DESCRIPTION: SEQ ID NO:57:
   ATG TCC TAC GAC TAC ATG GAA GGT GGT GAC ATC CGC GTA CGT CGT CTG      48
   Met Ser Tyr Asp Tyr Met Glu Gly Gly Asp Ile Arg Val Arg Arg Leu
    1               5                  10                  15
   TTC TGC CGT ACC CAG TGG TAC CTG CGT ATC GAC AAA CGC GGC AAA GTC      96
   Phe Cys Arg Thr Gln Trp Tyr Leu Arg Ile Asp Lys Arg Gly Lys Val
               20                  25                  30
   AAG GGC ACC CAA GAG ATG AAA AAC AAC TAC AAT ATT ATG GAA ATC CGT     144
   Lys Gly Thr Gln Glu Met Lys Asn Asn Tyr Asn Ile Met Glu Ile Arg
           35                  40                  45
   ACT GTT GCT GTT GGT ATC GTT GCA ATC AAA GGT GTT GAA TCT GAA TTC     192
   Thr Val Ala Val Gly Ile Val Ala Ile Lys Gly Val Glu Ser Glu Phe
       50                  55                  60
   TAC CTG GCA ATG AAC AAA GAA GGT AAA CTG TAC GCA AAA AAA GAA TGC     240
   Tyr Leu Ala Met Asn Lys Glu Gly Lys Leu Tyr Ala Lys Lys Glu Cys
   65                  70                  75                  80
   AAC GAA GAC TGC AAC TTC AAA GAA CTG ATC CTG GAA AAC CAC TAC AAC     288
   Asn Glu Asp Cys Asn Phe Lys Glu Leu Ile Leu Glu Asn His Tyr Asn
                   85                  90                  95
   ACC TAC GCA TCT GCT AAA TGG ACC CAC AAC GGT GGT GAA ATG TTC GTT     336
   Thr Tyr Ala Ser Ala Lys Trp Thr His Asn Gly Gly Glu Met Phe Val
               100                 105                 110
   GCT CTG AAC CAG AAA GGT ATT CCT GTT CGT GGT AAG GAA ACC AAG AAA     384
   Ala Leu Asn Gln Lys Gly Ile Pro Val Arg Gly Lys Glu Thr Lys Lys
           115                 120                 125
   GAA CAG AAA ACC GCT CAC TTC CTG CCG ATG GCA ATC ACT TAA             426
   Glu Gln Lys Thr Ala His Phe Leu Pro Met Ala Ile Thr  *
       130                 135                 140
(2) INFORMATION FOR SEQ ID NO:58:
   (i) SEQUENCE CHARACTERISTICS:
      (A) LENGTH: 142 amino acids
      (B) TYPE: amino acid
      (D) TOPOLOGY: linear
   (ii) MOLECULE TYPE: protein
   (xi) SEQUENCE DESCRIPTION: SEQ ID NO:58:
   Met Ser Tyr Asp Tyr Met Glu Gly Gly Asp Ile Arg Val Arg Arg Leu
    1               5                  10                  15
   Phe Cys Arg Thr Gln Trp Tyr Leu Arg Ile Asp Lys Arg Gly Lys Val
               20                  25                  30
   Lys Gly Thr Gln Glu Met Lys Asn Asn Tyr Asn Ile Met Glu Ile Arg
           35                  40                  45
   Thr Val Ala Val Gly Ile Val Ala Ile Lys Gly Val Glu Ser Glu Phe
       50                  55                  60
   Tyr Leu Ala Met Asn Lys Glu Gly Lys Leu Tyr Ala Lys Lys Glu Cys
   65                  70                  75                  80
   Asn Glu Asp Cys Asn Phe Lys Glu Leu Ile Leu Glu Asn His Tyr Asn
                   85                  90                  95
   Thr Tyr Ala Ser Ala Lys Trp Thr His Asn Gly Gly Glu Met Phe Val
               100                 105                 110
   Ala Leu Asn Gln Lys Gly Ile Pro Val Arg Gly Lys Glu Thr Lys Lys
           115                 120                 125
   Glu Gln Lys Thr Ala His Phe Leu Pro Met Ala Ile Thr  *
       130                 135                 140
(2) INFORMATION FOR SEQ ID NO:59:
   (i) SEQUENCE CHARACTERISTICS:
      (A) LENGTH: 426 base pairs
      (B) TYPE: nucleic acid
      (C) STRANDEDNESS: unknown
      (D) TOPOLOGY: unknown
   (ii) MOLECULE TYPE: cDNA
   (ix) FEATURE:
      (A) NAME/KEY: CDS
      (B) LOCATION: 1..426
   (xi) SEQUENCE DESCRIPTION: SEQ ID NO:59:
   ATG TCC TAC GAC TAC ATG GAA GGT GGT GAC ATC CGC GTA CGT CGT CTG        48
   Met Ser Tyr Asp Tyr Met Glu Gly Gly Asp Ile Arg Val Arg Arg Leu
    1               5                  10                  15
   TTC TGC CGT ACC CAG TGG TAC CTG CGT ATC GAC AAA CGC GGC AAA GTC        96
   Phe Cys Arg Thr Gln Trp Tyr Leu Arg Ile Asp Lys Arg Gly Lys Val
               20                  25                  30
   AAG GGC ACC CAA GAG ATG AAA AAC AAC TAC AAT ATT ATG GAA ATC CGT       144
   Lys Gly Thr Gln Glu Met Lys Asn Asn Tyr Asn Ile Met Glu Ile Arg
           35                  40                  45
   ACT GTT GCT GTT GGT ATC GTT GCA ATC AAA GGT GTT GAA TCT GAA TTC        192
   Thr Val Ala Val Gly Ile Val Ala Ile Lys Gly Val Glu Ser Glu Phe
        50                  55                  60
   TAC CTG GCA ATG AAC AAA GAA GGT AAA CTG TAC GCA AAA AAA GAA TGC        240
   Tyr Leu Ala Met Asn Lys Glu Gly Lys Leu Tyr Ala Lys Lys Glu Cys
    65                  70                  75                  80
   AAC GAA GAC TGC AAC TTC AAA GAA CTG ATC CTG GAA AAC CAC TAC AAC        288
   Asn Glu Asp Cys Asn Phe Lys Glu Leu Ile Leu Glu Asn His Tyr Asn
                    85                  90                  95
   ACC TAC GCA TCT GCT AAA TGG ACC CAC AAC GGT GGT GAA ATG TTC GTT        336
   Thr Tyr Ala Ser Ala Lys Trp Thr His Asn Gly Gly Glu Met Phe Val
               100                 105                 110
   GCT CTG AAC CAG AAA GGT ATC CCT GTT CGT GGT AAG CAA ACC AAG AAA        384
   Ala Leu Asn Gln Lys Gly Ile Pro Val Arg Gly Lys Gln Thr Lys Lys
           115                 120                 125
   GAA CAG AAA ACC GCT CAC TTC CTG CCG ATG GCA ATC ACT TAA                426
   Glu Gln Lys Thr Ala His Phe Leu Pro Met Ala Ile Thr  *
       130                 135                 140
(2) INFORMATION FOR SEQ ID NO:60:
   (i) SEQUENCE CHARACTERISTICS:
      (A) LENGTH: 142 amino acids
      (B) TYPE: amino acid
      (D) TOPOLOGY: linear
   (ii) MOLECULE TYPE: protein
   (xi) SEQUENCE DESCRIPTION: SEQ ID NO:60:
   Met Ser Tyr Asp Tyr Met Glu Gly Gly Asp Ile Arg Val Arg Arg Leu
    1               5                  10                  15
   Phe Cys Arg Thr Gln Trp Tyr Leu Arg Ile Asp Lys Arg Gly Lys Val
               20                  25                  30
   Lys Gly Thr Gln Glu Met Lys Asn Asn Tyr Asn Ile Met Glu Ile Arg
           35                  40                  45
   Thr Val Ala Val Gly Ile Val Ala Ile Lys Gly Val Glu Ser Glu Phe
       50                  55                  60
   Tyr Leu Ala Met Asn Lys Glu Gly Lys Leu Tyr Ala Lys Lys Glu Cys
   65                  70                  75                  80
   Asn Glu Asp Cys Asn Phe Lys Glu Leu Ile Leu Glu Asn His Tyr Asn
                   85                  90                  95
   Thr Tyr Ala Ser Ala Lys Trp Thr His Asn Gly Gly Glu Met Phe Val
               100                 105                 110
   Ala Leu Asn Gln Lys Gly Ile Pro Val Arg Gly Lys Gln Thr Lys Lys
           115                 120                 125
   Glu Gln Lys Thr Ala His Phe Leu Pro Met Ala Ile Thr  *
       130                 135                 140
(2) INFORMATION FOR SEQ ID NO:61:
   (i) SEQUENCE CHARACTERISTICS:
      (A) LENGTH: 426 base pairs
      (B) TYPE: nucleic acid
      (C) STRANDEDNESS: unknown
      (D) TOPOLOGY: unknown
   (ii) MOLECULE TYPE: cDNA
   (ix) FEATURE:
      (A) NAME/KEY: CDS
      (B) LOCATION: 1..426
   (xi) SEQUENCE DESCRIPTION: SEQ ID NO:61:
   ATG TCC TAC GAC TAC ATG GAA GGT GGT GAC ATC CGC GTA CGT CGT CTG        48
   Met Ser Tyr Asp Tyr Met Glu Gly Gly Asp Ile Arg Val Arg Arg Leu
     1               5                  10                  15
   TTC TGC CGT ACC CAG TGG TAC CTG CGT ATC GAC AAA CGC GGC AAA GTC        96
   Phe Cys Arg Thr Gln Trp Tyr Leu Arg Ile Asp Lys Arg Gly Lys Val
                20                  25                  30
   AAG GGC ACC CAA GAG ATG AAA AAC AAC TAC AAT ATT ATG GAA ATC CGT       144
   Lys Gly Thr Gln Glu Met Lys Asn Asn Tyr Asn Ile Met Glu Ile Arg
            35                  40                  45
   ACT GTT GCT GTT GGT ATC GTT GCA ATC AAA GGT GTT GAA TCT GAA TTC       192
   Thr Val Ala Val Gly Ile Val Ala Ile Lys Gly Val Glu Ser Glu Phe
        50                  55                  60
   TAC CTG GCA ATG AAC AAA GAA GGT AAA CTG TAC GCA AAA AAA GAA TGC       240
   Tyr Leu Ala Met Asn Lys Glu Gly Lys Leu Tyr Ala Lys Lys Glu Cys
    65                  70                  75                  80
   AAC GAA GAC TGC AAC TTC AAA GAA CTG ATC CTG GAA AAC CAC TAC AAC       288
   Asn Glu Asp Cys Asn Phe Lys Glu Leu Ile Leu Glu Asn His Tyr Asn
                    85                  90                  95
   ACC TAC GCA TCT GCT AAA TGG ACC CAC AAC GGT GGT GAA ATG TTC GTT       336
   Thr Tyr Ala Ser Ala Lys Trp Thr His Asn Gly Gly Glu Met Phe Val
               100                 105                 110
   GCT CTG AAC CAG AAA GGT ATC CCT GTT CGT GGT AAG AAA ACC AAG AAA       384
   Ala Leu Asn Gln Lys Gly Ile Pro Val Arg Gly Lys Lys Thr Lys Lys
           115                 120                 125
   GAA CAG GAA ACC GCT CAC TTC CTG CCG ATG GCA ATC ACT TAA               426
   Glu Gln Glu Thr Ala His Phe Leu Pro Met Ala Ile Thr  *
       130                 135                 140
(2) INFORMATION FOR SEQ ID NO:62:
   (i) SEQUENCE CHARACTERISTICS:
      (A) LENGTH: 142 amino acids
      (B) TYPE: amino acid
      (D) TOPOLOGY: linear
   (ii) MOLECULE TYPE: protein
   (xi) SEQUENCE DESCRIPTION: SEQ ID NO:62:
   Met Ser Tyr Asp Tyr Met Glu Gly Gly Asp Ile Arg Val Arg Arg Leu
    1               5                  10                  15
   Phe Cys Arg Thr Gln Trp Tyr Leu Arg Ile Asp Lys Arg Gly Lys Val
               20                  25                  30
   Lys Gly Thr Gln Glu Met Lys Asn Asn Tyr Asn Ile Met Glu Ile Arg
           35                  40                  45
   Thr Val Ala Val Gly Ile Val Ala Ile Lys Gly Val Glu Ser Glu Phe
       50                  55                  60
   Tyr Leu Ala Met Asn Lys Glu Gly Lys Leu Tyr Ala Lys Lys Glu Cys
   65                  70                  75                  80
   Asn Glu Asp Cys Asn Phe Lys Glu Leu Ile Leu Glu Asn His Tyr Asn
                   85                  90                  95
   Thr Tyr Ala Ser Ala Lys Trp Thr His Asn Gly Gly Glu Met Phe Val
               100                 105                 110
   Ala Leu Asn Gln Lys Gly Ile Pro Val Arg Gly Lys Lys Thr Lys Lys
           115                 120                 125
   Glu Gln Glu Thr Ala His Phe Leu Pro Met Ala Ile Thr  *
       130                 135                 140
(2) INFORMATION FOR SEQ ID NO:63:
   (i) SEQUENCE CHARACTERISTICS:
      (A) LENGTH: 426 base pairs
      (B) TYPE: nucleic acid
      (C) STRANDEDNESS: unknown
      (D) TOPOLOGY: unknown
   (ii) MOLECULE TYPE: cDNA
   (ix) FEATURE:
      (A) NAME/KEY: CDS
      (B) LOCATION: 1..426
   (xi) SEQUENCE DESCRIPTION: SEQ ID NO:63:
   ATG TCC TAC GAC TAC ATG GAA GGT GGT GAC ATC CGC GTA CGT CGT CTG         48
   Met Ser Tyr Asp Tyr Met Glu Gly Gly Asp Ile Arg Val Arg Arg Leu
     1               5                  10                  15
   TTC TGC CGT ACC CAG TGG TAC CTG CGT ATC GAC AAA CGC GGC AAA GTC         96
   Phe Cys Arg Thr Gln Trp Tyr Leu Arg Ile Asp Lys Arg Gly Lys Val
                20                  25                  30
   AAG GGC ACC CAA GAG ATG AAA AAC AAC TAC AAT ATT ATG GAA ATC CGT        144
   Lys Gly Thr Gln Glu Met Lys Asn Asn Tyr Asn Ile Met Glu Ile Arg
            35                  40                  45
   ACT GTT GCT GTT GGT ATC GTT GCA ATC AAA GGT GTT GAA TCT GAA TTC        192
   Thr Val Ala Val Gly Ile Val Ala Ile Lys Gly Val Glu Ser Glu Phe
        50                  55                  60
   TAC CTG GCA ATG AAC AAA GAA GGT AAA CTG TAC GCA AAA AAA GAA TGC        240
   Tyr Leu Ala Met Asn Lys Glu Gly Lys Leu Tyr Ala Lys Lys Glu Cys
    65                  70                  75                  80
   AAC GAA GAC TGC AAC TTC AAA GAA CTG ATC CTG GAA AAC CAC TAC AAC        288
   Asn Glu Asp Cys Asn Phe Lys Glu Leu Ile Leu Glu Asn His Tyr Asn
                    85                  90                  95
   ACC TAC GCA TCT GCT AAA TGG ACC CAC AAC GGT GGT GAA ATG TTC GTT        336
   Thr Tyr Ala Ser Ala Lys Trp Thr His Asn Gly Gly Glu Met Phe Val
               100                 105                 110
   GCT CTG AAC CAG AAA GGT ATC CCT GTT CGT GGT AAG AAA ACC AAG AAA        384
   Ala Leu Asn Gln Lys Gly Ile Pro Val Arg Gly Lys Lys Thr Lys Lys
           115                 120                 125
   GAA CAG CAA ACC GCT CAC TTC CTG CCG ATG GCA ATC ACT TAA                426
   Glu Gln Gln Thr Ala His Phe Leu Pro Met Ala Ile Thr  *
       130                 135                 140
(2) INFORMATION FOR SEQ ID NO:64:
   (i) SEQUENCE CHARACTERISTICS:
      (A) LENGTH: 142 amino acids
      (B) TYPE: amino acid
      (D) TOPOLOGY: linear
   (ii) MOLECULE TYPE: protein
   (xi) SEQUENCE DESCRIPTION: SEQ ID NO:64:
   Met Ser Tyr Asp Tyr Met Glu Gly Gly Asp Ile Arg Val Arg Arg Leu
    1               5                  10                  15
   Phe Cys Arg Thr Gln Trp Tyr Leu Arg Ile Asp Lys Arg Gly Lys Val
               20                  25                  30
   Lys Gly Thr Gln Glu Met Lys Asn Asn Tyr Asn Ile Met Glu Ile Arg
           35                  40                  45
   Thr Val Ala Val Gly Ile Val Ala Ile Lys Gly Val Glu Ser Glu Phe
       50                  55                  60
   Tyr Leu Ala Met Asn Lys Glu Gly Lys Leu Tyr Ala Lys Lys Glu Cys
   65                  70                  75                  80
   Asn Glu Asp Cys Asn Phe Lys Glu Leu Ile Leu Glu Asn His Tyr Asn
                   85                  90                  95
   Thr Tyr Ala Ser Ala Lys Trp Thr His Asn Gly Gly Glu Met Phe Val
               100                 105                 110
   Ala Leu Asn Gln Lys Gly Ile Pro Val Arg Gly Lys Lys Thr Lys Lys
           115                 120                 125
   Glu Gln Gln Thr Ala His Phe Leu Pro Met Ala Ile Thr  *
       130                 135                 140
(2) INFORMATION FOR SEQ ID NO:65:
   (i) SEQUENCE CHARACTERISTICS:
      (A) LENGTH: 426 base pairs
      (B) TYPE: nucleic acid
      (C) STRANDEDNESS: unknown
      (D) TOPOLOGY: unknown
   (ii) MOLECULE TYPE: cDNA
   (ix) FEATURE:
      (A) NAME/KEY: CDS
      (B) LOCATION: 1..426
   (xi) SEQUENCE DESCRIPTION: SEQ ID NO:65:
   ATG TCC TAC GAC TAC ATG GAA GGT GGT GAC ATC CGC GTA CGT CGT CTG      48
   Met Ser Tyr Asp Tyr Met Glu Gly Gly Asp Ile Arg Val Arg Arg Leu
    1               5                  10                  15
   TTC TGC CGT ACC CAG TGG TAC CTG CGT ATC GAC AAA CGC GGC AAA GTC      96
   Phe Cys Arg Thr Gln Trp Tyr Leu Arg Ile Asp Lys Arg Gly Lys Val
               20                  25                  30
   AAG GGC ACC CAA GAG ATG AAA AAC AAC TAC AAT ATT ATG GAA ATC CGT     144
   Lys Gly Thr Gln Glu Met Lys Asn Asn Tyr Asn Ile Met Glu Ile Arg
           35                  40                  45
   ACT GTT GCT GTT GGT ATC GTT GCA ATC AAA GGT GTT GAA TCT GAA TTC     192
   Thr Val Ala Val Gly Ile Val Ala Ile Lys Gly Val Glu Ser Glu Phe
       50                  55                  60
   TAC CTG GCA ATG AAC AAA GAA GGT AAA CTG TAC GCA AAA AAA GAA TGC     240
   Tyr Leu Ala Met Asn Lys Glu Gly Lys Leu Tyr Ala Lys Lys Glu Cys
   65                  70                  75                  80
   AAC GAA GAC TGC AAC TTC AAA GAA CTG ATC CTG GAA AAC CAC TAC AAC     288
   Asn Glu Asp Cys Asn Phe Lys Glu Leu Ile Leu Glu Asn His Tyr Asn
                   85                  90                  95
   ACC TAC GCA TCT GCT AAA TGG ACC CAC AAC GGT GGT GAA ATG TTC GTT     336
   Thr Tyr Ala Ser Ala Lys Trp Thr His Asn Gly Gly Glu Met Phe Val
               100                 105                 110
   GCT CTG AAC CAG AAA GGT ATC CCT GTT CGT GGT AAG AAA ACC AAG AAA     384
   Ala Leu Asn Gln Lys Gly Ile Pro Val Arg Gly Lys Lys Thr Lys Lys
           115                 120                 125
   GAA GAG GAA ACC GCT CAC TTC CTG CCG ATG GCA ATC ACT TAA             426
   Glu Glu Glu Thr Ala His Phe Leu Pro Met Ala Ile Thr  *
       130                 135                 140
(2) INFORMATION FOR SEQ ID NO:66:
   (i) SEQUENCE CHARACTERISTICS:
      (A) LENGTH: 142 amino acids
      (B) TYPE: amino acid
      (D) TOPOLOGY: linear
   (ii) MOLECULE TYPE: protein
   (xi) SEQUENCE DESCRIPTION: SEQ ID NO:66:
   Met Ser Tyr Asp Tyr Met Glu Gly Gly Asp Ile Arg Val Arg Arg Leu
    1               5                  10                  15
   Phe Cys Arg Thr Gln Trp Tyr Leu Arg Ile Asp Lys Arg Gly Lys Val
               20                  25                  30
   Lys Gly Thr Gln Glu Met Lys Asn Asn Tyr Asn Ile Met Glu Ile Arg
           35                  40                  45
   Thr Val Ala Val Gly Ile Val Ala Ile Lys Gly Val Glu Ser Glu Phe
       50                  55                  60
   Tyr Leu Ala Met Asn Lys Glu Gly Lys Leu Tyr Ala Lys Lys Glu Cys
   65                  70                  75                  80
   Asn Glu Asp Cys Asn Phe Lys Glu Leu Ile Leu Glu Asn His Tyr Asn
                   85                  90                  95
   Thr Tyr Ala Ser Ala Lys Trp Thr His Asn Gly Gly Glu Met Phe Val
               100                 105                 110
   Ala Leu Asn Gln Lys Gly Ile Pro Val Arg Gly Lys Lys Thr Lys Lys
           115                 120                 125
   Glu Glu Glu Thr Ala His Phe Leu Pro Met Ala Ile Thr  *
       130                 135                 140
(2) INFORMATION FOR SEQ ID NO:67:
   (i) SEQUENCE CHARACTERISTICS:
      (A) LENGTH: 33 amino acids
      (B) TYPE: amino acid
      (C) STRANDEDNESS: unknown
      (D) TOPOLOGY: unknown
   (ii) MOLECULE TYPE: protein
   (xi) SEQUENCE DESCRIPTION: SEQ ID NO:67:
   His Ala Asp Gly Ser Phe Ser Asp Glu Met Asn Thr Ile Leu Asp Asn
   1               5                   10                  15
   Leu Ala Ala Arg Asp Phe Ile Asn Trp Leu Ile Gln Thr Lys Ile Thr
               20                  25                  30
   Asp
(2) INFORMATION FOR SEQ ID NO:68:
   (i) SEQUENCE CHARACTERISTICS:
      (A) LENGTH: 33 amino acids
      (B) TYPE: amino acid
      (C) STRANDEDNESS: unknown
      (D) TOPOLOGY: unknown
   (ii) MOLECULE TYPE: protein
   (xi) SEQUENCE DESCRIPTION: SEQ ID NO:68:
   His Gly Asp Gly Ser Phe Ser Asp Glu Met Asn Thr Ile Leu Asp Asn
   1               5                   10                  15
   Leu Ala Ala Arg Asp Phe Ile Asn Trp Leu Ile Gln Thr Lys Ile Thr
               20                  25                  30
   Asp
(2) INFORMATION FOR SEQ ID NO:69:
   (i) SEQUENCE CHARACTERISTICS:
      (A) LENGTH: 33 amino acids
      (B) TYPE: amino acid
      (C) STRANDEDNESS: unknown
      (D) TOPOLOGY: unknown
   (ii) MOLECULE TYPE: protein
   (xi) SEQUENCE DESCRIPTION: SEQ ID NO:69:
   His Ser Asp Gly Ser Phe Ser Asp Glu Met Asn Thr Ile Leu Asp Asn
   1               5                   10                  15
   Leu Ala Ala Arg Asp Phe Ile Asn Trp Leu Ile Gln Thr Lys Ile Thr
               20                  25                  30
   Asp

## Description

### FIELD OF THE INVENTION

The present invention relates to the combined use of keratinocyte growth factor protein products and glucagon-like peptide 2 protein products in the preparation of medicaments.

### BACKGROUND OF THE INVENTION

The complex process of tissue generation and regeneration is mediated by a number of protein factors sometimes referred to as soft tissue growth factors. These molecules are generally released by one cell type and act to influence proliferation of other cell types (Rubin et al. (1989). *Proc*. *Nat'l*. *Acad*. *Sci*. *USA,* 86:802-806). There are also some growth factors released from cells that themselves have the capacity to respond to such growth factors. Some soft tissue growth factors are secreted by particular cell types and influence the proliferation, differentiation, and/or maturation of responsive cells in the development of multicellular organisms (Finch et al. (1989), *Science,* 245:752-755). In addition to their roles in developing organisms, some soft tissue growth factors are significant in the continued health and maintenance of more mature systems. For instance, in mammals there are many systems where rapid cell turnover occurs. Such systems include the skin and the gastrointestinal tract, both of which are comprised of epithelial cells. Included within this group of soft tissue growth factors is a protein family of fibroblast growth factors (FGFs).

The fibroblast growth factor (FGF) family is now known to consist of at least fourteen members, namely FGF-1 to FGF-10 and homologous factors FHF-1 to FHF-4, which share a relatedness among primary structures: basic fibroblast growth factor, bFGF (Abraham et al. (1986), *EMBO J.,* 5:2523-2528); acidic fibroblast growth factor, aFGF (Jaye et al. (1986), *Science,* 233:541-545); int-2 gene product, int-2 (Dickson & Peters (1987), *Nature,* 326:833); hst/kFGF (Delli-Bovi et al. (1987), *Cell,* 50:729-737 and Yoshida et al. (1987), *Proc, Natl*. Acad. *Sci. USA,* 84:7305-7309); FGF-5 (Zhan et al. (1988), *Mol*. *Cell. Biol.,* 8:3487-3495); FGF-6 (Marics et al. (1989), *Oncogene,* 4:335-340); keratinocyte growth factor, KGF (Finch et al. (1989), Science, 24:752-755); hisactophilin (Habazzettl et al. (1992), Nature, 359:855-858); FGF-9 (Miyamoto et al. (1993), *Mol*. *Cell Biol*., 13(7):4251-4259); and fibroblast growth factor-10, also known as keratinocyte growth factor-2, KGF-2 (WO 96/25422). More recently, four homologous factors (or "FHFs") were identified from the human retina by a combination of random cDNA sequencing, searches of existing sequence databases and homology-based polymerase chain reactions (Smallwood et al. (1996), Proc. *Natl*. *Acad*. *Sci*. *USA,* 93:9850-9857). It has been proposed that FHF-1, FHF-2, FHF-3 and FHF-4 should be designated as FGF-11, FGF-12, FGF-13 and FGF-14, respectively, in accordance with the recommendation of the Nomenclature Committee (Coulier et al. (1997), *Journal of Molecular Evolution,* 44:43-56).

Drucker et al. (1996, Proc. Natl. Acad. Sci. USA 15 : 7911-6) disclose the induction of intestinal epithelial proliferation by glucagon-like peptide 2 (GLP-2) in nude mice. Brauchle et al (1996, Biological Abstracts 96 No 435010) speculates that keratinocyte growth factor (KGF) might stimulate proliferation of intestinal epithelial cells in a pancreatic manner.

The object of the present invention was to provide improved formulations for the induction of proliferation, cytoprotection and differentiation of epithelial cells.

### SUMMARY OF THE INVENTION

The present invention is directed to the use of a KGF protein product(s) and a GLP-2 protein product(s) for the preparation of a medicament for increasing the cytoprotection, proliferation and/or differentiation of epithelial cells in the gastrointestinal tract of a patient.

Another aspect of the present invention relates to the use of a KGF protein product(s) for the preparation of a medicament for increasing the cytoprotection, proliferation and/or differentiation of epithelial cells in the gastrointestinal tract of a patient, wherein said medicament is administered simultaneously, separately or sequentially with the administration of a GLP-2 protein product(s).

Yet another aspect of the present invention relates to the use of a GLP-2 protein product(s) for the preparation of a medicament for increasing the cytoprotection, proliferation and/or differentiation of epithelial cells in the gastrointestinal tract of a patient, wherein said medicament is administered simultaneously, separately or sequentially with the administration of a KGF protein product(s).

Additional aspects and advantages of the invention will be apparent to those skilled in the art upon consideration of the following description, which details the practice of the present invention.

### BRIEF DESCRIPTION OF THE FIGURES

Numerous aspects and advantages of the present invention will become apparent upon review of the Figures, wherein:

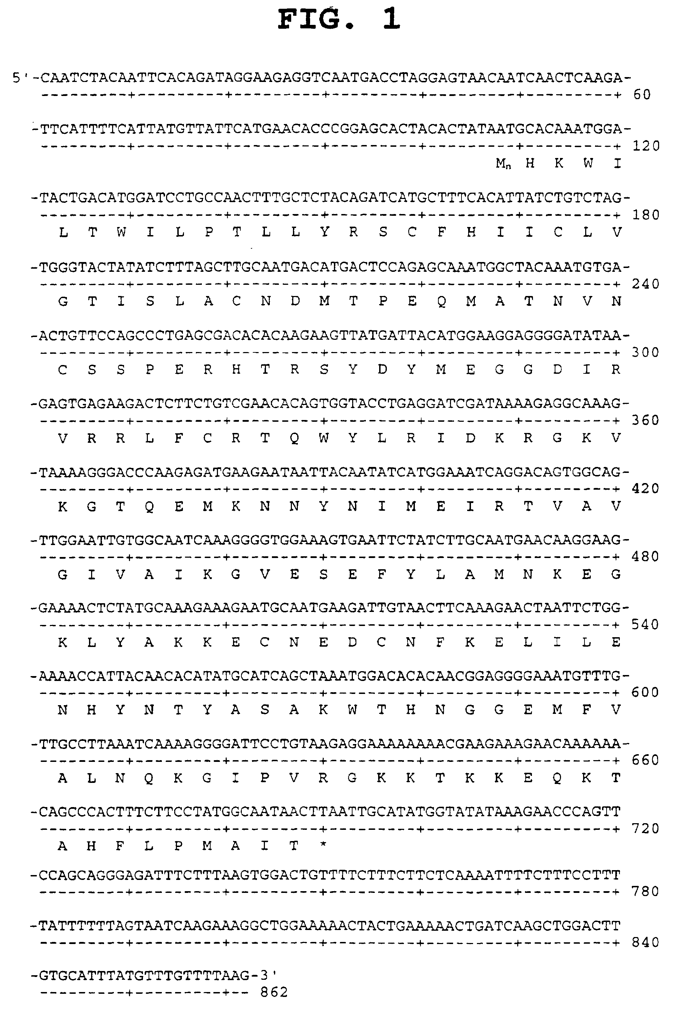

Figure 1 shows the nucleotide (SEQ ID NO:1) and amino acid (SEQ ID NO:2) sequences of KGF (the nucleotides encoding the mature form of mature KGF is depicted by bases 96 to 582 of SEQ ID NO:1 and the mature form of KGF is depicted by amino acid residues 32 to 194 of SEQ ID NO:2), with the initial amino acid being Mₙ wherein n equals 0 or 1.

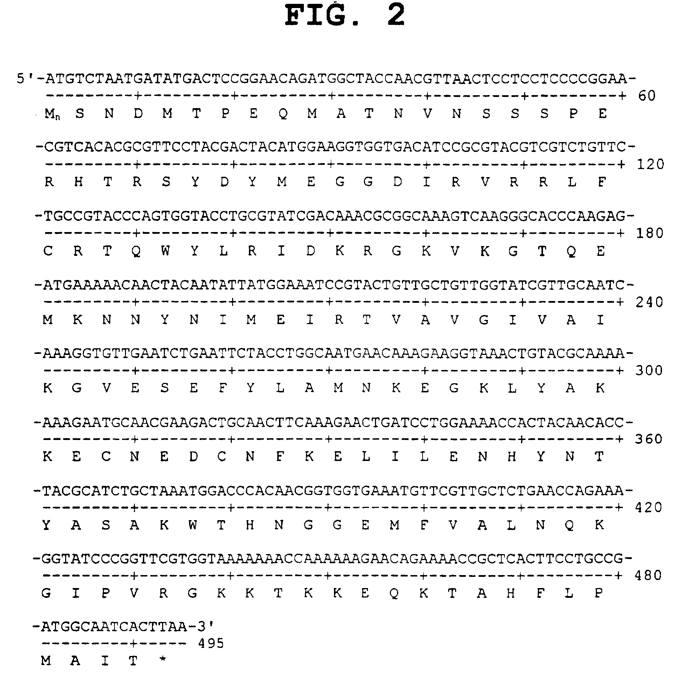

Figure 2 shows the nucleotide (SEQ ID NO:3) and amino acid (SEQ ID NO:4) sequences of C(1,15)S, a KGF analog having substitutions of serine for cysteine at amino acid positions 1 and 15 of KGF, with the initial amino acid being Mₙ wherein n equals 0 or 1.

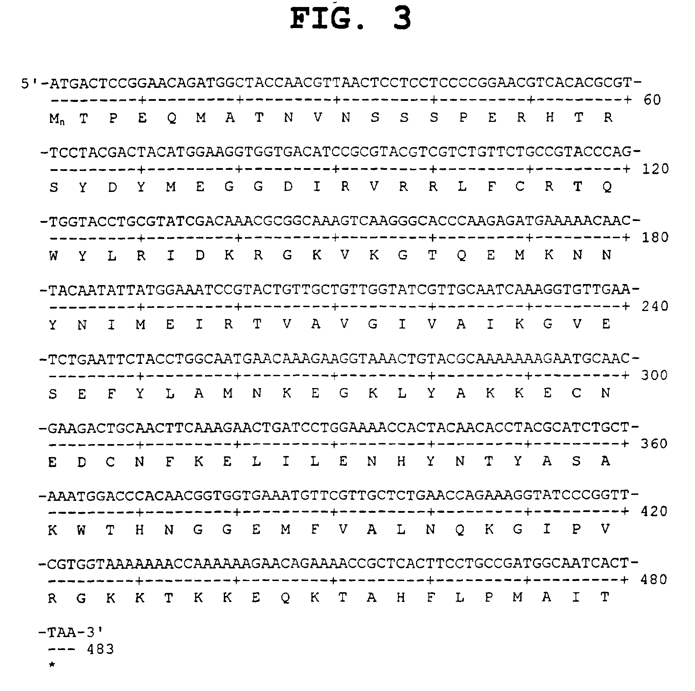

Figure 3 shows the nucleotide (SEQ ID NO:5) and amino acid (SEQ ID NO:6) sequences of ΔN3/C(15)S, a KGF analog having a deletion of the first 3 amino acids of the amino-terminus and a substitution of serine for cysteine at amino acid position 15 of KGF, with the initial amino acid being Mₙ wherein n equals 0 or 1.

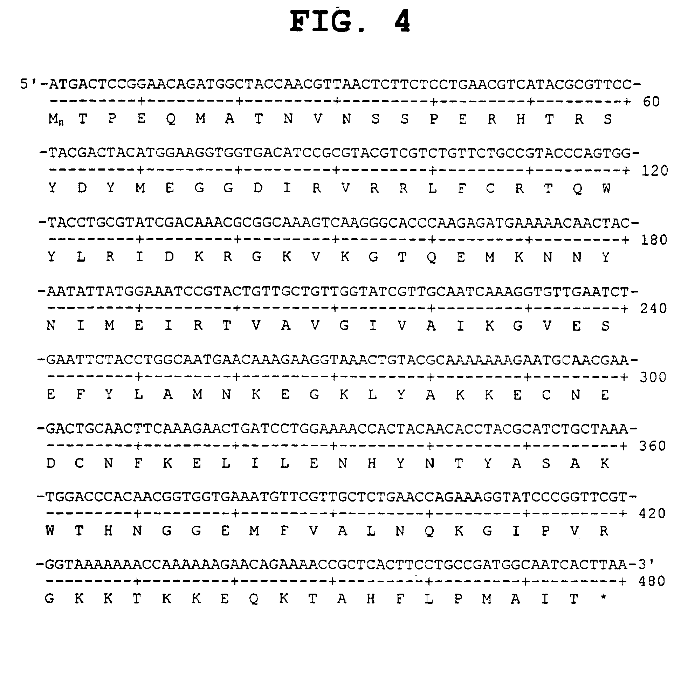

Figure 4 shows the nucleotide (SEQ ID NO:7) and amino acid (SEQ ID NO:8) sequences of ΔN3/C(15)-, a KGF analog having a deletion of the first 3 amino acids of the amino-terminus and a deletion of cysteine at amino acid position 15 of KGF, with the initial amino acid being Mₙ wherein n equals 0 or 1.

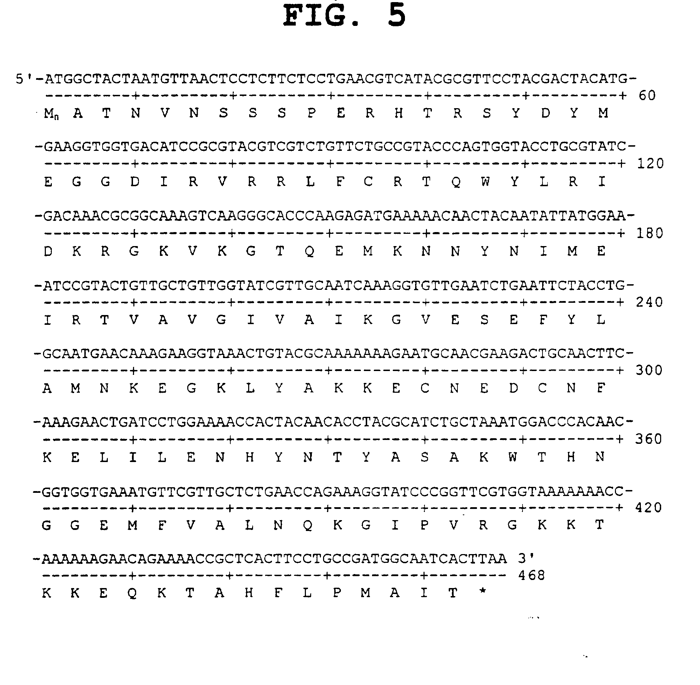

Figure 5 shows the nucleotide (SEQ ID NO:9) and amino acid (SEQ ID NO:10) sequences of ΔN8/C(15)S, a KGF analog having a deletion of the first 8 amino acids of the amino-terminus and a substitution of serine for cysteine at amino acid position 15 of KGF, with the initial amino acid being Mₙ wherein n equals 0 or 1.

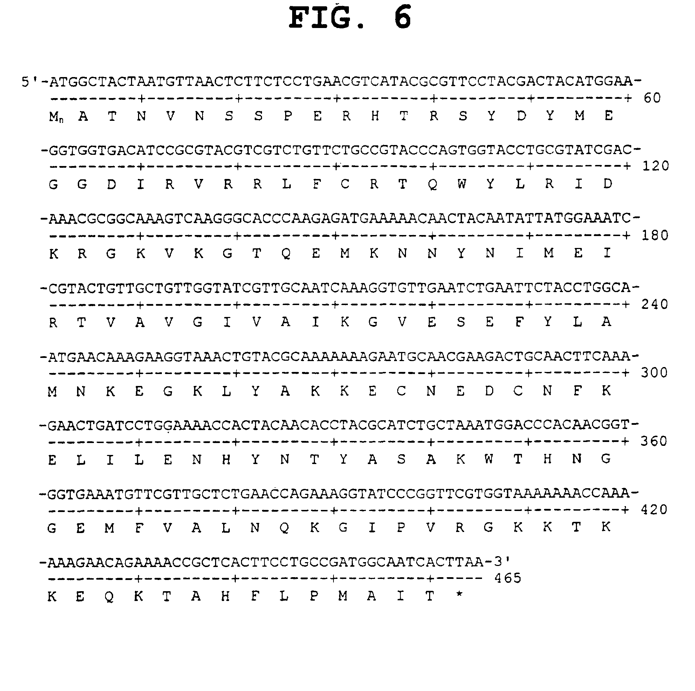

Figure 6 shows the nucleotide (SEQ ID NO:11) and amino acid (SEQ ID NO:12) sequences of ΔN8/C(15)-, a KGF analog having a deletion of the first 8 amino acids of the amino-terminus and a deletion of cysteine at amino acid position 15 of KGF, with the initial amino acid being Mₙ wherein n equals 0 or 1.

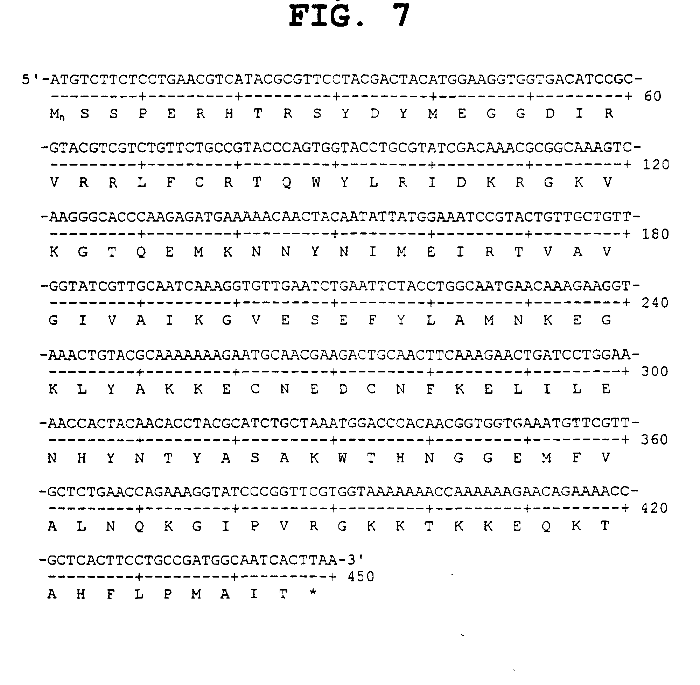

Figure 7 shows the nucleotide (SEQ ID NO:13) and amino acid (SEQ ID NO:14) sequences of ΔN15, a KGF analog having a deletion of the first 15 amino acids of the amino-terminus of KGF, with the initial amino acid being Mₙ wherein n equals 0 or 1.

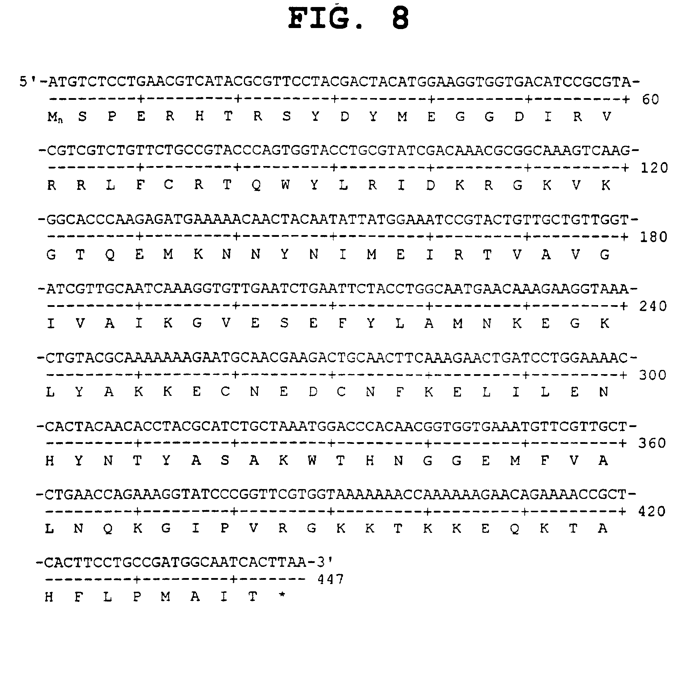

Figure 8 shows the nucleotide (SEQ ID NO:15) and amino acid (SEQ ID NO:16) sequences of ΔN16, a KGF analog having a deletion of the first 16 amino acids of the amino-terminus of KGF, with the initial amino acid being Mₙ wherein n equals 0 or 1.

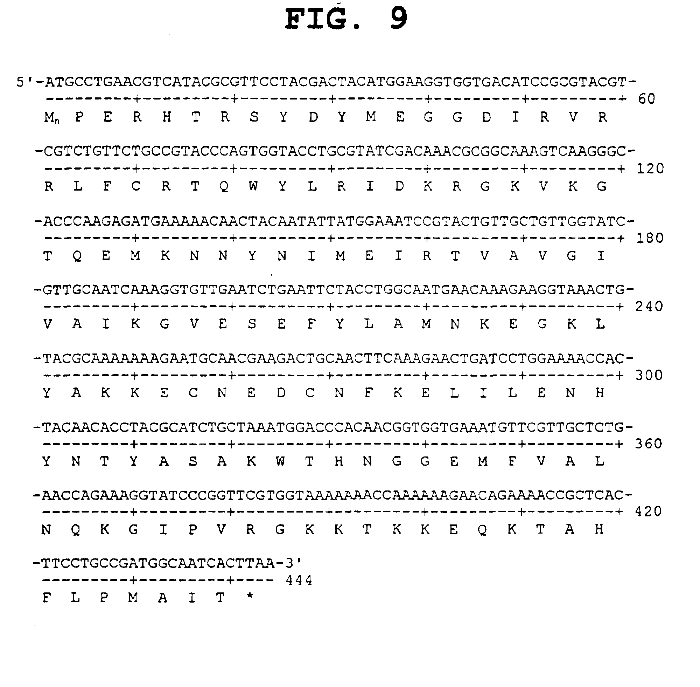

Figure 9 shows the nucleotide (SEQ ID NO:17) and amino acid (SEQ ID NO:18) sequences of ΔN17, a KGF analog having a deletion of the first 17 amino acids of the amino-terminus of KGF, with the initial amino acid being Mₙ wherein n equals 0 or 1.

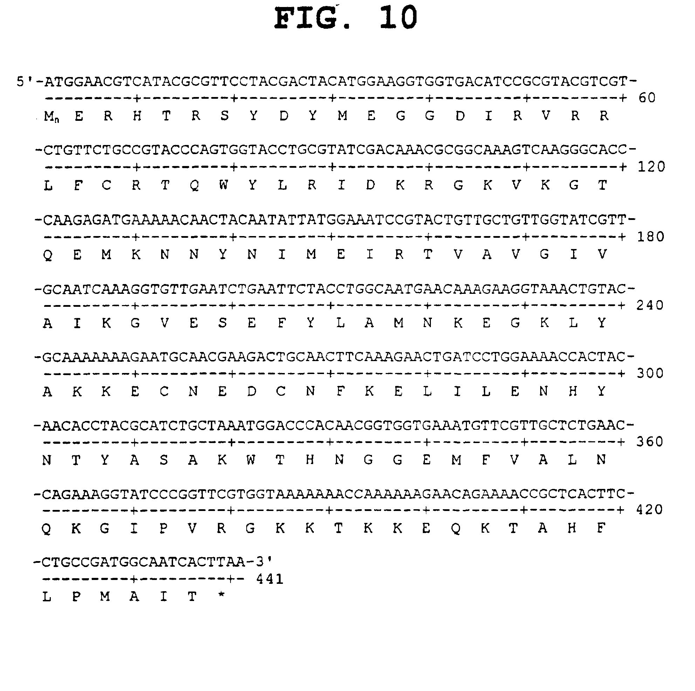

Figure 10 shows the nucleotide (SEQ ID NO:19) and amino acid (SEQ ID NO:20) sequences of ΔN18, a KGF analog having a deletion of the first 18 amino acids of the amino-terminus of KGF, with the initial amino acid being Mₙ wherein n equals 0 or 1.

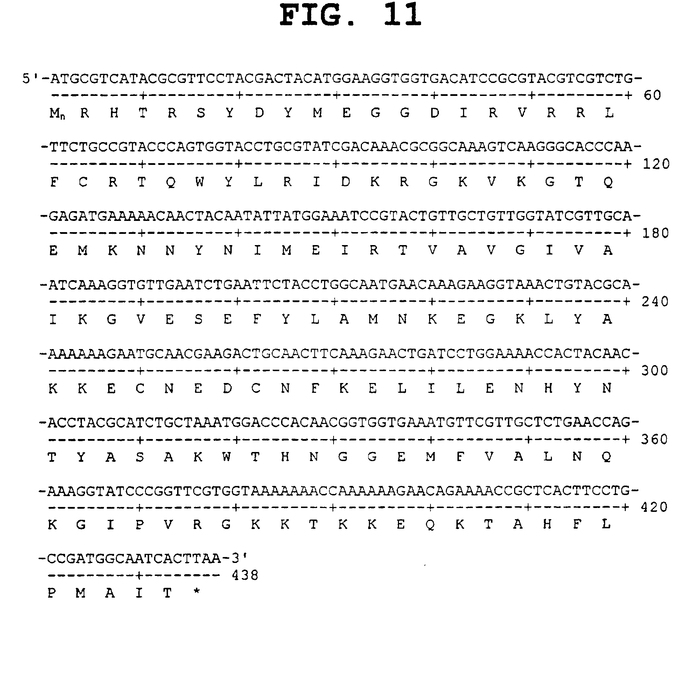

Figure 11 shows the nucleotide (SEQ ID NO:21) and amino acid (SEQ ID NO:22) sequences of ΔN19, a KGF analog having a deletion of the first 19 amino acids of the amino-terminus of KGF, with the initial amino acid being Mₙ wherein n equals 0 or 1.

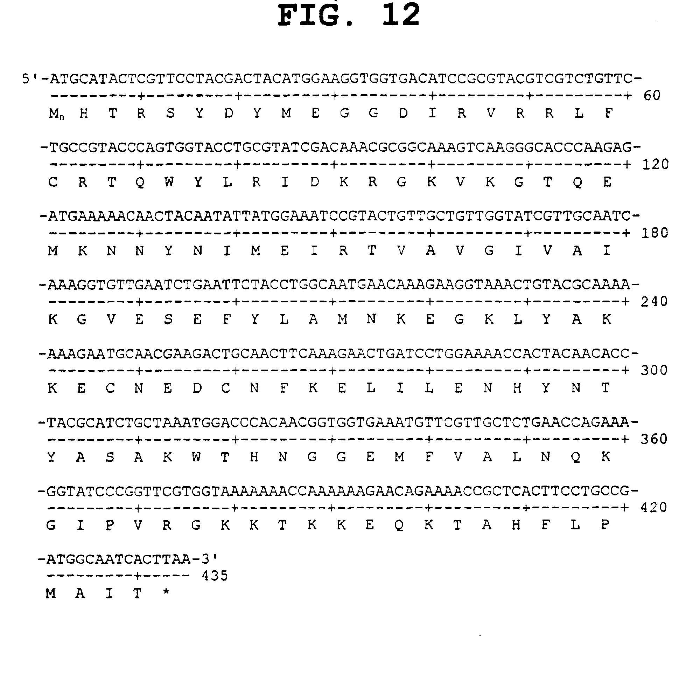

Figure 12 shows the nucleotide (SEQ ID NO:23) and amino acid (SEQ ID NO:24) sequences of ΔN20, a KGF analog having a deletion of the first 20 amino acids of the amino-terminus of KGF, with the initial amino acid being Mₙ wherein n equals 0 or 1.

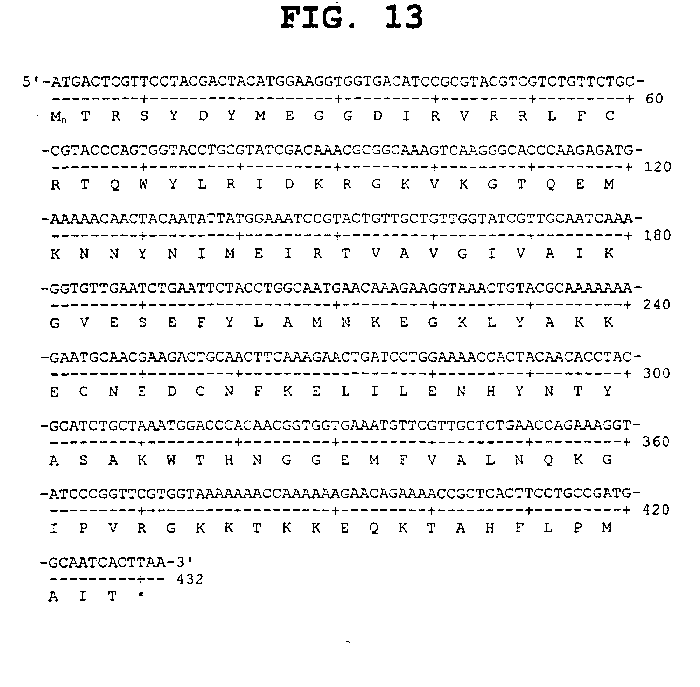

Figure 13 shows the nucleotide (SEQ ID NO:25) and amino acid (SEQ ID NO:26) sequences of ΔN21, a KGF analog having a deletion of the first 21 amino acids of the amino-terminus of KGF, with the initial amino acid being Mₙ wherein n equals 0 or 1.

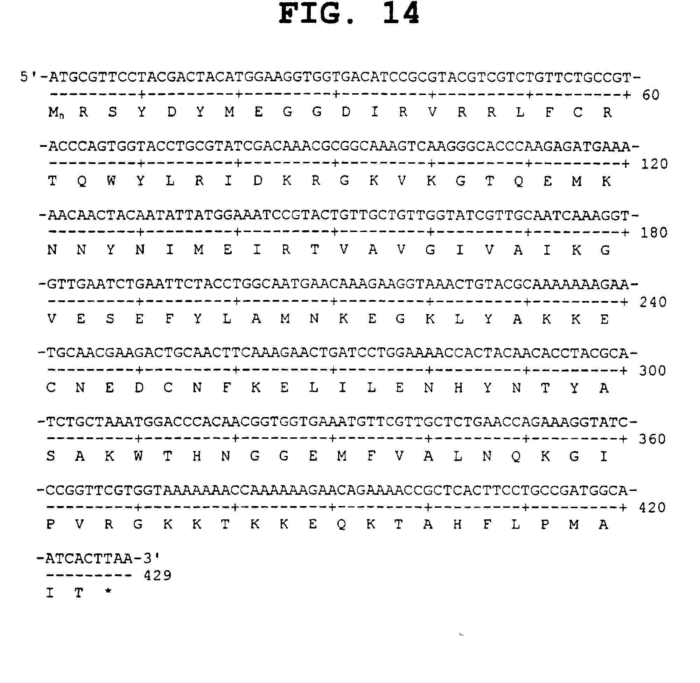

Figure 14 shows the nucleotide (SEQ ID NO:27) and amino acid (SEQ ID NO:28) sequences of ΔN22, a KGF analog having a deletion of the first 22 amino acids of the amino-terminus of KGF, with the initial amino acid being Mₙ wherein n equals 0 or 1.

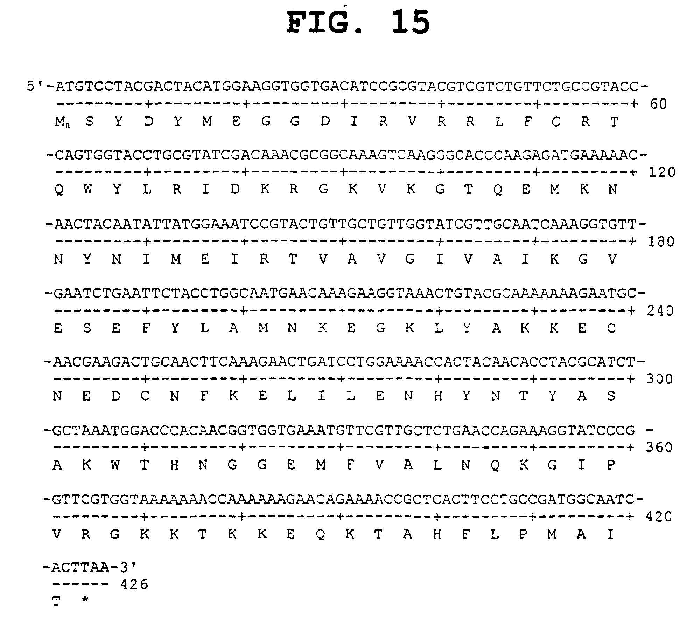

Figure 15 shows the nucleotide (SEQ ID NO:29) and amino acid (SEQ ID NO:30) sequences of ΔN23, a KGF analog having a deletion of the first 23 amino acids of the amino-terminus of KGF, with the initial amino acid being Mₙ wherein n equals 0 or 1.

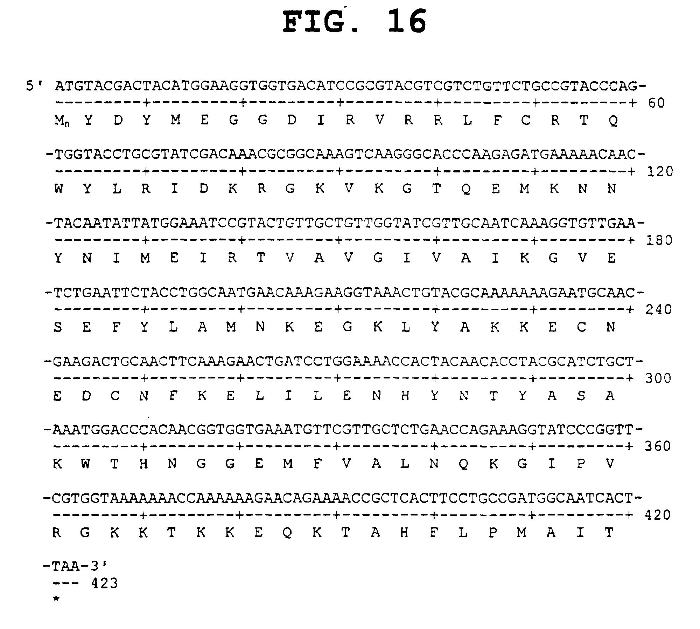

Figure 16 shows the nucleotide (SEQ ID NO:31) and amino acid (SEQ ID NO:32) sequences of ΔN24, a KGF analog having a deletion of the first 24 amino acids of the amino-terminus of KGF, with the initial amino acid being Mₙ wherein n equals 0 or 1.

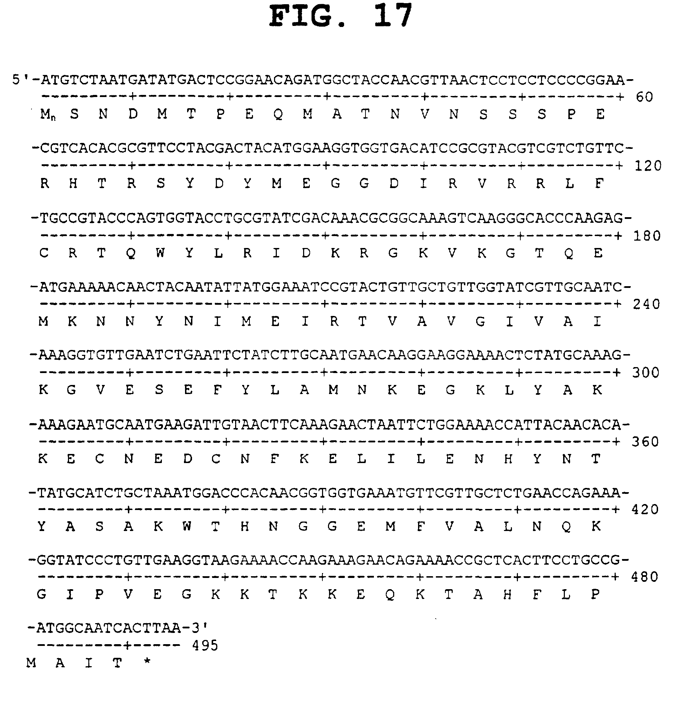

Figure 17 shows the nucleotide (SEQ ID NO:33) and amino acid (SEQ ID NO:34) sequences of C(1,15)S/R(144)E, a KGF analog having substitutions of serine for cysteine at amino acid positions 1 and 15 and a substitution of glutamic acid for arginine at amino acid position 144 of KGF, with the initial amino acid being Mₙ wherein n equals 0 or 1.

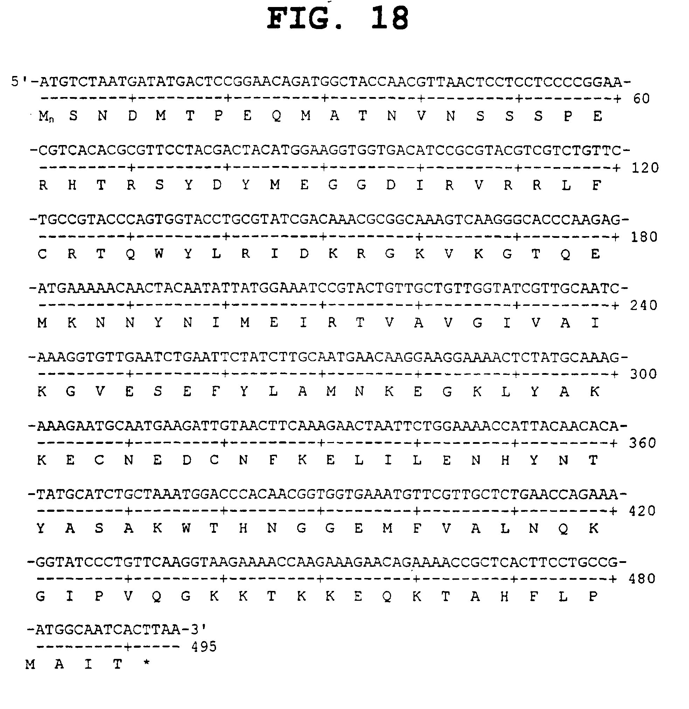

Figure 18 shows the nucleotide (SEQ ID NO:35) and amino acid (SEQ ID NO:36) sequences of C(1,15)S/R(144)Q, a KGF analog having substitutions of serine for cysteine at amino acid positions 1 and 15 and a substitution of glutamine for arginine at amino acid position 144 of KGF, with the initial amino acid being Mₙ wherein n equals 0 or 1.

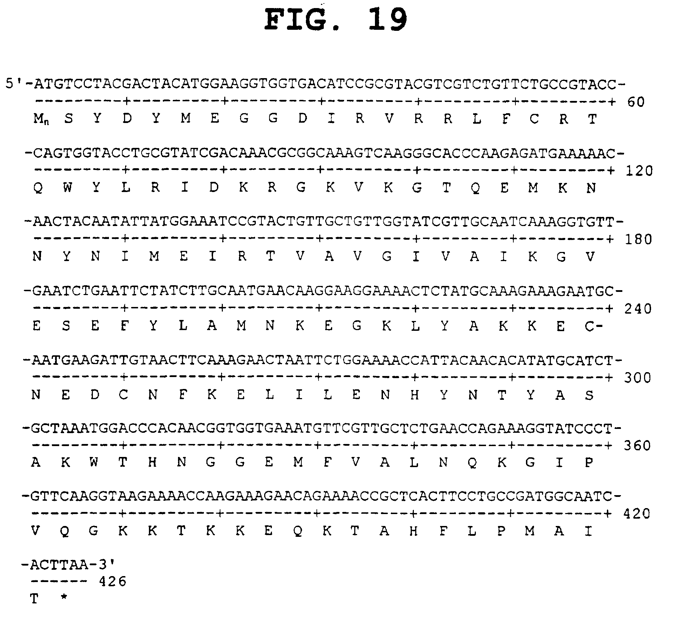

Figure 19 shows the nucleotide (SEQ ID NO:37) and amino acid (SEQ ID NO:38) sequences of ΔN23/R(144)Q, a KGF analog having a deletion of the first 23 amino acids of the amino-terminus and a substitution of glutamine for arginine at amino acid position 144 of KGF, with the initial amino acid being Mₙ wherein n equals 0 or 1.

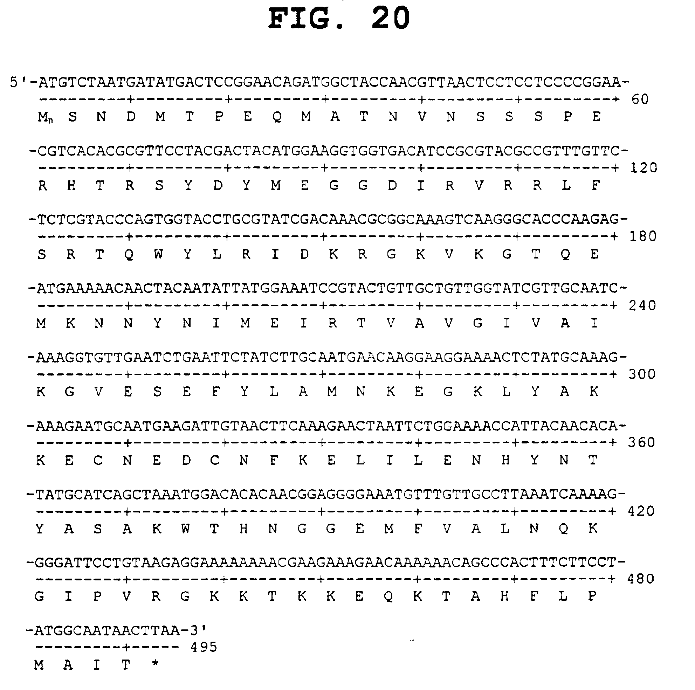

Figure 20 shows the nucleotide (SEQ ID NO:39) and amino acid (SEQ ID NO:40) sequences of C(1,15,40)S, a KGF analog having substitutions of serine for cysteine at amino acid positions 1, 15 and 40 of KGF, with the initial amino acid being Mₙ wherein n equals 0 or 1.

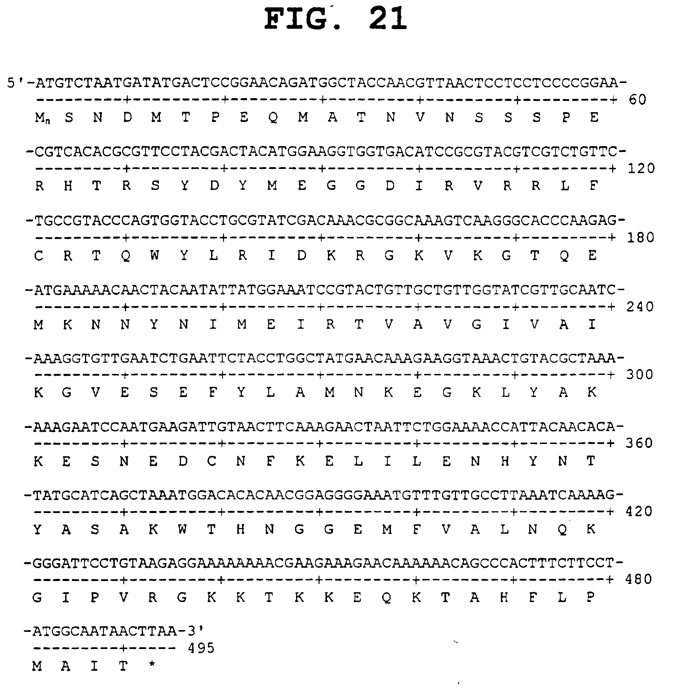

Figure 21 shows the nucleotide (SEQ ID NO:41) and amino acid (SEQ ID NO:42) sequences of C(1,15,102)S, a KGF analog having substitutions of serine for cysteine at amino acid positions 1, 15 and 102 of KGF, with the initial amino acid being Mₙ wherein n equals 0 or 1.

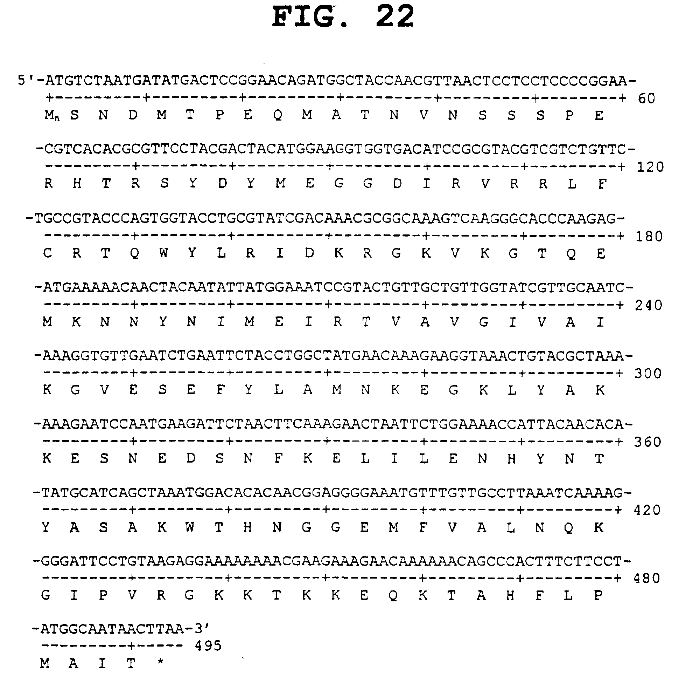

Figure 22 shows the nucleotide (SEQ ID NO:43) and amino acid (SEQ ID NO: 44) sequences of C(1,15,102,106)S, a KGF analog having substitutions of serine for cysteine at amino acid positions 1, 15, 102 and 106 of KGF, with the initial amino acid being Mₙ wherein n equals 0 or 1.

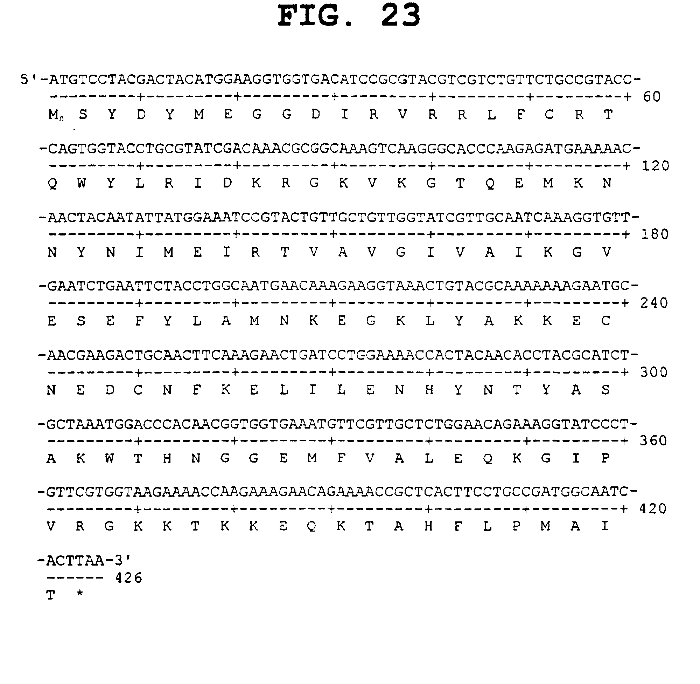

Figure 23 shows the nucleotide (SEQ ID NO:45) and amino acid (SEQ ID NO:46) sequences of ΔN23/N(137)E, a KGF analog having a deletion of the first 23 amino acids of the amino-terminus and a substitution of glutamic acid for asparagine at amino acid position 137 of KGF, with the initial amino acid being Mₙ wherein n equals 0 or 1.

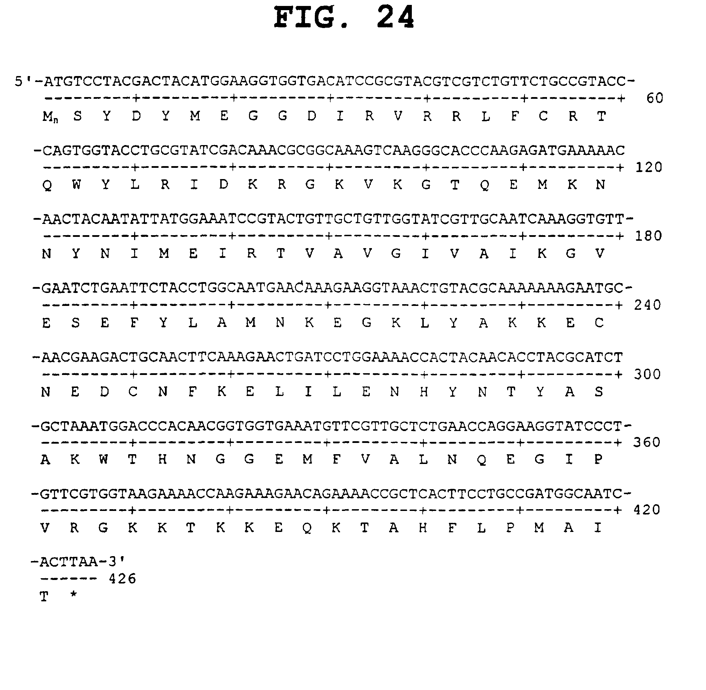

Figure 24 shows the nucleotide (SEQ ID NO:47) and amino acid (SEQ ID NO:48) sequences of ΔN23/K(139)E, a KGF analog having a deletion of the first 23 amino acids of the amino-terminus and a substitution of glutamic acid for lysine at amino acid position 139 of KGF, with the initial amino acid being Mₙ wherein n equals 0 or 1.

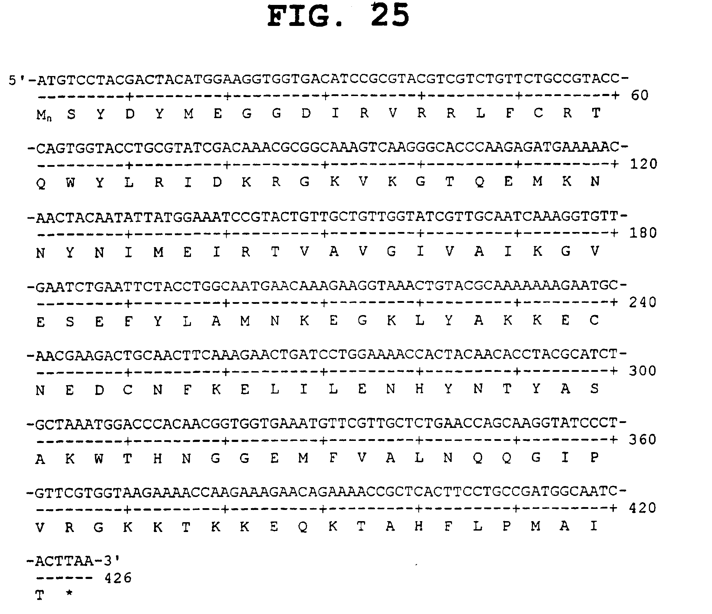

Figure 25 shows the nucleotide (SEQ ID NO:49) and amino acid (SEQ ID NO:50) sequences of ΔN23/K(139)Q, a KGF analog having a deletion of the first 23 amino acids of the amino-terminus and a substitution of glutamine for lysine at amino acid position 139 of KGF, with the initial amino acid being Mₙ wherein n equals 0 or 1.

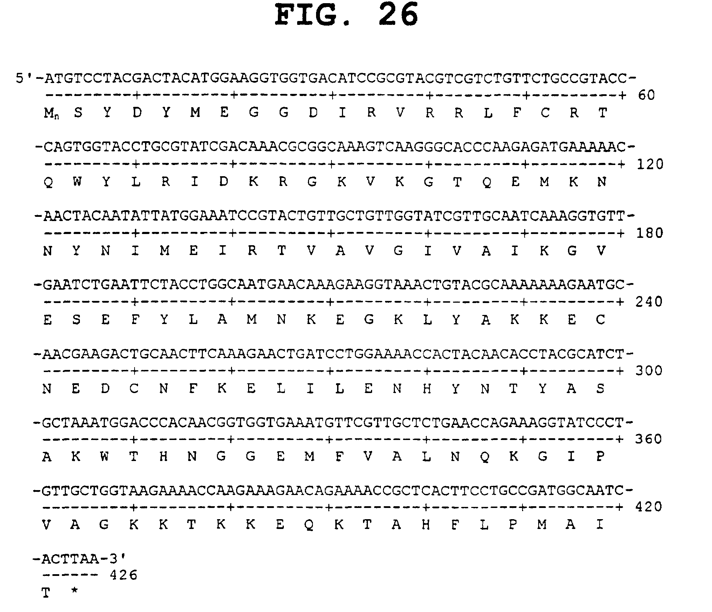

Figure 26 shows the nucleotide (SEQ ID NO:51) and amino acid (SEQ ID NO:52) sequences of ΔN23/R(144)A, a KGF analog having a deletion of the first 23 amino acids of the amino-terminus and a substitution of alanine for arginine at amino acid position 144 of KGF, with the initial amino acid being Mₙ wherein n equals 0 or 1.

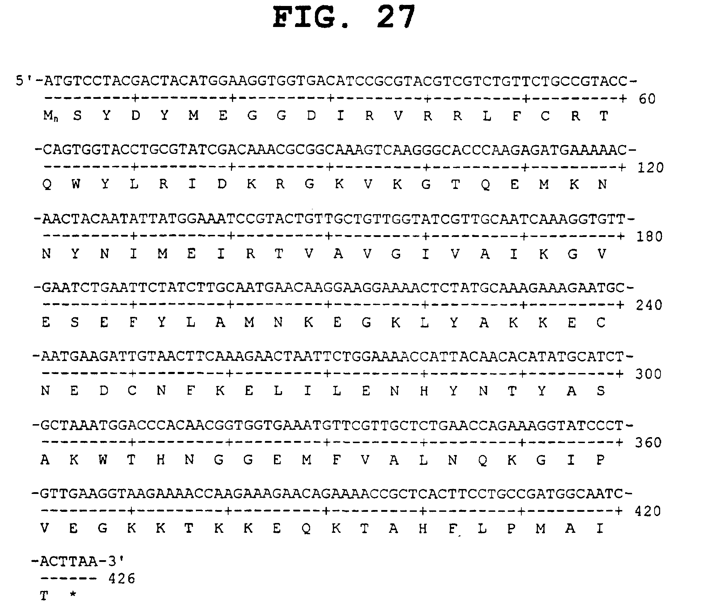

Figure 27 shows the nucleotide (SEQ ID NO:53) and amino acid (SEQ ID NO:54) sequences of ΔN23/R(144)E, a KGF analog having a deletion of the first 23 amino acids of the amino-terminus and a substitution of glutamic acid for arginine at amino acid position 144 of KGF, with the initial amino acid being Mₙ wherein n equals 0 or 1.

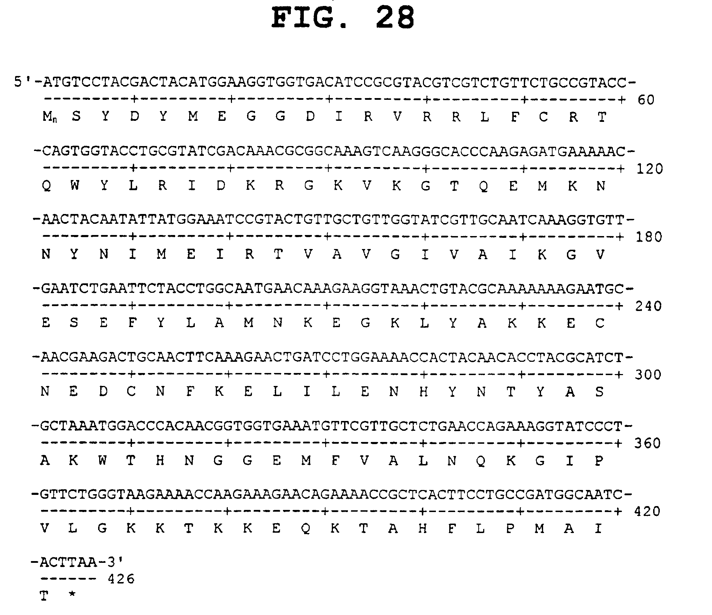

Figure 28 shows the nucleotide (SEQ ID NO:55) and amino acid (SEQ ID NO:56) sequences of ΔN23/R(144)L, a KGF analog having a deletion of the first 23 amino acids of the amino-terminus and a substitution of leucine for arginine at amino acid position 144 of KGF, with the initial amino acid being Mₙ wherein n equals 0 or 1.

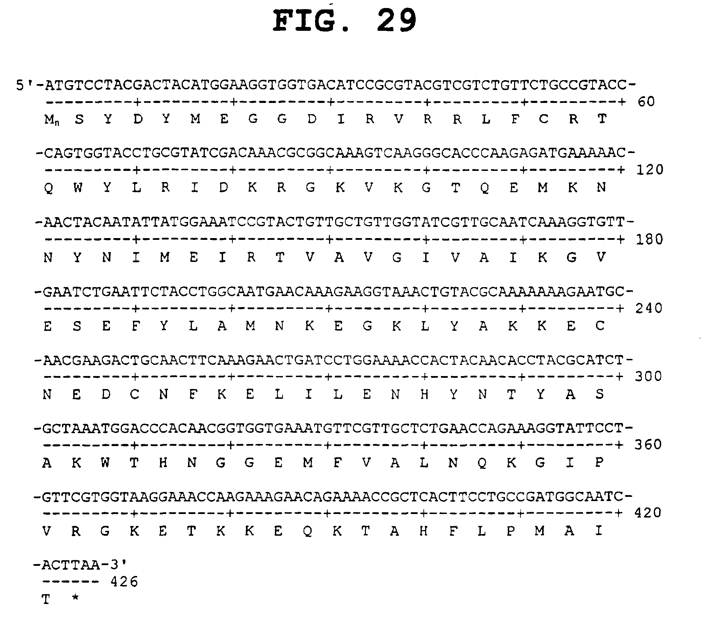

Figure 29 shows the nucleotide (SEQ ID NO:57) and amino acid (SEQ ID NO:58) sequences of ΔN23/K(147)E, a KGF analog having a deletion of the first 23 amino acids of the amino-terminus and a substitution of glutamic acid for lysine at amino acid position 147 of KGF, with the initial amino acid being Mₙ wherein n equals 0 or 1.

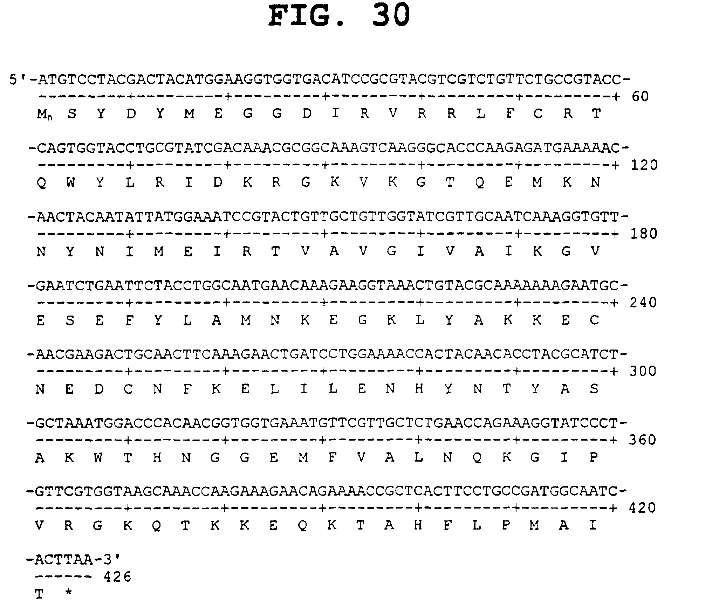

Figure 30 shows the nucleotide (SEQ ID NO:59) and amino acid (SEQ ID NO:60) sequences of ΔN23/K(147)Q, a KGF analog having a deletion of the first 23 amino acids of the amino-terminus and a substitution of glutamine for lysine at amino acid position 147 of KGF, with the initial amino acid being Mₙ wherein n equals 0 or 1.

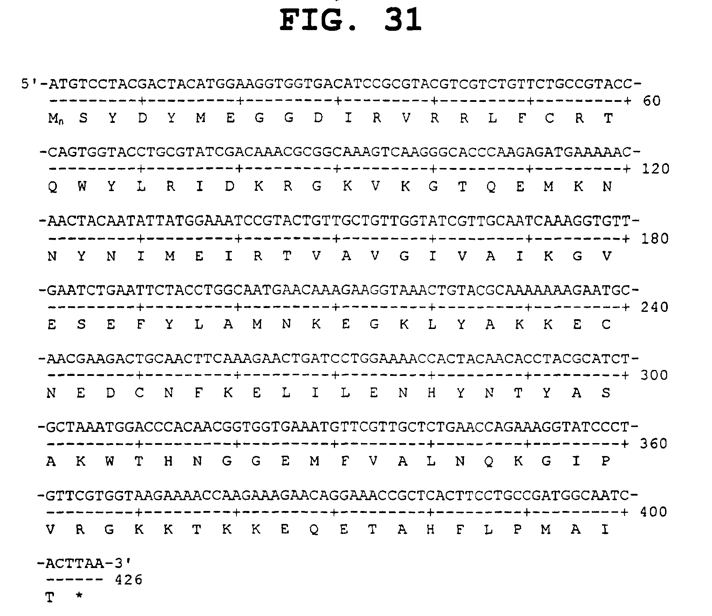

Figure 31 shows the nucleotide (SEQ ID NO:61) and amino acid (SEQ ID NO:62) sequences of ΔN23/K(153)E, a KGF analog having a deletion of the first 23 amino acids of the amino-terminus and a substitution of glutamic acid for lysine at amino acid position 153 of KGF, with the initial amino acid being Mₙ wherein n equals 0 or 1.

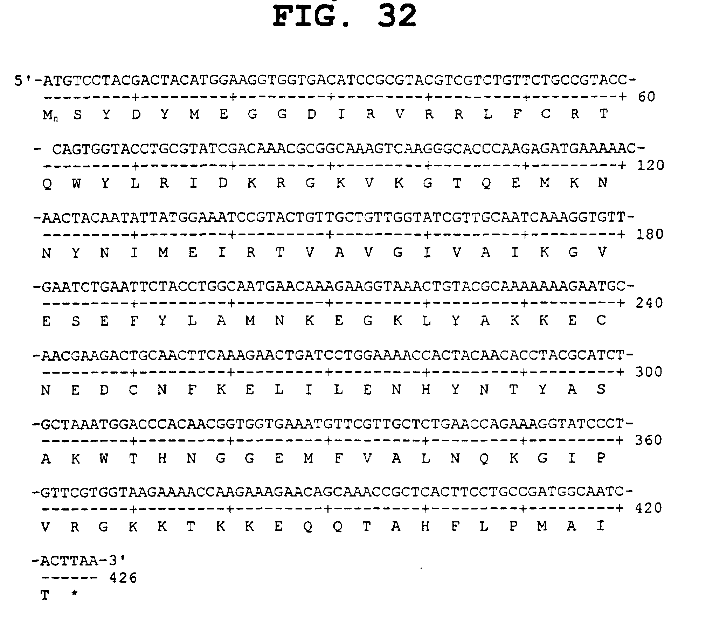

Figure 32 shows the nucleotide (SEQ ID NO:63) and amino acid (SEQ ID NO:64) sequences of ΔN23/K(153)Q, a KGF analog having a deletion of the first 23 amino acids of the amino-terminus and a substitution of glutamine for lysine at amino acid position 153 of KGF, with the initial amino acid being Mₙ wherein n equals 0 or 1.

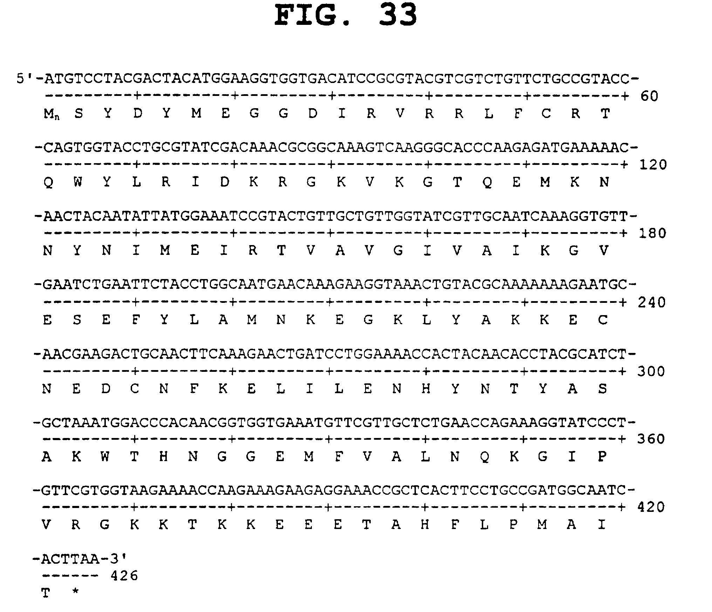

Figure 33 shows the nucleotide (SEQ ID NO:65) and amino acid (SEQ ID NO:66) sequences of ΔN23/Q(152)E/K(153)E, a KGF analog having a deletion of the first 23 amino acids of the amino-terminus and a substitution of glutamic acid for glutamine at amino acid position 152 of KGF and glutamic acid for lysine at amino acid position 153 of KGF, with the initial amino acid being Mₙ wherein n equals 0 or 1.

### DETAILED DESCRIPTION OF THE INVENTION

### KGF protein(s)

In accordance with present invention, the term "KGF protein(s)" is meant the protein defined by amino acids Cys³² to Thr¹⁹⁴ of SEQ ID NO:2 (mature KGF) and variant proteins thereof. [Unless otherwise indicated, amino acid numbering for molecules described herein shall correspond to that presented for the mature form of molecule (i.e., minus the signal sequence), as depicted by amino acids 32 to 194 of SEQ ID NO:2, with the initial MET in each such sequence being residue number "0".]

The term "KGF protein(s)" thus includes a protein in which one or more amino acid residues have been deleted from ("deletion variant(s)"), inserted into ("addition variant(s)"), and/or substituted for ("substitution variant(s)") residues within the amino acid sequence of SEQ ID NO:2 and which retains biological activity.

It will be appreciated by those skilled in the art that many combinations of deletions, insertions, and substitutions (individually or collectively "variant(s)") can be made, provided that the final protein is biologically active. The term "biological activity" as used herein means that a KGF protein(s) possesses some but not necessarily all the same properties of (and not necessarily to the same degree as) KGF. The selection of the particular properties of interest depends upon the desired use of the desired KGF protein(s).

There are two principal variables in the construction of amino acid sequence variant(s): the location of the mutation site and the nature of the mutation. In designing each variant(s), the location of each mutation site and the nature of each mutation will depend on the biochemical characteristic(s) to be modified. Each mutation site can be modified individually or in series, e.g., by (1) deleting the target amino acid residue, (2) inserting one or more amino acid residues adjacent to the located site or (3) substituting first with conservative amino acid choices and, depending upon the results achieved, then with more radical selections.

Amino acid sequence deletions generally range from about 1 to 30 amino acid residues, preferably from about 1 to 20 amino acids, more preferably from about 1 to 10 amino acid residues and most preferably from about 1 to 5 residues. Amino-terminal, carboxy-terminal and internal intrasequence deletions are contemplated. Deletions within the amino acid sequence of KGF may be made, for example, in regions of low homology with the sequences of other members of the FGF family. Deletions within the amino acid sequence of KGF in areas of substantial homology with the sequences of other members of the FGF family will be more likely to significantly modify the biological activity. The number of total deletions and/or consecutive deletions preferably will be selected so as to preserve the tertiary structure of KGF in the affected domain, e.g., cysteine crosslinking.

An amino acid sequence addition may include insertions of an amino- and/or carboxyl-terminal fusion ranging in length from one residue to one hundred or more residues, as well as internal intrasequence insertions of single or multiple amino acid residues. Internal additions may range generally from about 1 to 20 amino acid residues, preferably from about 1 to 10 amino acid residues, more preferably from about 1 to 5 amino acid residues, and most preferably from about 1 to 3 amino acid residues. Additions within the amino acid sequence of KGF may be made in regions of low homology with the sequences of other members of the FGF family. Additions within the amino acid sequence of KGF in areas of substantial homology with the sequences of other members of the FGF family will be more likely to significantly modify the biological activity. Insertions or additions preferably include amino acid sequences derived from the sequences of other FGF family members.

An amino-terminus addition is contemplated to include the addition of a methionine (for example, as an artifact of the direct expression in bacterial recombinant cell culture) or an amino acid residue or sequence of KGF. A further example of an amino-terminal addition includes the fusion of a signal sequence to the amino-terminus of KGF in order to facilitate the secretion of protein from recombinant host cells. Such signal sequences generally will be obtained from and thus be homologous to the intended host cell species. For prokaryotic host cells that do not recognize and process the native signal sequence, the signal sequence may be substituted by a prokaryotic signal sequence selected, for example, from the group of the alkaline phosphatase, penicillinase or heat-stable enterotoxin II leader sequences. For expreession in yeast cells, each polypeptide may have a signal sequence selected, for example, from the group of the yeast invertase, alpha factor or acid phosphatase leader sequences. In mammalian cell expression specifically, signal sequences of KGF are satisfactory, although other mammalian signal sequences may be suitable, for example sequences derived from other FGF family members may be suitable.

An example of an amino- or a carboxy-terminus addition includes chimeric proteins comprising the amino-terminal or carboxy-terminal fusion of a KGF protein with all or part of the constant domain of the heavy or light chain of human immunoglobulin (individually or collectively, ("KGF Fc(s)"). Such chimeric polypeptides are preferred wherein the immunoglobulin portion of each comprises all of the domains except the first domain of the constant region of the heavy chain of human immunoglobulin such as IgG (e.g., IgG1 or IgG3), IgA, IgM or IgE. A skilled artisan will appreciate that any amino acid of the immunoglobulin portion can be deleted or substituted with one or more amino acids, or one or more amino acids can be added as long as the KGF protein portion is capable of stimulating epithelial cells and the immunoglobulin portion shows one or more of its characteristic properties.

Another group of variant(s) is amino acid substitution variant(s) of the amino acid sequence of KGF. These are variant(s) wherein at least one amino acid residue in KGF is removed and a different residue inserted in its place. Substitution variant(s) include allelic variant(s), which are characterized by naturally-occurring nucleotide sequence changes in the species population that may or may not result in an amino acid change. One skilled in the art can use any information known about the binding or active site of the polypeptide in the selection of possible mutation sites.

One method for identifying amino acid residues or regions for mutagenesis of a protein is called "alanine scanning mutagenesis", as described by Cunningham and Wells (1989), *Science,* 244:1081-1085. In this method, an amino acid residue or group of target residues of a protein is identified (e.g., charged residues such as Arg, Asp, His, Lys and Glu) and replaced by a neutral or negatively-charged amino acid (most preferably alanine or polyalanine) to affect the interaction of the amino acids with the surrounding aqueous environment in or outside the cell. Those domains/residues demonstrating functional sensitivity to the substitutions are then refined by introducing additional or alternate residues at the sites of substitution. Thus, the site for introducing an amino acid sequence modification is predetermined. To optimize the performance of a mutation at a given site, alanine scanning or random mutagenesis may be conducted and the variant(s) may be screened for the optimal combination of desired activity and degree of activity.

The sites of greatest interest for substitutional mutagenesis include sites in which particular residues within KGF are substantially different from various species or other FGF family members in terms of side-chain bulk, charge, and/or hydrophobicity. Other sites of interest include those in which particular residues within KGF are identical among various species or other FGF family members, as such positions are generally important for the biological activity of a protein. A skilled artisan will appreciate that initially these sites should be modified by substitution in a relatively conservative manner.

Such conservative substitutions are shown in Table 1 under the heading of "Preferred Substitutions". If such substitutions result in a change in biological activity, then more substantial changes (Exemplary Substitutions) may be introduced and/or other additions/deletions may be made and the resulting polypeptides screened.

**TABLE 1:**

| Amino Acid Substitutions | | |
|---|---|---|
| Original Residue | Preferred Substitutions | Exemplary Substitutions |
| Ala (A) | Val | Val; Leu; Ile |
| Arg (R) | Lys | Lys; Gln; Asn |
| Asn (N) | Gln | Gln; His; Lys; |
| | | Arg |
| Asp (D) | Glu | Glu |
| Cys (C) | Ser | Ser |
| Gln (Q) | Asn | Asn |
| Glu (E) | Asp | Asp |
| Gly (G) | Pro | Pro |
| His (H) | Arg | Asn; Gln; Lys; |
| | | Arg |
| Ile (I) | Leu | Leu; Val; Met; |
| | | Ala; Phe; |
| | | norleucine |
| Leu (L) | Ile | norleucine; |
| | | Ile; Val; Met; |
| | | Ala; Phe |
| Lys (K) | Arg | Arg; Gln; Asn |
| Met (M) | Leu | Leu; Phe; Ile |
| Phe (F) | Leu | Leu; Val; Ile; |
| | | Ala |
| Pro (P) | Gly | Gly |
| Ser (S) | Thr | Thr |
| Thr (T) | Ser | Ser |
| Trp (W) | Tyr | Tyr |
| Tyr (Y) | Phe | Trp; Phe; Thr; |
| | | Ser |
| Val (V) | Leu | Ile; Leu; Met; |
| | | Phe; Ala; |
| | | norleucine |

In making such changes, the hydropathic index of amino acids may be considered. The importance of the hydropathic amino acid index in conferring interactive biological function on a protein is generally understood in the art (Kyte and Doolittle (1982), *J*. *Mol*. *Biol*., 157:105-131). It is known that certain amino acids may be substituted for other amino acids having a similar hydropathic index or score and still retain a similar biological activity.

It is also understood in the art that the substitution of like amino acids can be made effectively on the basis of hydrophilicity, particularly where the functionally equivalent protein or peptide thereby created is intended for use in immunological embodiments, as in the present case. U.S. Patent 4,554,101, the disclosure of which is incorporated herein by reference, states that the greatest local average hydrophilicity of a protein, as governed by the hydrophilicity of its adjacent amino acids, correlates with its immunogenicity and antigenicity, *i*.*e*., with a biological property of the protein.

U.S. Patent 4,554,101 also teaches the identification and preparation of epitopes from primary amino acid sequences on the basis of hydrophilicity. Through the methods disclosed in U.S. Patent 4,554,101 a skilled artisan would be able to identify epitopes, for example, within the amino acid sequence of KGF. These regions are also referred to as "epitopic core regions". Numerous scientific publications have been devoted to the prediction of secondary structure, and to the identification of epitopes, from analyses of amino acid sequences (Chou and Fasman (1974), *Biochemistry,* 13(2):222-245; Chou and Fasman (1974), *Biochemistry*, 13(2):211-222;. Chou and Fasman (1978), *Adv*. *Enzymol*. *Relat*. *Areas Mol*. *Biol*., 47:45-148; Chou and Fasman (1978), *Ann. Rev*. *Biochem*., 47:251-276 and Chou and Fasman (1979), *Biophys*. *J*., 26:367-384). Moreover, computer programs are currently available to assist with predicting antigenic portions and epitopic core regions of proteins. Examples include those programs based upon the Jameson-Wolf analysis (Jameson and Wolf (1988), *Comput*. *Appl. Biosci*., 4(1):181-186 and Wolf et al. (1988), *Comput. Appl*. *Biosci*., 4(1):187-191); the program PepPlot® (Brutlag et al. (1990), *CABS,* 6:237-245 and Weinberger et al. (1985), *Science,* 228:740-742, the disclosures of which are incorporated herein by reference); and other programs for protein tertiary structure prediction (Fetrow and Bryant (1993), *BIOTECHNOLOGY*, 11:479-483.

In contrast, substantial modifications in the functional and/or chemical characteristics of KGF may be accomplished by selecting substitutions that differ significantly in their effect on maintaining (a) the structure of the polypeptide backbone in the area of the substitution, for example, as a sheet or helical conformation, (b) the relative charge or hydrophobicity of the protein at the target site or (c) the bulk of the side chain. Naturally-occurring residues are divided into groups based on common side chain properties:
1) hydrophobic: norleucine, Met, Ala, Val, Leu, Ile;
2) neutral hydrophilic: Cys, Ser, Thr;
3) acidic: Asp, Glu;
4) basic: Asn, Gln, His, Lys, Arg;
5) aromatic: Trp, Tyr, Phe; and
6) residues that influence chain orientation: Gly, Pro.

Non-conservative substitutions may involve the exchange of a member of one of these groups for another. Such substituted residues may be introduced into regions of KGF that, for example, are homologous with other FGF family members or into non-homologous regions of the protein.

A variety of amino acid substitutions or deletions may be made to modify or add N-linked or O-linked glycosylation sites, resulting in a protein with altered glycosylation. The sequence may be modified to add glycosylation sites to or to delete N-linked or O-linked glycosylation sites from the KGF proteins. An asparagine-linked glycosylation recognition site comprises a tripeptide sequence which is specifically recognized by appropriate cellular glycosylation enzymes. These tripeptide sequences are either Asn-Xaa-Thr or Asn-Xaa-Ser, where Xaa can be any amino acid other than Pro. Specific mutations of the sequences of KGF may involve substitution of a non-native Asn at position 50 and Thr at position 52 to insert an N-linked glcosylation site. Such modifications may be of particular utility in the addition of a carbohydrate, which may reduce aggregation (to increase thermal and storage stability), increase half-life in circulation, decrease isoelectric point, increase solubility and decrease antigenecity of the protein.

In a specific embodiment, a variant polypeptide will preferably be substantially homologous to the amino acid sequence of KGF (SEQ ID NO:2). The term "substantially homologous" as used herein means a degree of homology that is in excess of 80%, preferably in excess of 90%, more preferably in excess of 95% or most preferably even 99%. The percentage of homology as described herein is calculated as the percentage of amino acid residues found in the smaller of the two sequences which align with identical amino acid residues in the sequence being compared when four gaps in a length of 100 amino acids may be introduced to assist in that alignment, as set forth by Dayhoff (1972), *Atlas of Protein Sequence and Structure*, 5:124, National Biochemical Research Foundation, Washington, D.C. Also included within the term "substantially homologous" are variant(s) of KGF which may be isolated by virtue of cross-reactivity with antibodies to amino acids sequence of SEQ ID NO:2, or whose genes may be isolated through hybridization with the DNA of SEQ ID NO:1 or with segments thereof.

A KGF protein(s) may be rapidly screened to assess its physical properties.. For example, the level of biological activity (e.g., receptor binding and/or affinity, mitogenic, cell proliferative and/or *in vivo* activity) may be tested using a variety of assays. One such assay includes a mitogenic assay to test the ability of a protein to stimulate DNA synthesis (Rubin et al. (1989), *supra*). Another such assay includes a cell proliferative assay to test the ability of a protein to stimulate cell proliferation (Falco et al. (1988), *Oncogene*, 2:573-578).

Specific variant(s) of KGF are disclosed in WO 9008771, WO 9622369, WO 9611949 and WO 9611951. One example of KGF includes proteins having residues corresponding to Cys¹ and Cys¹⁵ of SEQ ID NO:2 replaced or deleted, with the resultant molecule having improved stability as compared with the parent molecule (WO 96/11949). Another example of KGF includes charge-change polypeptides wherein one or more of amino acid residues 41-154 of native KGF (preferably residues Arg⁴¹, Gln⁴³, Lys⁵⁵, Lys⁹⁵, Lys¹²⁸, Asn¹³⁷, Gln¹³⁸, Lys¹³⁹, Arg¹⁴⁴, Lys¹⁴⁷, Gln¹⁵², Lys¹⁵³ or Thr¹⁵⁴) are deleted or substituted with a neutral residue or negatively charged residue selected to effect a protein with a reduced positive charge (WO 96/11951). A still further example of KGF includes proteins generated by substituting at least one amino acid having a higher loop-forming potential for at least one amino acid within a loop-forming region of Asn¹¹⁵-His¹¹⁶-Tyr¹¹⁷-Asn¹¹⁸-Thr¹¹⁹ of native KGF (WO 96/11950). A still yet further example includes proteins having one or more amino acid substitutions, deletions or additions within a region of 123-133 (amino acids 154-164 of SEQ ID NO:2) of native KGF; these proteins may have agonistic or antagonistic activity.

Specifically disclosed proteins include the following: C(1,15) S (Figure 2); ΔN3/C(15)S (Figure 3); ΔN3/C(15) - (Figure 4); ΔN8/C(15)S (Figure 5); ΔN8/C(15) - (Figure 6); ΔN15 (Figure 7); ΔN16 (Figure 8); ΔN17 (Figure 9); ΔN18 (Figure 10); ΔN19 (Figure 11); ΔN20 (Figure 12); ΔN21 (Figure 13); ΔN22 (Figure 14); ΔN23 (Figure 15); ΔN24 (Figure 16); C(1,15)S/R(144)E (Figure 17); C(1,15)S/R(144)Q (Figure 18); ΔN23/R(144)Q (Figure 19); C(1,15,40)S (Figure 20); C(1,15,102)S (Figure 21); C(1,15,102,106)S (Figure 22); ΔN23/N(137)E (Figure 23); ΔN23/K(139)E (Figure 24); ΔN23/K(139)Q (Figure 25); ΔN23/R(144)A (Figure 26); ΔN23/R(144)E (Figure 27); ΔN23/R(144)L (Figure 28); ΔN23/K(147)E (Figure 29); ΔN23/K(147)Q (Figure 30); ΔN23/K(153)E (Figure 31); ΔN23/K(153)Q (Figure 32) and ΔN23/Q(152)E/K(153)E (Figure 33).

### Polypeptide Derivatives

The use of chemically-modified derivatives of KGF protein(s) in which the protein is linked to a polymer in order to modify properties of the protein (referred herein as "derivatives") are included within the scope of the present invention. Such derivatives may be prepared by one skilled in the art given the disclosures herein. Conjugates may be prepared using glycosylated, non-glycosylated or de-glycosylated KGF proteins and suitable chemical moieties. Typically non-glycosylated proteins and water soluble polymers will be used.

Water soluble polymers are desirable because the protein to which each is attached will not precipitate in an aqueous environment, such as a physiological environment. Preferably, the polymer will be pharmaceutically acceptable for the preparation of a therapeutic product or composition. One skilled in the art will be able to select the desired polymer based on such considerations as whether the polymer/protein conjugate will be used therapeutically and, if so, the therapeutic profile of the protein (e.g., duration of sustained release; resistance to proteolysis; effects, if any, on dosage; biological activity; ease of handling; degree or lack of antigenicity and other known effects of a water soluble polymer on a therapeutic protein).

Suitable, clinically acceptable, water soluble polymers include, but are not limited to, polyethylene glycol (PEG), polyethylene glycol propionaldehyde, copolymers of ethylene glycol/propylene glycol, monomethoxy-polyethylene glycol, carboxymethylcellulose, dextran, polyvinyl alcohol (PVA), polyvinyl pyrrolidone, poly-1, 3-dioxolane, poly-1,3,6-trioxane, ethylene/maleic anhydride copolymer, poly (β-amino acids) (either homopolymers or random copolymers), poly(n-vinyl pyrrolidone)polyethylene glycol, polypropylene glycol homopolymers (PPG) and other polyalkylene oxides, polypropylene oxide/ethylene oxide copolymers, polyoxyethylated polyols (POG) (e.g., glycerol) and other polyoxyethylated polyols, polyoxyethylated sorbitol, or polyoxyethylated glucose, colonic acids or other carbohydrate polymers, Ficoll or dextran and mixtures thereof. As used herein, polyethylene glycol is meant to encompass any of the forms that have been used to derivatize other proteins, such as mono-(C1-C10) alkoxy- or aryloxy-polyethylene glycol. Polyethylene glycol propionaldehyde may have advantages in manufacturing due to its stability in water.

The water soluble polymers each may be of any molecular weight and may be branched or unbranched. Generally, the higher the molecular weight or the more branches, the higher the polymer:protein ratio. The water soluble polymers each typically have an average molecular weight of between about 2kDa to about 100kDa (the term "about" indicating that in preparations of a water soluble polymer, some molecules will weigh more, some less, than the stated molecular weight). The average molecular weight of each water soluble polymer preferably is between about 5kDa and about 40kDa, more preferably between about 10kDa and about 35kDa and most preferably between about 15kDa and about 30kDa.

There are a number of attachment methods available to those skilled in the art, including acylation reactions or alkylation reactions (preferably to generate an amino-terminal chemically modified protein) with a reactive water soluble molecule. See, for example, EP 0 401 384; see also, Malik et al. (1992), *Exp. Hematol.,* 20:1028-1035; Francis (1992), *Focus on Growth Factors,* 3(2):4-10, published by Mediscript, Mountain Court, Friern Barnet Lane, London N20 OLD, UK; EP 0 154 316; EP 0 401 384; WO 92/16221; WO 95/34326; WO 95/13312; WO 96/11953; US96/19459; and the other publications cited herein that relate to pegylation.

A specific embodiment used according to the present invention is an unbranched monomethoxy-polyethylene glycol aldehyde molecule having an average molecular weight of either about 20kDa or aboput 33kDa (e.g., between 30kDa and 35kDa), or a tertiary butyl polyethylene glycol additive having an average molecular weight of about 33kDa (e.g., between 30kDa and 35kDa) conjugated via reductive alkylation to the KGF protein(s).

### Polyvalent Forms

Polyvalent forms, i.e., molecules comprising more than one active moiety, may be used. In one embodiment, the molecule may possess multiple KGF protein(s). Additionally, the molecule may possess at least one KGF protein(s) and, depending upon the desired characteristic of polyvalent form, at least one other molecule.

In one embodiment, the polyvalent form may be used, for example, by chemically coupling at least one KGF protein(s) and another moeity with any clincally accepted linker (e.g., a water soluble polymer). In principle the linker must not impart new immunogenecity nor, by virtue of the new amino acid residues, alter the hydrophobicity and charge balance of the structure which affects its biodistribution and clearance.

The water soluble polymers can be, based on the monomers listed above, homopolymers, random or block copolymers, terpolymers straight chain or branched, substituted or unsubstituted. The polymer can be of any length or molecular weight, but these characteristics can affect the biological properties. Polymer average molecular weights particularly useful for decreasing clearance rates in pharmaceutical applications are in the range of 2,000 to 35,000 daltons. In addition, the length of the polymer can be varied to optimize or confer the desired biological activity.

The active moieties may be linked using conventional coupling techniques (see WO 92/16221, WO 95/13312 and WO 95/34326).

Alternatively, a bivalent molecule may consist of two tandem repeats of KGF protein(s) separated by a polypeptide linker region. The design of the polypeptide linkers is similar in design to the insertion of short loop sequences between domains in the *de novo* design of proteins (Mutter (1988), *TIBS,* 13:260-265 and Regan and DeGrado (1988), *Science,* 241:976-978). Several different linker constructs have been assembled and shown to be useful for forming single chain antibodies; the most functional linkers vary in size from 12 to 25 amino acids (amino acids having unreactive side groups, e.g., alanine, serine and glycine) which together constitute a hydrophilic sequence, have a few oppositely charged residues to enhance solubility and are flexible (Whitlow and Filpula (1991), *Methods: A Companion to Methods in Enzymology,* 2:97-105; and Brigido et al. (1993), *J. Immunol*., 150:469-479).

Additionally, KGF protein(s) may be chemically coupled to a biotin, and resulting conjugate is then allowed to bind to avidin, resulting in tetravalent avidin/biotin/KGF protein(s). KGF protein(s) may also be covalently coupled to dinitrophenol (DNP) or trinitrophenol (TNP) and the resulting conjugates precipitated with anti-DNP or anti-TNP-IgM to form decameric conjugates.

In yet another embodiment, recombinant fusion proteins may also be used wherein each recombinant chimeric molecule has a KGF protein sequence amino-terminally or carboxy-terminally fused to all or part of the constant domains, but at least one constant domain, of the heavy or light chain of human immunoglobulin. For example, a chimeric KGF protein/IgG1 (or IgG1/KGF protein) fusion protein may be used which is produced from a light chain-containing chimeric gene: a KGF protein/human kappa light chain chimera (KGF protein/Ck) or a human kappa light chain/KGF protein chimera (Ck/KGF protein); or a heavy chain-containing chimeric gene: a KGF protein/human gamma-1 heavy chain chimera (KGF protein/Cg-1) or a human gamma-1 heavy chain/KGF protein chimera (Cg-1/KGF protein). Following transcription and translation of a heavy-chain chimeric gene, or of a light chain-containing gene and a heavy-chain chimeric gene, the gene products may be assembled into a single chimeric molecule having a KGF protein(s) displayed bivalently. Additional details relating to the construction of such chimeric molecules are disclosed in United States Patent 5,116,964, WO 89/09622, WO 91/16437 and EP 315062.

In yet a further embodiment, recombinant fusion proteins may also be used wherein each recombinant chimeric molecule has at least one KGF protein(s), as described above, and at least a portion of the region 186-401 of osteoprotegerin (OPG), as described in European Patent Application No. 96309363.8. Either the KGF protein(s) or the portion of osteoprotogerin may be at the amino-terminus or the carboxy-terminus of the chimeric molecule.

The production of KGF protein(s) is described in further detail below. Such proteins may be prepared, for example, by recombinant techniques or by *in vitro* chemical synthesis of the desired KGF protein(s).

### Polynucleotides

Based upon the present description and using the universal codon table, one of ordinary skill in the art can readily determine all of the nucleic acid sequences which encode the amino acid sequence of KGF protein(s).

Recombinant expression techniques conducted in accordance with the descriptions set forth below may be followed to produce these polynucleotides and to express the encoded proteins. For example, by inserting a nucleic acid sequence which encodes a KGF protein(s) into an appropriate vector, one skilled in the art can readily produce large quantities of the desired nucleotide sequence. The sequences can then be used to generate detection probes or amplification primers. Alternatively, a polynucleotide encoding a KGF protein(s) can be inserted into an expression vector. By introducing the expression vector into an appropriate host, the desired protein(s) may be produced in large amounts.

As further described herein, there are numerous host/vector systems available for the propagation of desired nucleic acid sequences and/or the production of the desired protein. These include, but are not limited to, plasmid, viral and insertional vectors, and prokaryotic and eukaryotic hosts. One skilled in the art can adapt a host/vector system which is capable of propagating or expressing heterologous DNA to produce or express the sequences of the present invention.

Furthermore, it will be appreciated by those skilled in the art that, in view of the present disclosure, the nucleic acid sequences for preparing the proteins used according to the present invention include the nucleic acid sequence of Figure 1, as well as degenerate nucleic acid sequences thereof, nucleic acid sequences which encode variant(s) of KGF and those nucleic acid sequences which hybridize (under hybridization conditions disclosed in the cDNA library screening section below, or equivalent conditions or more stringent conditions) to complements of the nucleic acid sequence of Figure 1.

Also recombinant DNA constructs involving vector DNA together with the DNA sequences encoding the desired proteins are used for preparing the proteins used according to the present invention. In each such DNA construct, the nucleic acid sequence encoding a desired protein (with or without signal peptides) is in operative association with a suitable expression control or regulatory sequence capable of directing the replication and/or expression of the desired protein in a selected host.

### Preparation of Polynucleotides

A nucleic acid sequence encoding a KGF protein(s) can readily be obtained in a variety of ways including, without limitation, chemical synthesis, cDNA or genomic library screening, expression library screening and/or PCR amplification of cDNA. These methods and others which are useful for isolating such nucleic acid sequences are set forth in Sambrook et al. (1989), *Molecular Cloning: A Laboratory Manual,* Cold Spring Harbor Laboratory Press, Cold Spring Harbor, NY; by Ausubel et al. (1994), *Current Protocols in Molecular Biology,* Current Protocols Press; and by Berger and Kimmel (1987), *Methods in Enzymology: Guide to Molecular Cloning Techniques*, Vol. 152, Academic Press, Inc., San Diego, CA.

Chemical synthesis of a nucleic acid sequence which encodes a desired proteins can be accomplished using methods well known in the art, such as those set forth by Engels et al. (1989), *Angew. Chem*. *Intl*. *Ed.,* 28:716-734 and Wells et al. (1985), *Gene,* 34:315. These methods include, *inter alia,* the phosphotriester, phosphoramidite and H-phosphonate methods of nucleic acid sequence synthesis. Large nucleic acid sequences, for example those larger than about 100 nucleotides in length, can be synthesized as several fragments. The fragments can then be ligated together to form a suitable nucleic acid sequence. A preferred method is polymer-supported synthesis using standard phosphoramidite chemistry.

Alternatively, a suitable nucleic acid sequence may be obtained by screening an appropriate cDNA library (i.e., a library prepared from one or more tissue or cell sources believed to express the protein) or a genomic library (a library prepared from total genomic DNA). The source of the cDNA library is typically a tissue from any species that is believed to express a desired protein in reasonable quantities. The source of the genomic library may be any tissue or tissues from any mammalian or other species believed to harbor a gene encoding a desired protein.

Hybridization media can be screened for the presence of a DNA encoding a desired protein using one or more nucleic acid probes (oligonucleotides, cDNA or genomic DNA fragments that possess an acceptable level of homology to the cDNA or gene to be cloned) that will hybridize selectively with cDNA(s) or gene(s) present in the library. The probes typically used for such screening encode a small region of DNA sequence from the same or a similar species as the species from which the library is prepared. Alternatively, the probes may be degenerate, as discussed herein.

Hybridization is typically accomplished by annealing an oligonucleotide probe or cDNA to the clones under conditions of stringency that prevent non-specific binding but permit binding of those clones that have a significant level of homology with the probe or primer. Typical hybridization and washing stringency conditions depend in part on the size (i.e., number of nucleotides in length) of the cDNA or oligonucleotide probe and whether the probe is degenerate. The probability of identifying a clone is also considered in designing the hybridization medium (e.g., whether a cDNA or genomic library is being screened).

Where a DNA fragment (such as a cDNA) is used as a probe, typical hybridization conditions include those as set forth in Ausubel et al. (1994), *supra.* After hybridization, the hybridization medium is washed at a suitable stringency, depending on several factors such as probe size, expected homology of probe to clone, the hybridization medium being screened, the number of clones being screened and the like.

Exemplary stringent hybridization conditions are hybridization in 6 x SSC at 62-67°C, followed by washing in 0.1 x SSC at 62-67°C for approximately one hour. Alternatively, exemplary stringent hybridization conditions are hybridization at 45-55% formamide, 6 x SSC at 40-45°C, followed by washing in 0.1 x SSC at 62-67°C for approximately one hour. Also included are DNA sequences which hybridize to the nucleic acid sequences set forth in Figure 1 under relaxed hybridization conditions and which encode KGF protein(s). Examples of such relaxed stringency hybridization conditions are 6 x SSC at 45-55°C or hybridization with 30-40% formamide at 40-45°C, followed by washing in 1-2 x SSC at 55°C for approximately 30 minutes. See Maniatis et al. (1982), *Molecular Cloning* (A Laboratory Manual), Cold Spring Harbor Laboratory, pages 387 to 389.

There are also exemplary protocols for stringent washing conditions where oligonucleotide probes are used to screen hybridization media. For example, a first protocol uses 6 X SSC with 0.05 percent sodium pyrophosphate at a temperature of between about 35°C and 63°C, depending on the length of the probe. For example, 14 base probes are washed at 35-40°C, 17 base probes at 45-50°C, 20 base probes at 52-57°C, and 23 base probes at 57-63°C. The temperature can be increased 2-3°C where the background non-specific binding appears high. A second protocol uses tetramethylammonium chloride (TMAC) for washing. One such stringent washing solution is 3 M TMAC, 50mM Tris-HCl, pH 8.0 and 0.2% SDS.

Another method for obtaining a suitable nucleic acid sequence encoding a KGF protein is the polymerase chain reaction (PCR). In this method, cDNA is prepared from poly(A)+RNA or total RNA using the enzyme reverse transcriptase. Two primers, typically complementary to two separate regions of cDNA (oligonucleotides) encoding the desired protein, are then added to the cDNA along with a polymerase such as *Tag* polymerase, and the polymerase amplifies the cDNA region between the two primers.

The oligonucleotide sequences selected as probes or primers should be of adequate length and sufficiently unambiguous so as to minimize the amount of non-specific binding that may occur during screening or PCR amplification. The actual sequence of the probes or primers is usually based on conserved or highly homologous sequences or regions. Optionally, the probes or primers can be fully or partially degenerate, i.e., can contain a mixture of probes/primers, all encoding the same amino acid sequence but using different codons to do so. An alternative to preparing degenerate probes is to place an inosine in some or all of those codon positions that vary by species. The oligonucleotide probes or primers may be prepared by chemical synthesis methods for DNA, as described herein.

### Vectors

DNA encoding the desired protein may be inserted into vectors for further cloning (amplification of the DNA) or for expression. Suitable vectors are commercially available or may be specifically constructed. The selection or construction of an appropriate vector will depend on (1) whether it is to be used for DNA amplification or for DNA expression, (2) the size of the DNA to be inserted into the vector and (3) the intended host cell to be transformed with the vector.

The vectors each typically involve a nucleic acid sequence which encodes a desired protein operatively linked to one or more of the following expression control or regulatory sequences capable of directing, controlling or otherwise effecting the expression of a desired protein by a selected host cell. Each vector contains various components, depending on its function (amplification of DNA or expression of DNA) and its compatibility with the intended host cell. The vector components generally include, but are not limited to, one or more of the following: a signal sequence, an origin of replication, one or more selection or marker genes, a promoter, an enhancer element, a transcription termination sequence and the like. These components may be obtained from natural sources or be synthesized by known procedures.

Examples of suitable prokaryotic cloning vectors include bacteriophages such as lambda derivatives, or plasmids from *E. coli* (e.g. pBR322, col E1, pUC, the F-factor and Bluescript® plasmid derivatives (Stratagene, LaJolla, CA)). Other appropriate expression vectors, of which numerous types are known in the art for the host cells described herein, can also be used for this purpose.

### Signal Sequence

The nucleic acid encoding a signal sequence may be inserted 5' of the sequence encoding a desired protein, e.g, it may be a component of a vector or it may be a part of a nucleic acid encoding a desired protein. The nucleic acid encoding the native signal sequence of KGF is known (WO 90/08771).

### Origin of Replication

Expression and cloning vectors each generally include a nucleic acid sequence that enables the vector to replicate in one or more selected host cells. In a cloning vector, this sequence is typically one that enables the vector to replicate independently of the host chromosomal DNA and includes an origin of replication or autonomously replicating sequence. Such sequences are well known. The origin of replication from the plasmid pBR322 is suitable for most Gram-negative bacteria, and various origins (e.g., SV40, polyoma, adenovirus, VSV or BPV) are useful for cloning vectors in mammalian cells. Generally, the origin of replication is not needed for mammalian expression vectors (for example, the SV40 origin is often used only because it contains the early promoter).

### Selection Gene

The expression and cloning vectors each typically contain a selection gene. This gene encodes a "marker" protein necessary for the survival or growth of the transformed host cells when grown in a selective culture media. Host cells that are not transformed with the vector will not contain the selection gene and, therefore, will not survive in the culture media. Typical selection genes encode proteins that (a) confer resistance to antibiotics or other toxins, e.g., ampicillin, neomycin, methotrexate or tetracycline; (b) complement auxotrophic deficiencies or (c) supply critical nutrients not available from the culture media.

Other selection genes may be used to amplify the genes to be expressed. Amplification is the process wherein genes which are in greater demand for the production of a protein critical for growth are reiterated in tandem within the chromosomes of successive generations of recombinant cells. Examples of suitable selectable markers for mammalian cells include dihydrofolate reductase (DHFR) and thymidine kinase. The cell transformants are placed under selection pressure which only the transformants are uniquely adapted to survive by virtue of the marker being present in the vectors. Selection pressure is imposed by culturing the transformed cells under conditions in which the concentration of selection agent in the media is successively changed, thereby leading to amplification of both the selection gene and the DNA that encodes the desired protein. As a result, increased quantities of the desired protein are synthesized from the amplified DNA.

For example, cells transformed with the DHFR selection gene are first identified by culturing all of the transformants in a culture media that contains methotrexate, a competitive antagonist of DHFR. An appropriate host cell when wild-type DHFR is used is the Chinese hamster ovary cell line deficient in DHFR activity (Urlaub and Chasin (1980), *Proc*. *Natl*. *Acad. Sci*., *USA*, 77(7):4216-4220. The transformed cells are then exposed to increased levels of methotrexate. This leads to the synthesis of multiple copies of the DHFR gene and, concomitantly, multiple copies of other DNA present in the expression vector, such as the DNA encoding a desired protein.

### Promoter

Expression and cloning vectors each will typically contain a promoter that is recognized by the host organism and is operably linked to a nucleic acid sequence encoding the desired protein. A promoter is an untranslated sequence located upstream (5') to the start codon of a structural gene (generally within about 100 to 1000 bp) that controls the transcription and translation of a particular nucleic acid sequence. A promoter may be conventionally grouped into one of two classes, inducible promoters and constitutive promoters. An inducible promoter initiates increased levels of transcription from DNA under its control in response to some change in culture conditions, such as the presence or absence of a nutrient or a change in temperature. A large number of promoters, recognized by a variety of potential host cells, are well known. A promoter may be operably linked to DNA encoding a desired protein by removing the promoter from the source DNA by restriction enzyme digestion and inserting the desired promoter sequence. The native KGF promoter sequence may be used to direct amplification and/or expression of the DNA encoding a desired protein. A heterologous promoter is preferred, however, if it permits greater transcription and higher yields of the expressed protein as compared to the native promoter and if it is compatible with the host cell system that has been selected for use. For example, any one of the native promoter sequences of other FGF family members may be used to direct amplification and/or expression of the DNA encoding a desired protein.

Promoters suitable for use with prokaryotic hosts include the beta-lactamase and lactose promoter systems; alkaline phosphatase, a tryptophan (trp) promoter system; a bacterial luminescence (luxR) gene system and hybrid promoters such as the tac promoter. Other known bacterial promoters are also suitable. Their nucleotide sequences have been published, thereby enabling one skilled in the art to ligate them to the desired DNA sequence(s) using linkers or adaptors as needed to supply any required restriction sites.

Suitable promoter sequences for use with yeast hosts are also well known in the art. Suitable promoters for use with mammalian host cells are well known and include those obtained from the genomes of viruses such as polyoma virus, fowlpox virus, adenovirus (such as Adenovirus 2), bovine papilloma virus, avian sarcoma virus, cytomegalovirus, a retrovirus, hepatitis-B virus and, most preferably, Simian Virus 40 (SV40). Other suitable mammalian promoters include heterologous mammalian promoters, e.g., heat-shock promoters and the actin promoter.

### Enhancer Element

The expression and cloning vectors each will typically contain an enhancer sequence to increase the transcription by higher eukaryotes of a DNA sequence encoding a desired protein. Enhancers are cis-acting elements of DNA, usually from about 10-300 bp in length, that act on the promoter to increase its transcription.

Enhancers are relatively orientation and position independent. They have been found 5' and 3' to the transcription unit. Yeast enhancers are advantageously used with yeast promoters. Several enhancer sequences available from mammalian genes are known (e.g., globin, elastase, albumin, alpha-feto-protein and insulin). Additionally, viral enhancers such as the SV40 enhancer, the cytomegalovirus early promoter enhancer, the polyoma enhancer and adenovirus enhancers are exemplary enhancing elements for the activation of eukaryotic promoters. While an enhancer may be spliced into a vector at a position 5' or 3' to a DNA encoding a desired protein, it is typically located at a site 5' from the promoter.

### Transcription Termination

Expression vectors used in eukaryotic host cells each will typically contain a sequence necessary for the termination of transcription and for stabilizing the mRNA. Such sequences are commonly available from the 5' and occasionally 3' untranslated regions of eukaryotic DNAs or cDNAs. These regions contain nucleotide segments transcribed as polyadenylated fragments in the untranslated portion of the mRNA encoding a desired protein.

### Vector Construction

The construction of a suitable vector containing one or more of the above-listed components (together with the desired coding sequence) may be accomplished by standard ligation techniques. Isolated plasmids or DNA fragments are cleaved, tailored and religated in the desired order to generate the required vector. To confirm that the correct sequence has been constructed, the ligation mixture may be used to transform *E. coli*, and successful transformants may be selected by known techniques as described herein. Quantities of the vector from the transformants are then prepared, analyzed by restriction endonuclease digestion and/or sequenced to confirm the presence of the desired construct.

A vector that provides for the transient expression of DNA encoding a desired protein in mammalian cells may also be used. In general, transient expression involves the use of an expression vector that is able to replicate efficiently in a host cell, such that the host cell accumulates many copies of the expression vector and, in turn, synthesizes high levels of the desired protein encoded by the expression vector. Each transient expression system, comprising a suitable expression vector and a host cell, allows for the convenient positive identification of proteins encoded by cloned DNAs as well as for the rapid screening of such proteins for desired biological or physiological properties.

### Host Cells

Any of a variety of recombinant host cells, each of which contains a nucleic acid sequence for use in expressing a desired protein, is also provided by the present invention. Exemplary prokaryotic and eukaryotic host cells include bacterial, mammalian, fungal, insect, yeast or plant cells.

Prokaryotic host cells include, but are not limited to, eubacteria such as Gram-negative or Gram-positive organisms (e.g., *E. coli* (HB101, DH5a, DH10, and MC1061); *Bacilli* spp. such as *B*. *subtilis;* *Pseudomonas* spp. such as *P. aeruginosa; Streptomyces* spp.; *Salmonella* spp. such as *S. typhimurium;* or *Serratia spp.* such *as S*. *marcescans.* In a specific embodiment, a desired protein may be expressed in *E. coli.*

In addition to prokaryotic host cells, a KGF protein(s) may be expressed in glycosylated form by any one of a number of suitable host cells derived from multicellular organisms. Such host cells are capable of complex processing and glycosylation activities. In principle, any higher eukaryotic cell culture might be used, whether such culture involves vertebrate or invertebrate cells, including plant and insect cells.

Eukaryotic microbes such as filamentous fungi or yeast may be suitable hosts for the expression of a desired protein. *Saccharomyces cerevisiae*, or common baker's yeast, is the most commonly used among lower eukaryotic host microorganisms, but a number of other genera, species and strains are well known and commonly available.

Vertebrate cells may be used, as the propagation of vertebrate cells in culture (tissue culture) is a well-known procedure. Examples of useful mammalian host cell lines include, but are not limited to, monkey kidney CV1 line transformed by SV40 (COS-7), human embryonic kidney line (293 cells or 293 cells subcloned for growth in suspension culture), baby hamster kidney cells and Chinese hamster ovary cells. Other suitable mammalian cell lines include, but are not limited to, HeLa, mouse L-929 cells, 3T3 lines derived from Swiss, Balb-c or NIH mice, and BHK or HaK hamster cell lines. In a specific embodiment, a desired protein may be expressed in COS cells or in baculovirus cells.

A host cell may be transfected and preferably transformed with a desired nucleic acid under appropriate conditions permitting expression of the nucleic acid. The selection of suitable host cells and methods for transformation, culture, amplification, screening and product production and purification are well known in the art (Gething and Sambrook (1981), *Nature,* 293:620-625 or, alternatively, Kaufman et al. (1985), *Mol. Cell*. *Biol*., 5(7):1750-1759, or U.S. Pat. No. 4,419,446). For example, for mammalian cells without cell walls, the calcium phosphate precipitation method may be used. Electroporation, microinjection and other known techniques may also be used.

It is also possible that a desired protein may be produced by homologous recombination or with recombinant production methods utilizing control elements introduced into cells already containing DNA encoding a KGF protein(s). Homologous recombination is a technique originally developed for targeting genes to induce or correct mutations in transcriptionally active genes (Kucherlapati (1989), *Prog. in Nucl*. *Acid Res. and Mol*. *Biol*., 36:301). The basic technique was developed as a method for introducing specific mutations into specific regions of the mammalian genome (Thomas et al. (1986), *Cell,* 44:419-428; Thomas and Capecchi (1987), *Cell*, 51:503-512 and Doetschman et al. (1988), *Proc*. *Natl*. *Acad*. *Sci*., 85:8583-8587, or to correct specific mutations within defective genes (Doetschman et al. (1987), *Nature,* 330:576-578). Exemplary techniques are described in U.S. Patent No. 5,272,071; WO 92/01069; WO 93/03183; WO 94/12650 and WO 94/31560, into.

### Culturing the Host Cells

The method for culturing each of the one or more recombinant host cells for production of a desired protein will vary depending upon many factors and considerations; the optimum production procedure for a given situation will be apparent to those skilled in the art through minimal experimentation. Such recombinant host cells are cultured in suitable media and the expressed protein is then optionally recovered, isolated and purified from the culture media (or from the cell, if expressed intracellularly) by appropriate means known to those skilled in the art.

Specifically, each of the recombinant cells used to produce a desired protein may be cultured in media suitable for inducing promoters, selecting suitable recombinant host cells or amplifying the gene encoding the desired protein. The media may be supplemented as necessary with hormones and/or other growth factors (such as insulin, transferrin or epidermal growth factor), salts (such as sodium chloride, calcium, magnesium and phosphate), buffers (such as HEPES), nucleosides (such as adenosine and thymidine), antibiotics (such as gentamicin), trace elements (defined as inorganic compounds usually present at final concentrations in the micromolar range), and glucose or another energy source. Other supplements may also be included, at appropriate concentrations, as will be appreciated by those skilled in the art. Suitable culture conditions, such as temperature, pH and the like, are also well known to those skilled in the art for use with the selected host cells.

The resulting expression product may then be purified to near homogeneity using procedures known in the art. Exemplary purification techniques are taught in WO 90/08771 and WO 96/11952.

### Uses

KGF protein(s) and chemically-modified derivatives thereof having biological activity (collectively, "KGF protein product(s)") are used according to the present invention.

### Pharmaceutical Compositions

The use according to the present invention encompasses pharmaceutical preparations each containing therapeutically- or prophylatically-effective amounts of a KGF protein product(s).

Pharmaceutical compositions each will generally include a therapeutically-effective or prophylatically-effective amount of a KGF protein product(s) in admixture with a vehicle in combination with a GLP-2 protein product(s). The vehicle preferably includes one or more pharmaceutically and physiologically acceptable formulation materials in admixture with the KGF protein product(s) in combination with a GLP-2 protein product(s).

The primary solvent in a vehicle may be either aqueous or non-aqueous in nature. In addition, the vehicle may contain other pharmaceutically acceptable excipients for modifying or maintaining the pH, preferably to maintain the the pH between about 6.5 and 7.0 (e.g., buffers such as citrates, phosphates, and amino acids such as glycine); osmolarity (e.g., mannitol and sodium chloride); viscosity; clarity; color; sterility; stability (e.g., sucrose and sorbitol); odor of the formulation; rate of dissolution (e.g., solubilizers or solubilizing agents such as alcohols, polyethylene glycols and sodium chloride); rate of release; as well as bulking agents for lyophilized formulation (e.g., mannitol and glycine); surfactants (e.g., polysorbate 20, polysorbate 80, triton, and pluronics); antioxidants (e.g., sodium sulfite and sodium hydrogen-sulfite); preservatives (e.g., benzoic acid and salicylic acid); flavoring and diluting agents; emulsifying agents; suspending agents; solvents; fillers; delivery vehicles; diluents and/or pharmaceutical adjuvants. Other effective administration forms such as parenteral slow-release formulations, inhalant mists, orally-active formulations, or suppositories are also envisioned.

The composition may also involve particulate preparations of polymeric compounds such as bulk erosion polymers (e.g., poly(lactic-co-glycolic acid) (PLGA) copolymers, PLGA polymer blends, block copolymers of PEG, and lactic and glycolic acid, poly(cyanoacrylates)); surface erosion polymers (e.g., poly(anhydrides) and poly(ortho esters)); hydrogel esters (e.g., pluronic polyols, poly(vinyl alcohol), poly(vinylpyrrolidone), maleic anhydride-alkyl vinyl ether copolymers, cellulose, hyaluronic acid derivatives, alginate, collagen, gelatin, albumin, and starches and dextrans) and composition systems thereof; or preparations of liposomes or microspheres. Such compositions may influence the physical state, stability, rate of *in vivo* release, and rate of *in vivo* clearance of the present proteins and derivatives. The optimal pharmaceutical formulation for a desired protein will be determined by one skilled in the art depending upon the route of administration and desired dosage. Exemplary pharmaceutical compositions are disclosed in *Remington's Pharmaceutical Sciences,* 18th Ed. (1990), Mack Publishing Co., Easton, PA 18042, pages 1435-1712; Gombotz and Pettit (1995), *Bioconjugate Chem*., 6:332-351; Leone-Bay, et al. (1995), *Journal of Medicinal Chemistry*, 38:4263-4269; Haas, et al. (1995), *Clinical Immunology and Immunopathology*, 76(1):93; WO 94/06457; WO 94/21275; FR 2706772 and WO 94/21235.

Specific sustained release compositions are available from the a variety of suppliers including Depotech Corp. (Depofoam™, a multivesicular liposome) and Alkermes, Inc. (ProLease™, a PLGA microsphere). As used herein, hyaluronan is intended to include hyaluronan, hyaluronic acid, salts thereof (such as sodium hyaluronate), esters, ethers, enzymatic derivatives and cross-linked gels of hyaluronic acid, and chemically modified derivatives of hyaluronic acid (such as hylan). Exemplary forms of hyaluronan are disclosed in U.S. Patent Nos. 4,582,865, 4,605,691, 4,636,524, 4,713,448, 4,716,154, 4,716,224, 4,772,419, 4,851,521, 4,957,774, 4,863,907, 5,128,326, 5,202,431, 5,336,767, 5,356,883; European Patent Application Nos. 0 507 604 A2 and 0 718 312 A2; and WO 96/05845. Suppliers of hyaluronan include BioMatrix, Inc., Ridgefield, NJ; Fidia S.p.A., Abano Terme, Italy; Kaken Pharmaceutical Co., Ltd., Tokyo, Japan; Pharmacia AB, Stockholm, Sweden; Genzyme Corporation, Cambridge, MA; Pronova Biopolymer, Inc. Portsmouth, NH; Calbiochem-Novabiochem AB, Lautelfingen, Switzerland; Intergen Company, Purchase, NY and Kyowa Hakko Kogyo Co., Ltd., Tokyo, Japan.

For treatment and/or prevention of oral indications, a liquid solution or suspension can be used in a manner similar to a mouthwash, where the liquid is swished around in the mouth so as to maximize treatment of lesions (United States Patent 5,102,870, the teachings of which are incorporated by reference). Longer contact with the mucosal surface can be attained by selecting a suitable vehicle which is capable of coating mucosa. Typical examples are pectin containing formulations such as Orabase™ (Colgate-Hoyt Laboratories, Norwood, MA), sucralfate suspensions, Kaopectate and Milk of Magnesia. The formulation can also be a spreadable cream, gel, lotion or ointment having a pharmaceutically acceptable non-toxic vehicle or carrier. KGF protein product(s) in combination with a GLP-2 protein product(s) can also be incorporated into a slow dissolving lozenge or troche, a chewing gum base, or a buccal or slow delivery prosthesis hooked onto a back molar, for example.

Once the pharmaceutical composition has been formulated, it may be stored in sterile vials as a solution, suspension, gel, emulsion, solid, or a dehydrated or lyophilized powder. Such formulations may be stored either in a ready-to-use form or in a form (e.g., lyophilized) requiring reconstitution prior to administration.

In a specific embodiment, the use according to the present invention is directed to kits for producing a single-dose administration unit. The kits may each contain both a first container having a dried protein and a second container having an aqueous formulation. Kits included within the use according to this invention are single and multi-chambered pre-filled syringes; exemplary pre-filled syringes (e.g., liquid syringes, and lyosyringes such as Lyo-Ject®, a dual-chamber pre-filled lyosyringe) are available from Vetter GmbH, Ravensburg, Germany.

A KGF protein product(s) may be applied in combination with a GLP-2 protein product(s) in therapeutically- and prophylactically-effective amounts to organs or tissues specifically characterized by having damage to or clinically insufficient numbers of epithelium cells. It should be noted that KGF protein product(s) formulations described herein may be used for veterinary as well as human applications and that the term "patient" should not be construed in a limiting manner.

The frequency of dosing the KGF protein product(s) in combination with a GLP-2 protein product(s) to a patient will depend on the disease and the condition of the patient, as well as the pharmacokinetic parameters of KGF protein product(s) and GLP-2 protein product(s) as formulated, and the route of administration. The KGF protein product(s) in combination with a GLP-2 protein product(s) may be administered once, administered daily, or administered with an initial bolus dose followed by a continuous dose or sustained delivery. It is also contemplated that other modes of continuous or near-continuous dosing may be practiced. For example, chemical derivatization may result in sustained release forms of the protein which have the effect of a continuous presence in the bloodstream, in predictable amounts, based on a determined dosage regimen.

A patient in need of stimulation (including cytoprotection, proliferation and/or differentiation) of epithelial cells may be administered an effective amount of a KGF protein product(s) in combination with a GLP-2 protein product(s) to elicit the desired response in the patient. The dosage regimen involved in a method of preventing or treating a specific condition generally will be determined by the attending physician, considering various factors which modify the action of drugs, e.g., the age, condition, body weight, sex and diet of the patient, the severity of any infection, the time of administration and other clinical factors. Appropriate dosages may be ascertained through use of established assays for determining dosages utilized in conjunction with appropriate dose-response data. Typical dosages will range from 0.001 mg/kg body weight to 500 mg/kg body weight, preferably up to 200 mg/kg body weight, more preferably 100 mg/kg body weight.

The KGF protein product(s) in combination with a GLP-2 protein product(s) may be administered via topical, enteral or parenteral administration including, without limitation, infusion, intraarterial, intraarticular, intracapsular, intracardiac, intradermal, intramuscular, intraorbital, intrathecal, intravenous, intraperitoneal, intraspinal, intrasternal injection, intraventricular, subcutaneous, subcuticular, subcapsular, subarachnoid and transtracheal. The KGF protein product(s) may be administered via oral administration or administered through mucus membranes, that is, buccally, intranasally, rectally or sublingually for systemic delivery. The KGF protein product(s) in combination with a GLP-2 protein product(s) may be used once or administered repeatedly, depending on the disease and the condition of the patient. In some cases, the KGF protein product(s) may be administered as an adjunct to other therapy and also with other pharmaceutical preparations.

A KGF protein product(s) in combination with a GLP-2 protein product(s) may be applied in therapeutically- and prophylactically-effective amounts to organs or tissues specifically characterized by having damage to or clinically insufficient numbers of epithelium cells. It should be noted that a KGF protein product(s) in combination with a GLP-2 protein product(s) may be used for veterinary as well as human applications and that the term "patient" should not be construed in a limiting manner.

In accordance with the present invention, a KGF protein product(s) in combination with a GLP-2 protein product(s) is used for preparing a medicament to induce stimulation (including cytoprotection, proliferation and/or differentiation), proliferation and/or differentiation of epithelial cells in the gastrointestinal tract including cells in the oral cavity, in the esophagus, in the glandular stomach and small intestine, in the colon and the intestinal mucosa, in the rectum and in the anal canal. Indications in which a KGF protein product(s) in combination with a GLP-2 protein product(s) may be successfully administered include, but are not limited to: gastric and duodenal ulcers; gut toxicity in radiation- and chemotherapy-treatment regimes; erosions of the gastrointestinal tract (*e*.*g*., esophagus, stomach and intestines) include erosive gastritis, esophagitis, esophageal reflux or inflammatory bowel diseases, such as Crohn's disease (affecting primarily the small intestine) and ulcerative colitis (affecting primarily the large bowel); disorders or damage to salivary gland tissue including radiation/chemotherapy effects, autoimmune diseases such as Sjogren's Syndrome which can cause salivary gland insufficiency (sicca syndrome); insufficient production of mucus throughout the gastrointestinal tract; progressive gum disease; ulcerations and/or inflammations including conditions resulting from chemotherapy and/or infection.

Therapeutic agents such as analgesics and anesthetics also can be administered to alleviate pain and such as anti-infectictives, anti-bacterials, antifungals and/or antiseptics also can be administered to prevent and/or treat secondary infection of the lesions.

This invention thus has significant implications in terms of enabling the application of KGF protein product(s) in combination with a GLP-2 protein product(s) specifically characterized by the prophylactic and/or therapeutic use of KGF and GLP-2 to reduce, delay and/or block the onset of damage to or deficiencies in these particular types of cells. The following is a more specific description of diseases and medical conditions which can be treated with KGF protein product(s) in combination with a GLP-2 protein product(s) in accordance with the invention.

KGF protein product(s) are useful to increase cytoprotection, proliferation and/or differentiation

KGF protein product(s) in combination with a GLP-2 protein product(s) are useful to increase cytoprotection, proliferation and/or differentiation of epithelial cells in the gastrointestinal tract (e.g., the oral cavity, esophagus, stomach, small intestine, colon, rectum and anal canal). The terms "gastrointestinal tract", as defined herein, and "gut" are art-recognized terms and are used interchangeably herein. Specifically, KGF protein product(s) in combination with a GLP-2 protein product(s) are useful to treat and/or prevent gastric ulcers, duodenal ulcers, inflammatory bowel disease, gut toxicity and erosions of the gastrointestinal tract.

Gastric ulcers cause significant morbidity, have a relatively high recurrence rate, and heal by scar formation on the mucosal lining. KGF protein product(s) are useful to prevent degeneration of glandular mucosa and to regenerate glandular mucosa more rapidly, e.g., offering a significant therapeutic improvement in the treatment of gastric ulcers. Standard *in vivo* models of gastric ulcers are known (Tarnawski et al. (1991), "Indomethacin Impairs Quality of Experimental Gastric Ulcer Healing: A Quantitative Histological and Ultrastructural Analysis", In:*Mechanisms of Injury, Protection and Repair of the Upper Gastrointestinal Tract,* (eds) Garner and O'Brien, Wiley & Sons; Brodie (1968), *Gastroenterology,* 55:25; and Ohning et al. (1994), *Gastroenterology,* 106(4 Suppl.):A624.

Duodenal ulcers, like gastric ulcers, cause significant morbidity and have a relatively high recurrence rate. KGF protein product(s) in combination with a GLP-2 protein product(s) are useful to prevent degeneration of the mucosal lining of the duodenum and to rapidly regenerate the mucosal lining of the duodenum to heal those ulcers and decrease their recurrence. Standard *in vivo* models of duodenal ulcers are known (Berg et al. (1949), *Proc. Soc*. *Exp. Biol. Med.,* 7:374-376; Szabo and Pihan, *Chronobiol*. *Int.* (1987), 6:31-42; and Robert et al. (1970), *Gastroenterology,* 59:95-102.

Gut toxicity is a major limiting factor associated with cancer treatment, both in radiation (abdominal, total body or local, e.g., head and neck) and chemotherapy. Of primary concern are those patients undergoing: chemotherapy for cancer such as leukemia, breast cancer or as an adjuvant to tumor removal; radiotherapy for head and neck cancer; and combined chemotherapy and radiotherapy for bone marrow transplants. The severity of damage is related to the type and dose of chemotherapeutic agent(s) and concomitant therapy such as radiotherapy.

Mucositis in portions of the gastrointestinal tract may account for significant pain and discomfort for these patients, and range in severity from redness and swelling to frank ulcerative lesions. The lesions often become secondarily infected and become much harder to heal. Standard *in vivo* models of radiation-induced gut toxicity are known (Withers and Elkind (1970), *Int*. *J. Radiat.,* 17(3):261-267. Standard *in vivo* models of chemotherapy-induced gut toxicity are known (Farrell et al., The American Society of Hematology, 38th Annual Meeting (Orlando, FL), December 6-8, 1996; Sonis et al. (1990), *Oral Surg. Oral Med & Oral Pathol.,* 69(4):437-443; and Moore (1985), *Cancer Chamotherapy Pharmacol.,* 15:11-15, into.

Exemplary chemotherapeutic agents include, but are not limited to, BCNU, busulfan, carboplatin, cyclophosphamide, cisplatin, cytosine arabinoside, daunorubicin, doxorubicin, etoposide, 5-fluorouracil, gemcytabine, ifosphamide, irinotecan, melphalan, methotrexate, navelbine, topotecan, taxol and taxotere, and exemplary treatment regimes include, but are not limited to, BEAM (busulfan, etoposide, cytosine arabinoside, methotrexate); cyclophosphamide and total body irradiation; cyclophosphamide, total body irradiation and etoposide; cyclophosphamide and busulfan; and 5-fluorouracil with leucovorin or levamisole.

Treatment, pretreatment and/or post-treatment, with KGF protein product(s) in combination with a GLP-2 protein product(s) are useful to generate a cytoprotective effect or regeneration or both, for example, on the small intestinal mucosa, allowing increased dosages of such therapies while reducing potential fatal side effects of gut toxicity.

KGF protein product(s) may preferentially be administered in the following settings in combination with a GLP-2 protein product(s). Colorectal patients routinely are administered 5-fluorouracil with leucovorin on days 1 to 5; KGF protein product(s) may be administered on days -2, -1 and 0. Head and neck cancer patients routinely are administered hypofractionated radiation therapy, plus 5-fluorouracil and cisplatin over a seven week period; KGF protein product(s) may be administered on days -2, -1 and 0 and thereafter once per week until the end of the radiation therapy. In lymphoma transplantation patients are frequently administered BEAM therapy for 6 days (days 1 to 6); KGF protein product(s) may be administered on days -2, -1 and 0 and as a three day post-treatment (days 7 to 9).

In specific embodiments, KGF protein product(s) in combination with a GLP-2 protein product(s) may be administered prophylactically and/or therapeutically to reduce, delay and/or block the onset of mucositis (due to chemotherapy and/or radiotherapy), or to delay and/or block the onset of cytopenia.

Typically, bone marrow, peripheral blood progenitor cells or stem cells (McNiece et al. (1989), *Blood,* 74:609-612 and Moore et al. (1979), *Blood Cells,* 5:297-311, are removed from a patient prior to myelosuppressive cytoreductive therapy (chemotherapy alone or with radiation therapy) and are then readministered to the patient concurrent with or following cytoreductive therapy in order to counteract the myelosuppressive effects of such therapy.

Many different approaches have been undertaken to protect an organism from the side effects of radiation or toxic chemicals. One approach is to replace bone marrow cells before toxicity has developed. Another approach is to use progenitor cells from the peripheral blood (PBPC). These PBPC can be collected by apheresis or phlebotomy following cytokine therapy alone (G-CSF or GM-CSF), or with chemotherapy or cytokines. They can be given back fresh or cryopreserved. If desired, the cells may be CD34+ selected, Tn-cell depleted, tumor cell depleted, or the progenitor cells can be expanded (caused to multiply) by means known in the art, prior to administration. The benefits of re-infusion of autologous or allogeneic progenitors following myelosuppressive therapy have been described in the literature (Morse et al. (1992), *Ann*. *Clin*. *Lab. Sci*., 22:221-225; Kessinger and Armitage (1991), *Blood,* 77:211-213; Kessinger et al. (1989), Blood, 74:1260-1265; Takam et al. (1989), *Blood,* 74:1245-1251 and Kessinger et al. (1988), *Blood,* 171:723-727, the disclosures of which are hereby incorporated by reference).

As used herein, the term "cytokine" is a generic term for proteins released by one cell population which act on another cell as intercellular mediators. Examples of such cytokines are lymphokines, monokines and traditional polypeptide hormones. The cytokines are used in combination with a KGF protein product(s) to protect against, mitigate and/or reverse myeloid or hematopoietic toxicity associated with cytotoxic agents.

The mode of administration of KGF protein product(s), in combination with a GLP-2 protein product(s), should be coordinated and optimized. Depending upon the circumstances, an appropriate dose of KGF protein product(s) can be administered prior to or subsequent to administration of the therapeutic agent(s). For example, a parameter to be considered is whether the GLP-2 is administered in a single dose or in multiple doses during the course of therapy. Certain cytokines are cleared rapidly from the body and will require periodic or continuous administration in order for their efficacy to be maximized. The manner of administration can differ, depending on whether a pretreatment or post-treatment of the GLP-2 is given. For example, if the GLP-2 is given prior to the cytotoxic agent, it is preferable to administer the GLP-2 by intravenous bolus injection for several hours and, optionally, to repeat such administration on one or more days during and after completion of the cytotoxic therapy.

In a specific embodiment, KGF protein product(s) in combination with a GLP-2 protein product(s) are administered (e.g., intraveneously) at 0.1 to 500 micrograms/kg/dose, preferably up to about 200 micrograms/kg/dose, prior to (e.g., 1 to 3 days) and/or after chemotherapy or radiation therapy.

Erosions of the gastrointestinal tract (e.g., esophagus, stomach and intestine) include erosive gastritis, esophagitis, esophageal reflux and inflammatory bowel diseases. Inflammatory bowel diseases, such as Crohn's disease (affecting primarily the small intestine) and ulcerative colitis (affecting primarily the large bowel), are chronic diseases of unknown etiology which result in the destruction of the mucosal surface, inflammation, scar and adhesion formation during repair, and significant morbidity to the affected individuals. KGF protein product(s) in combination with a GLP-2 protein product(s) are useful to regenerate the mucosal lining and decrease the recurrence of these erosions, resulting in faster healing, and may be of benefit in controlling progression of the disease. Standard *in vivo* models of erosion of the gastrointestinal tract are known (Geisinger et al. (1990), *Mod-Pathol*., 3(5):619-624; Carlborg et al. (1983), *Laryngoscope,* 93(2):184-187; Carlborg et al. (1980), *Eur-Surg-Res.,* 12(4):270-282; Keshavarzian et al. (1991), *Alcohol-Clin-Exp-Res.,* 15(1):116-121; Katz et al. (1988), *Dig-Dis-Sci*., 33(2):217-224; and Zeeh et al. (1996), *Gastroenterology,* 110(4):1077-1083. Standard *in vivo* models of inflammatory bowel disease are well known (Morris et al. (1989), *Gastroenterology,* 96:795-803; Rachmilewitz et al. (1989), *Gastroenterology,* 97:326-327; Allgayer et al. (1989), *Gastroenterology,* 96:1290-1300; and Kim and Borstad (1992), *Scand*. *J. Gastroenterol,* 27(7):529-537.

Animal studies have established the relationship between total parenteral nutrition (TPN) and intestinal mucosal atrophy (Buchman et al. (1995), *Journal of Parenteral and Enteral Nutrition*, 19:453-460). The decrease in intestinal villus height is attributed to the lack of growth stimulus provided through oral intake of nutrients. This is reflected in a reduction in the labeling index, a measure of growth. Decreases in villus height are also correlated with decreases in specific activities of enzymes involved in nutrient absorption. KGF protein product(s) in combination with a GLP-2 protein product(s) are useful to either protect against atrophy during the fasting and/or facilitate regrowth upon reintroduction of oral nutrients.

The present invention is directed to the use of KGF protein products and GLP-2 protein products in combination to increase cytoprotection, proliferation and/or differentiation of epithelial cells in the gastrointestinal tract. Specifically, the combination therapy is useful to treat and/or prevent gastric ulcers, duodenal ulcers, inflammatory bowel disease, gut toxicity and erosions of the gastrointestinal tract. The term "GLP-2 peptide" refers collectively herein to human GLP-2 (SEQ ID NO:67) and to variants of thereof (defined, for example, in WO 96/32414 and, for example, hGLP-2(G2), a variant GLP-2 in which glycine is substituted for asparagine at position 2 (SEQ ID NO:68) and hGLP-2(52), a variant GLP-2 in which serine is substituted for asparagine at position 2 (SEQ ID NO:69), described herein). GLP-2 and variants thereof may be produced and purified by standard techniques (see, for example, WO 96/32414. WO 96/32414 reported that GLP-2 has effects on small bowel and pancreatic islets.

KGF protein product(s) in combination with a GLP-2 protein product(s) are useful to treat and/or prevent gum disease. Standard *in vivo* models of gum disease are known.

KGF protein product(s) in combination with a GLP-2 protein product(s) are useful to treat and/or prevent disorders or damage to salivary gland tissue, including radiation/chemotherapy effects (as discussed above) and autoimmune diseases such as Sjogren's Syndrome which can cause salivary glandinsufficiency (sicca syndrome). Standard *in vivo* models of salivary gland tissue damage are known.

The following examples are included to more fully illustrate the present invention. It is understood that modifications can be made in the procedures set forth without departing from the spirit of the invention.

### EXAMPLES

Standard methods for many of the procedures described in the following examples, or suitable alternative procedures, are provided in widely recognized manuals of molecular biology such as, for example, *Molecular Cloning,* Second Edition, Sambrook *et al*., Cold Spring Harbor Laboratory Press (1987) and *Current Protocols in Molecular Biology,* Ausabel *et al.* (1990), Greene Publishing Associates/Wiley Interscience, New York. All chemicals are either analytical grade or USP grade.

### Materials

The test materials used in the following *in vivo* studies were a KGF having a deletion of the first 23 amino acids of the amino-terminus of KGF (i.e., ΔN23 KGF) using standard techniques (WO 96/11949). All proteins were produced by recombinant expression in *E. coli* and purified to homogeneity. Each protein was administered as a subcutaneous formulation.

hGLP-2(G2) polypeptide was synthesized by the Fmoc (fluorenylmethoxycarbonyl)/t-butyl based solid phase peptide chemistry method. An ABI 431A (Perkin Elmer Corp., Foster City, CA) peptide synthesizer utilizing a single coupling program was used to carry out the chain assembly. Commercially available preloaded Fmoc-amino acid-HMP (hydroxymethyl-phenylacetyl) derivatized polystyrene resin was used to prepare the carboxy-terminal acid peptides. Subsequent amino acids were coupled as HOBT (hydroxybenztriazole) esters. Side-chain protection was employed as follows: Arg(PMC(2,2,5,7,8-pentamethylchroman-6-sulfonyl)), Asn(Trt), Asp(OtBu), Cys(Trt), Gln(Trt), Glu(OtBu), His(Trt), Lys(Boc), Ser(tBu), Thr(tBu), and Tyr(tBu). Gly(FmocHmb) was incorporated to remove the risk of formation of aspartimide or piperidide by-products. Upon removal of the final N-terminal Fmoc, side-chain protecting groups were removed and the peptides cleaved from the resin by treatment for 4 hours with TFA:triisopropylsilane:water:phenol (85:5:5:5). The resulting suspension was filtered, and the filtrate volume reduced by roto-evaporation. The crude peptide was precipitated and washed with ether, then dried in-vacuo overnight.

All crude peptides were purified to >95% homogeneity by reverse-phase HPLC on a preparative (2.5 cm x 25 cm) Vydac C₁₈ column with linear gradients of 99.95% acetonitrile plus 0.05% TFA versus 0.1% aqueous TFA.

Mass spectra for synthetic peptides were obtained on a Sciex API (Perkin Elmer Corp.) single quadropole mass spectrometer. All mass spectral samples were fractions off of the preparative HPLC purification.

Amino acid analyses were performed on a ABI 420A hydrolyzer/derivatizer with a 130A separation system (Perkin Elmer Corp.). The peptides were hydrolyzed using 6N HCl at 200°C for 30 minutes and then derivatized as the PTC (phenylisothiocynate) derivative. The amino acid mixture was then separated by HPLC on a Brownlee PTC C₁₈, 5 micron pore size, 2.1 x 220 mm with a linear gradient of 70% acetonitrile versus water. Both solvents were buffered with sodium acetate to pH=5.4. The amino acid determinations were quantitated by comparison of the unknown peak ratios with an equimolar amino acid standard.

### EXAMPLE 1: Intestinal Growth in Normal Animals

Normal BDF1 mice were treated with ΔN23 KGF given as a single daily injection of 5 mg/kg/day alone, hGLP-2(G2) given as a twice daily injection of 30 ug/injection, or both. The mice were treated for 6 days and euthanized for removal of the intestines on day 7. The entire length of the small intestine was removed,and hung under 5 g tension for measurement of length and subdivision into 3 segments. The first 10 cm was designated duodenum, the last 10 cm designated ileum and the remaining central portion jejunum. The segments were flushed with ice cold saline, patted dry and weighed. The mean wet weight values were converted to percent increases relative to control and the data is presented in Table 2 below.

**Table 2**

| Protein | Duodenum | Jejunum | Ileum | Total Gut |
|---|---|---|---|---|
| hGLP-2(G2) | 31.5 | 25.6 | 20.3 | 26.4 |
| ΔN23 KGF | 34.5 | 38.7 | 13.4 | 33.7 |
| ΔN23 KGF + hGLP-2(G2) treatment | 84.8 | 85.4 | 40.6 | 78.4 |

As seen in Table 2, treatment with hGLP-2(G2) or ΔN23 KGF administered alone each had a significant effect on the wet weight in the gut. Surprisingly, pretreatment with ΔN23 KGF and hGLP-2(G2) showed a synergistic effect on the increase of wet weight in the gut.

### EXAMPLE 2 - Chemotherapy-induced Mucositis Model

An animal model of chemotherapy-induced mucositis was used to investigate the combination therapy of ΔN23 KGF and hGLP-2(G2).

Mice (15/group) treated with 5-fluorouracil (Roche Laboratories, Nutley NJ), were administered with vehicle alone (saline), and with ΔN23 KGF (formulated in saline and/or hGLP-2(G2) in 0.1M ammonium bicarbonate, according to the following schedule:
Control group: On days -2 to 0, a group of mice were administered saline on days -2 to 0, and injected intraperitoneally with 5-fluorouracil (5-FU, 60 mg/kg/day) on days 1 to 4.
ΔN23 KGF pretreatment: On days -2 to 0, a group of mice were injected subcutaneously with ΔN23 KGF (5 mg/kg) . On days 1 to 4, the mice were injected intraperitoneally with 5-fluorouracil (5-FU, 60 mg/kg/day).
hGLP-2(G2) pretreatment: On days -5 to 0, a group of mice were injected subcutaneously with hGLP-2(G2) (3 mg/kg (split dose)). On days 1 to 4, the mice were injected intraperitoneally with 5-fluorouracil (5-FU, 60 mg/kg/day).
ΔN23 KGF and hGLP-2(G2) pretreatment: On days -2 to 0, a group of mice were injected subcutaneously with hGLP-2(G2) (3 mg/kg (split dose)) and injected subcutaneously with ΔN23 KGF (5 mg/kg). On days 1 to 4, the mice were injected intraperitoneally with 5-fluorouracil (5-FU, 60 mg/kg/day).

The effects on body weights and survival of the various groups were analyzed. Body weights were taken daily from day 0 to termination on day 30. Mice were examined visually twice/day for signs of morbidity for 30 days and all moribund animals were euthanized. The percent change in body weight on day 6 nadir is set forth in the Table 3.

**Table 3**

| Protein | % Change in Body Weight | Incidence of Hepatic Abcesses (%) |
|---|---|---|
| Saline | -11.6 | 3/5 (60) |
| ΔN23 KGF concurrent treatment | -11.1 | - |
| hGLP-2(G2) pretreatment | -11.0 | 3/6 (50) |
| ΔN23 KGF pretreatment | -4.1 | 2/18 (18) |
| ΔN23 KGF + hGLP-2(G2) pretreatment | -3.9 | 4/14 (27) |

As seen in Table 3, pretreatment with hGLP-2(G2) administered alone did not protect against weight loss, but pretreatment with ΔN23 KGF administered alone had a significant effect. Pretreatment with ΔN23 KGF and hGLP-2(G2) showed an improvement relative to pretreatment with ΔN23 KGF or hGLP-2(G2) alone. Hepatic abscesses are commonly found in control, but not ΔN23 KGF-pretreated, surviving mice indicating that 5-FU's toxicity is in part due to loss of the gastrointestinal barrier function. In addition, ΔN23 KGF-pretreated mice lose less weight and consume more food and water during the 5-FU treatment period.

Pretreatment with ΔN23 KGF increased survivability at 30 days relative to saline (75% vs. 35%), but not concurrent treatment (0% vs. 35%). Pretreatment with ΔN23 KGF and hGLP-2(G2) resulted in all animals survived for 30 days. hGLP-2(G2) given concurrently with ΔN23 KGF as a pretreatment significantly improved survival compared to survival in group given ΔN23 KGF alone as a pretreatment.

While the present invention has been described above both generally and in terms of preferred embodiments, it is understood that other variations and modifications will occur to those skilled in the art in light of the description above.

## Claims

1. Use of a KGF protein product(s) and a GLP-2 protein product(s) for the preparation of a medicament for increasing the cytoprotection, proliferation and/or differentiation of epithelial cells in the gastrointestinal tract of a patient.

2. Use according to claim 1, wherein the KGF protein product(s) is selected from the group consisting of C(1,15)S, ΔN15, ΔN16, ΔN17, ΔN18, ΔN19, ΔN20, ΔN21, ΔN22, ΔN23, ΔN24, ΔN3/C(15)S, ΔN3/C(15)-, ΔN8C(15)S, ΔN8/C(15)-, C(1,15)S/R(144)E, C(1,15)S/R(144)Q and ΔN23/R(144)Q.

3. Use according to claim 1 or 2, wherein the GLP-2 protein product(s) is selected from the group consisting of human GLP-2, hGLP-2(G2) and hGLP-2(S2).

4. Use according to any of claims 1 to 3, wherein the GLP-2 protein product(s) and KGF protein product(s) are provided in prophylactically effective amounts.

5. Use according to any of claims 1 to 4, wherein the medicament is for the treatment of mucositis.

6. Use of a KGF protein product(s) for the preparation of a medicament for increasing the cytoprotection, proliferation and/or differentiation of epithelial cells in the gastrointestinal tract of a patient, wherein said medicament is administered simultaneously, separately or sequentially with the administration of a GLP-2 protein product(s).

7. Use according to claim 6, wherein the KGF protein product(s) is an analog of KGF selected from the group consisting of C(1,15)S, ΔN15, ΔN16, ΔN17, ΔN18, ΔN19, ΔN20, ΔN21, ΔN22, ΔN23, ΔN24, ΔN3/C(15)S, ΔN3/C(15)-, ΔN8C(15)S, ΔN8/C(15)-, C(1,15)S/R(144)E, C(1,15)S/R(144)Q and ΔN23/R(144)Q.

8. Use according to claim 6 or 7, wherein the GLP-2 protein product(s) is selected from the group consisting of human GLP-2, hGLP-2(G2) and hGLP-2(S2).

9. Use according to any of claims 6 to 8, wherein the GLP-2 protein product(s) and KGF protein product(s) are provided in prophylactically effective amounts.

10. Use according to any of claims 6 to 9, wherein the medicament is for the treatment of gut toxicity.

11. Use according to claim 10, wherein the medicament is for the treatment of mucositis.

12. Use of a GLP-2 protein product(s) for the preparation of a medicament for increasing the cytoprotection, proliferation and/or differentiation of epithelial cells in the gastrointestinal tract of a patient, wherein said medicament is administered simultaneously, separately or sequentially with the administration of a KGF protein product(s).

13. Use according to claim 12, wherein the GLP-2 protein product(s) is selected from the group consisting of human GLP-2, hGLP-2(G2) and hGLP-2(S2).

14. Use according to claim 12 or 13, wherein the KGF protein product(s) is an analog of KGF selected from the group consisting of C(1,15)S, ΔN15, ΔN16, ΔN17, ΔN18, ΔN19, ΔN20, ΔN21, ΔN22, ΔN23, ΔN24, ΔN3/C(15)S, ΔN3/C(15)-, ΔN8C(15)S, ΔN8/C(15)-, C(1,15)S/R(144)E, C(1,15)S/R(144)Q and ΔN23/R(144)Q.

15. Use according to any of claims 12 to 14, wherein the GLP-2 protein product(s) and KGF protein products(s) are provided in prophylactically effective amounts.

16. Use according to any of claims 12 to 15, wherein the medicament is for treatment of gut toxicity.

17. Use according to claim 16, wherein the medicament is for the treatment of mucositis.

## Patentansprüche

1. Verwendung eines KGF-Proteinprodukts (von KGF-Proteinprodukten) und eines GLP-2-Proteinprodukts (von GLP-2-Proteinprodukten) zum Herstellen eines Medikaments zum Steigem der Zytoprotektion, Proliferation und/oder Differenzierung von Epithelzellen im Gastrointestinaltrakt eines Patienten.

2. Verwendung nach Anspruch 1, wobei das KGF-Proteinprodukt (die KGF-Proteinprodukte) aus der Gruppe ausgewählt ist (sind), die aus C(1,15)S, ΔN15, ΔN16, ΔN17, ΔN18, ΔN19, ΔN20, ΔN21, ΔN22, ΔN23, ΔN24, ΔN3/C(15)S, ΔN3/C(15)-, ΔN8C(15)S, ΔN8/C(15)-, C(1,15)S/R(144)E, C(1,15)S/R(144)Q und ΔN23/R(144)Q besteht.

3. Verwendung nach Anspruch 1 oder 2, wobei das GLP-2-Proteinprodukt (die GLP-2-Proteinprodukte) aus der Gruppe ausgewählt ist (sind), die aus menschlichem GLP-2, hGLP-2(G2) und hGLP-2(S2) besteht.

4. Verwendung nach einem der Ansprüche 1 bis 3, wobei das GLP-2-Proteinprodukt (die GLP-2-Proteinprodukte) und das KGF-Proteinprodukt (die KGF-Proteinprodukte) in prophylaktisch wirksamen Mengen bereitgestellt werden.

5. Verwendung nach einem der Ansprüche 1 bis 4, wobei das Medikament zum Behandeln von Mukositis bestimmt ist.

6. Verwendung eines KGF-Proteinprodukts (von KGF-Proteinprodukten) zum Herstellen eines Medikaments zum Steigem der Zytoprotektion, Proliferation und/oder Differenzierung von Epithelzellen im Gastrointestinaltrakt eines Patienten, wobei das Medikament gleichzeitig mit der Gabe eines GLP-2-Proteinprodukts (von GLP-2-Proteinprodukten), getrennt davon oder darauf folgend verabreicht wird.

7. Verwendung nach Anspruch 6, wobei das KGF-Proteinprodukt (die KGF-Proteinprodukte) ein Analogon von KGF ist (sind), ausgewählt aus der Gruppe, die aus C(1,15)S, ΔN15, ΔN16, ΔN17, ΔN18, ΔN19, ΔN20, ΔN21, ΔN22, ΔN23, ΔN24, ΔN3/C(15)S, ΔN3/C(15)-, ΔN8C(15)S, ΔN8/C(15)-, C(1,15)S/R(144)E, C(1,15)S/R(144)Q und ΔN23/R(144)Q besteht.

8. Verwendung nach Anspruch 6 oder 7, wobei das GLP-2-Proteinprodukt (die GLP-2-Proteinprodukte) aus der Gruppe ausgewählt ist (sind), die aus menschlichem GLP-2, hGLP-2(G2) und hGLP-2(S2) besteht.

9. Verwendung nach einem der Ansprüche 6 bis 8, wobei das GLP-2-Proteinprodukt (die GLP-2-Proteinprodukte) und das KGF-Proteinprodukt (die KGF-Proteinprodukte) in prophylaktisch wirksamen Mengen bereitgestellt werden.

10. Verwendung nach einem der Ansprüche 6 bis 9, wobei das Medikament zum Behandeln von Darmtoxizität bestimmt ist.

11. Verwendung nach Anspruch 10, wobei das Medikament zum Behandeln von Mukositis bestimmt ist.

12. Verwendung eines GLP-2-Proteinprodukts (von GLP-2-Proteinprodukten) zum Herstellen eines Medikaments zum Steigem der Zytoprotektion, Proliferation und/oder Differenzierung von Epithelzellen im Gastrointestinaltrakt eines Patienten, wobei das Medikament gleichzeitig mit der Gabe eines KGF-Proteinprodukts (von KGF-Proteinprodukten), getrennt davon oder darauf folgend verabreicht wird.

13. Verwendung nach Anspruch 12, wobei das GLP-2-Proteinprodukt (die GLP-2-Proteinprodukte) aus der Gruppe ausgewählt ist (sind), die aus menschlichem GLP-2, hGLP-2(G2) und hGLP-2(S2) besteht.

14. Verwendung nach Anspruch 12 oder 13, wobei das KGF-Proteinprodukt (die KGF-Proteinprodukte) ein Analogon von KGF ist (sind), ausgewählt aus der Gruppe, die aus C(1,15)S, ΔN15, ΔN16, ΔN17, ΔN18, ΔN19, ΔN20, ΔN21, ΔN22, ΔN23, ΔN24, ΔN3/C(15)S, ΔN3/C(15)-, ΔN8C(15)S, ΔN8/C(15)-, C(1,15)S/R(144)E, C(1,15)S/R(144)Q und ΔN23/R(144)Q besteht.

15. Verwendung nach einem der Ansprüche 12 bis 14, wobei das GLP-2-Proteinprodukt (die GLP-2-Proteinprodukte) und das KGF-Proteinprodukt (die KGF-Proteinprodukte) in prophylaktisch wirksamen Mengen bereitgestellt werden.

16. Verwendung nach einem der Ansprüche 12 bis 15, wobei das Medikament zum Behandeln von Darmtoxizität bestimmt ist.

17. Verwendung nach Anspruch 16, wobei das Medikament zum Behandeln von Mukositis bestimmt ist.

## Revendications

1. Utilisation de produit(s) de protéine KGF et de produit(s) de protéine GLP-2 pour la préparation d'un médicament destiné à augmenter la cytoprotection, la prolifération et/ou la différenciation des cellules épithéliales dans le tractus gastro-intestinal d'un patient.

2. Utilisation selon la revendication 1, dans laquelle le ou les produits de protéine KGF sont choisis dans l'ensemble consistant en C(1,15)S, ΔN15, ΔN16, ΔN17, ΔN18, ΔN19, ΔN20, ΔN21, ΔN22, ΔN23, ΔN24, ΔN3/C(15)S, ΔN3/C(15)-, ΔN8C(15)S, ΔN8/C(15)-, C(1,15)S/R(144)E, C(1,15)S/R(144)Q et ΔN23/R(144)Q.

3. Utilisation selon la revendication 1 ou 2, dans laquelle le ou les produits de protéine GLP-2 sont choisis dans l'ensemble consistant en GLP-2 humaine, hGLP-2(G2) et hGLP-2(S2).

4. Utilisation selon l'une quelconque des revendications 1 à 3, dans laquelle le ou les produits de protéine GLP-2 et le ou les produits de protéine KGF sont fournis en quantités efficaces du point de vue prophylactique.

5. Utilisation selon l'une quelconque des revendications 1 à 4, dans laquelle le médicament est destiné au traitement d'inflammation de muqueuse.

6. Utilisation de produit(s) de protéine KGF pour la préparation d'un médicament destiné à augmenter la cytoprotection, la prolifération et/ou la différenciation des cellules épithéliales dans le tractus gastro-intestinal d'un patient, ledit médicament étant administré simultanément à, séparément de ou successivement à l'administration de produit(s) de protéine GLP-2.

7. Utilisation selon la revendication 6, dans laquelle le ou les produits de protéine KGF sont des analogues de KGF choisis dans l'ensemble consistant en C(1,15)S, ΔN15, ΔN16, ΔN17, ΔN18, ΔN19, ΔN20, ΔN21, ΔN22, ΔN23, ΔN24, ΔN3/C(15)S, ΔN3/C(15)-, ΔN8C(15)S, ΔN8/C(15)-, C(1,15)S/R(144)E, C(1,15)S/R(144)Q et ΔN23/R(144)Q.

8. Utilisation selon la revendication 6 ou 7, dans laquelle le ou les produits de protéine GLP-2 sont choisis dans l'ensemble consistant en GLP-2 humaine, hGLP-2(G2) et hGLP-2(S2).

9. Utilisation selon l'une quelconque des revendications 6 à 8, dans laquelle le ou les produits de protéine GLP-2 et le ou les produits de protéine KGF sont fournis en quantités efficaces du point de vue prophylactique.

10. Utilisation selon l'une quelconque des revendications 6 à 9, dans laquelle le médicament est destiné au traitement de toxicité intestinale.

11. Utilisation selon la revendication 10, dans laquelle le médicament est destiné au traitement d'inflammation de muqueuse.

12. Utilisation de produit(s) de protéine GLP-2 pour la préparation d'un médicament destiné à augmenter la cytoprotection, la prolifération et/ou la différenciation des cellules épithéliales dans le tractus gastro-intestinal d'un patient, ledit médicament étant administré simultanément à, séparément de ou successivement à l'administration de produit(s) de protéines KGF.

13. Utilisation selon la revendication 12, dans laquelle le ou les produits de protéine GLP-2 sont choisis dans l'ensemble consistant en GLP-2 humaine, hGLP-2(G2) et hGLP-2(S2).

14. Utilisation selon la revendication 12 ou 13, dans laquelle le ou les produits des protéines KGF sont des analogues de KGF choisis dans l'ensemble consistant en C(1,15)S, ΔN15, ΔN16, ΔN17, ΔN18, ΔN19, ΔN20, ΔN21, ΔN22, ΔN23, ΔN24, ΔN3/C(15)S, ΔN3/C(15)-, ΔN8C(15)S, ΔN8/C(15)-, C(1,15)S/R(144)E, C(1,15)S/R(144)Q et ΔN23/R(144)Q.

15. Utilisation selon l'une quelconque des revendications 12 à 14, dans laquelle le ou les produits de protéine GLP-2 et le ou les produits de protéine KGF sont fournis en quantités efficaces du point de vue prophylactique.

16. Utilisation selon l'une quelconque des revendications 12 à 15, dans laquelle le médicament est destiné au traitement de toxicité intestinale.

17. Utilisation selon la revendication 16, dans laquelle le médicament est destiné au traitement d'inflammation de muqueuse.
